# EUROPEAN PATENT APPLICATION

(11) **EP 1 785 421 A1**
(43) Date of publication of application: **16.05.2007**
(21) Application number: 05774538.2
(22) Date of filing: 29.08.2005
(51) Int. Cl.: C07D 451/06, A61K 9/72, A61K 31/46, A61K 45/00, A61P 11/00, A61P 11/06, A61P 11/08, A61P 43/00

(54) **TROPAN COMPOUND**

(30) Priority: 30.08.2004 JP 2004250284; 31.01.2005 JP 2005023439; 06.07.2005 JP 2005196926
(71) Applicant: ONO PHARMACEUTICAL CO., LTD., Osaka-shi, Osaka 541-8526 (JP)
(72) Inventor: NAKAI, Hisao, home, Shimamoto-cho, Mishima-gun, Osaka (JP); NISHIYAMA, Toshihiko, home, Shimamoto-cho, Mishima-gun, Osaka (JP); NAKAMURA, Nobuyuki, home, Shimamoto-cho, Mishima-gun, Osaka (JP); FUJITA, Manabu, home, Shimamoto-cho, Mishima-gun, Osaka (JP)
(74) Representative: Teipel, Stephan
(86) International application number: PCT/JP2005/015659
(87) International publication number: WO 2006/025324

(57) **Abstract**

The conventional anticholinergic drugs for administration through inhalation have been considered to have the possibility of aggravating dysuria associated with prostatic hyperplasia mediated by blood, and it has been demanded that the conventional anticholinergic drugs for administration through inhalation will have to show reduced side effects or adverse ractions.

The present invention relates to a compound represented by the general formula (I): 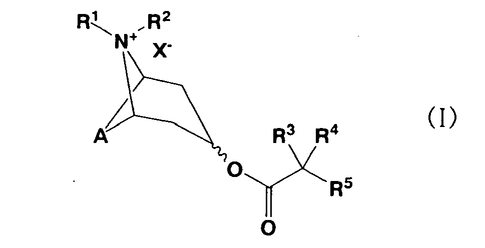 (wherein A represents; 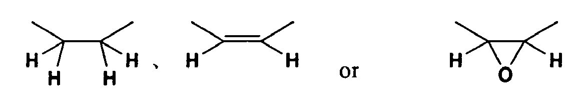 and R¹, R², R³ and R¹ each a hydrogen atom or a substituent;
R⁵ is a substituent; X⁻ is an anion;the symbol: 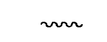 denotes an exo-form or endo-form, or their mixture), its salt or solvation product thereof. They are useful as a prophylactic and/or therapeutic agent with reduced side effects or adverse reactions for the diseases mediated by the muscarinic receptor.

## Description

### Technical Field:

The present invention relates to:
(1) Compounds represented by the general formula (I): 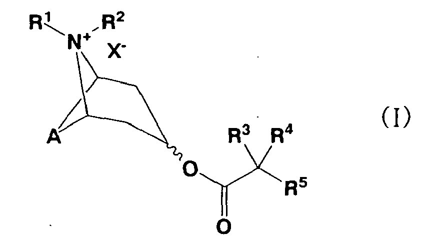 (wherein all the symbols are the same as defined below), their salts, their solvation products or prodrugs thereof,
(2) Process for producing the same,
(3) Pharmaceutical compositions comprising the same as an active ingredient, and
(4) Uses of the same.

### Background Art:

The anticholinergic drugs for administration through inhalation are the drug of first choice as a suppressant drug against obstruction of the airways in chronic obstructive pulmonary disease (COPD), and are the medicinal substances which act to ameliorate dyspnea and shortness of breath, and the like. In Europe and the USA, ipratropium and tiotropium find clinical application. However, such conventionally used anticholinergic drugs in some instances aggravate dysuria associated with prostatic hyperplasia, simply because the drugs, when administered through inhalation, immigrate from the lungs into the bloodstream to thereby allow blood-mediated relaxation of the smooth muscle of the bladder. Most of patients with COPD are the elderly people, while many of the elderly people often are potentially afflicted with enlargement of the prostate gland, and not a small number of the COPD patients complain of difficulties in passing the urine owing to the use of the anticholinergic drugs. Consequently, a compound, which undergoes quick metabolism in the blood after exerting action at the targeted site, can be considered to be the anticholinergic drug that can find safe, clinical application in the treatment of the elderly people and the COPD patients complicated with prostatic hyperplasia.

Compounds represented by the general formula (A): 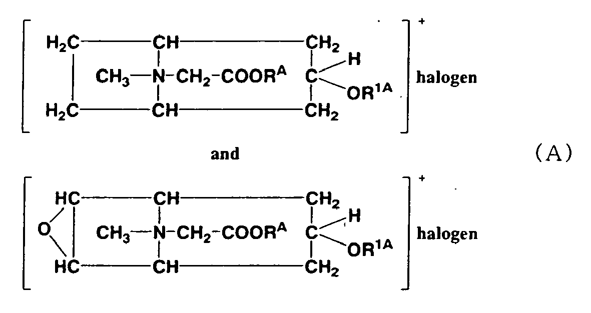 (wherein R^{A} is a straight-chain or branched C6 - 11 alkyl or cyclohexyl group; R^{1A} is a diphenylhydroxyacetyl or phenyl-methylhydroxyacetyl group) have been known to alleviate such side effects or adverse reactions (refer to US Patent No. 2872452). The said prior art literature reference did neither describe nor suggest their effects against the pulmonary diseases, although it described a comparison between the spasmolytic activity and the activity against mydriasis.

Also, there was given the description (The Official Gazette of Japanese Patent No. 2672311) that compounds represented by the general formula (B): 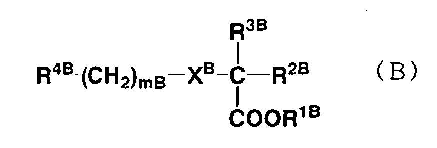 [wherein R^{1B} is a straight-chain or branched alkyl or cyclo alkyl group having a number of carbon atoms of up to 8; R^{2B} is an aryl, cycloalkyl or alkyl group having a number of carbon atoms of up to 8; R^{3B} is H or a group to be defined by R^{2B}; X^{B} is a group represented by the formula: 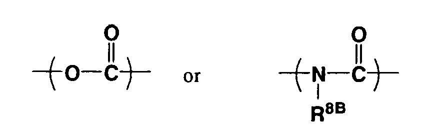 (where R^{8B} is H or a straight-chain or branched alkyl group having a number of carbon atoms of up to 5); m^{B} is an integer of 0 to 4; R^{4B} is a group represented by the formula: 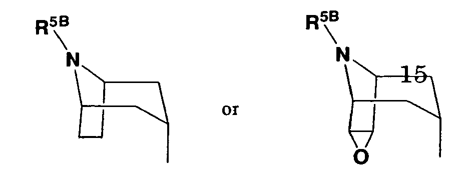 (where R^{5B} is an alkyl group having a number of carbon atoms of up to 5) ; it is to be noticed that the definitions for the symbols are as excerpted to such an extent as may be considered necessary], or quaternary ammonium salts of the compound of the formula (B) formed with the compound of the formula R^{7B}Y^{B} (wherein R^{7B} is an alkyl group having a number of carbon atoms of up to 5; and Y^{B} is a pharmaceutically acceptable anion) exhibit anticholinergic activity and (or) mydriatic activity. The prior art literature reference did neither describe nor suggest nothing about the effects on the respiratory diseases, although it described merely the mydriasis.

### Disclosure of the Invention

### [The Problem That the Invention Is Intended to Solve]

It has been well known that the conventionally used anticholinergic drugs intended for administration through inhalation antagonize the muscarinic receptors on the smooth muscle of the airways and furthermore immigrate into the blood from the airways. Such existing anticholinergic drugs for administration through inhalation are thought to suggest the possibility of aggravating the urinary obstruction associated with prostatic hyperplasia mediated by blood, and there has been demanded the anticholinergic drug which shows reduced side effects or adverse reactions.

### [Means for Solving the Problem]

The present inventors, with a specific view to solving the above-described problem, conducted intensive research, and as a result, found that the compounds represented by the general formula (I) can achieve such object, leading to completion of the present invention.

Namely, the present invention relates to:
(1) A compound represented by the general formula (I): 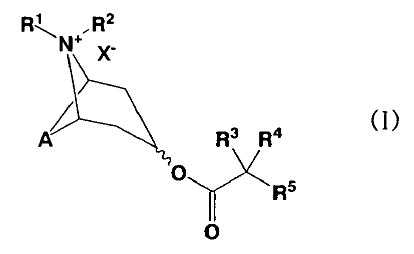 (wherein A represents the following structures: 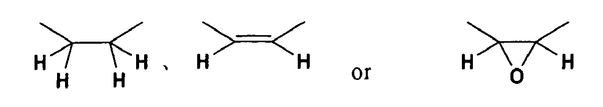 R¹ is a hydrogen atom or a substituent; R² is a hydrogen atom or a substituent; R³ is a hydrogen atom or a substituent; R⁴ is a hydrogen atom or a substituent; R⁵ is a substituent; X⁻ is an anion; the symbol: 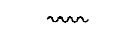 denotes an exo-form or endo-form, or their mixture), its salt or solvation product thereof;
(2) The compound as described above under the item (1), wherein R¹, R³, R⁴ and/or R⁵ each are(or is) an ester-linkage containing group;
(3) The compound as described above under the item (1), wherein R¹ is an ester-linkage containing group;
(4) The compound as described above under the item (3), wherein R¹ constitutes: 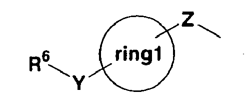 (wherein R⁶ is an ester-linkage containing group; the symbol: 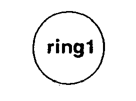 represents a cyclic group which may be substituted with a substituent(s); Y is a linkage(s) or a spacer of 1 to 5 carbon atoms for the main chain which may be substituted with a substituent(s); Z is a linkage(s) or a spacer of 1 to 5 carbon atoms for the main chain which may be substituted with a substituent(s));
(5) The compound as described above under the item (4), wherein the cyclic group as described above under the item (4) is a C3~15 monocyclic unsaturated carbon ring which may be partially or fully saturated, or a C4~15 bicyclic unsaturated carbon ring which may be partially or fully saturated, or a C3~15-membered monocyclic unsaturated heterocyclic ring containing 1 to 5 heteroatoms selected from oxygen, nitrogen and sulfur atoms which ring may be partially or fully saturated, or a C4~15 bicyclic unsaturated heterocyclic ring containing 1 to 5 heteroatoms selected from oxygen, nitrogen and sulfur atoms which ring may be partially or fully saturated;
(6) The compound as described above under the item (4), wherein the cyclic group as described above under the item (4) is benzene, thiophene or naphthalene;
(7) The compound as described above under the item (4), which is represented by the general formula (I-1): 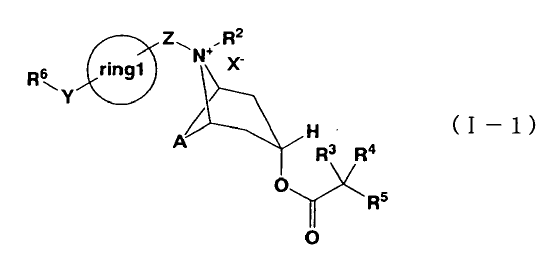 (wherein all the symbols have the same meanings as described above under the item (1) and (4));
(8) The compound as described above under the item (7), which is selected from (1R,3r,5S,8s)-8-{[6-(2-ethoxy-2-oxoethoxy)-2-naphthyl]methy 1}-3-{[hydroxy(diphenyl)acetyl]oxy}-8-methyl-8-azoniabicycl o[3,2,1]octane bromide, (1R,3s,5S,8r)-8-{[4-(3-ethoxy-3-oxopropyl)benzyl]-3-({hydro xy[di(2-thienyl]acetyl}oxy)-8-methyl-8-azoniabicyclo[3,2,1] oct-6-en bromide, (1R,3s,5S,8r)-8-{[5-(4-ethoxy-4-oxobutyl)-2-thienyl]methyl} -3-{[hydroxy(diphenyl)acetyl]oxy}-8-methyl-8-azoniabicyclo[ 3,2,1]oct-6-en chloride, (1R,2R,4S,5S,7s,9r)-9-[4-(3-ethoxy-3-oxopropyl)benzyl]-7-{[ hydroxy(di-3-thienyl)acetyl]oxy}-9-methyl-3-oxa-9-azoniatricy clo[3,3,1,0^{2,4}]nonane bromide and (1R,2R,4S,5S,7s,9r)-9-(4-{3-[2-(dimethylamino)-2-oxoethyl)-3-oxopropyl]benzyl)-7-{[hydroxy(di-2-thienyl)acetyl]oxy}-9-methyl-3-oxa-9-azoniatricyclo[3,3,1,0^{2,4}]nonane bromide;
(9) The compound as described above under the item (1), wherein R³ and/or R⁴ each are an ester-linkage containing group;
(10) The compound as described above under the item (9), wherein R³ is represented by the formula: 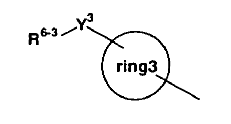 (wherein R⁶⁻³ is an ester-linkage containing group; Y³ is a linkage(s) or a spacer of 1 to 5 atoms for the main chain which may be substituted with a substituent(s); and the symbol: 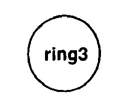 represents a cyclic group which may be substituted with a substituent(s));
(11) The compound as described above under the item (9), wherein R³ is represented by the formula: 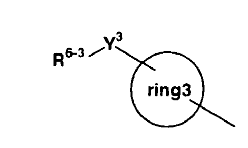 (wherein all the symbols are as defined above under the item (10)), and R⁴ is represented by the formula: 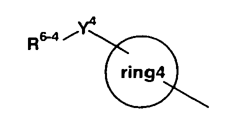 (wherein R⁶⁻⁴ is an ester-linkage containing group; Y⁴ is a linkage(s) or a spacer of 1 to 5 atoms for the main chain which may be substituted with a substituent (s); and the symbol: 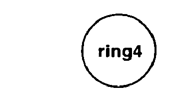 represents a cyclic group which may be substituted with a substituent(s));
(12) The compound as described above under the item (10), wherein "ring 3" is benzene, thiophene or furan;
(13) The compound as described above under the item (10), wherein A is represented by the formula: 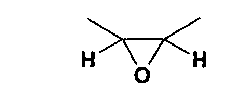
(14) The compound as described above under the item (10), wherein R¹ and R² each are a methyl group;
(15) The compound as described above under the item (14), which is selected from (1R,2R,4S,5S,7s)-7-({hydroxy[5-(methoxycarbonyl)-2-thienyl] -2-thienylacetyl}oxy)-9,9-dimethyl-3-oxa-9-azoniatricyclo-[3.3.1.0^{2,4}]nonane bromide, (1R,2R,4S,5S,7s)-7-({hydroxy[5-(methoxycarbonyl)-2-furyl]-2 -thienylacetyl}oxy)-9,9-dimethyl-3-oxa-9-azoniatricyclo-[3.3.1.0^{2,4}]nonane bromide and (1R,2R,4S,5S,7s)-7-{[[5-(ethoxycarbonyl)-2-thienyl]-(hydroxy)phenylacetyl]oxy}-9,9-dimethyl-3-oxa-9-azoniatricyclo[3.3.1.0^{2,4}]nonane bromide;
(16) The compound as described above under the item (1), wherein R⁵ is an ester-linkage containing group;
(17) The compound as described above under the item (16), wherein R⁵ is represented by: 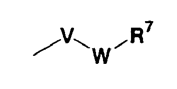 (wherein R⁷ is an ester-linkage containing group; V is a carbon atom which may be substituted with a substituent(s), a nitrogen atom which may be substituted with a substituent(s), a sulfur atom which may be oxidized or an oxygen atom; W is a linkage (s) or a spacer of 1 to 5 atoms for the main chain which may be substituted with a substituent (s);
(18) The compound as described above under the item (17), which is represented by the general formula (I-2): 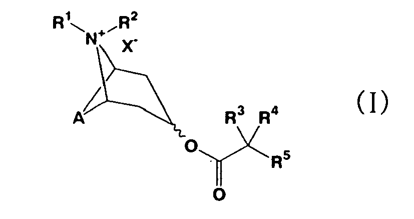 (wherein all the symbols are as defined above under the item (1) and (17)).
(19) The compound as described above under the item (18), wherein R³ and R⁴ each independently are a carbon cyclic group which may be substituted or a heterocyclic group which may be substituted;
(20) The compound represented by the general formula (I) as described above under the item (1), its salt or a prodrug of a solvation product thereof;
(21) A pharmaceutical composition which comprises the compound represented by the general formula (I) as describe above under the item (1), its salt or their solvation product, or a prodrug thereof as an active ingredient;
(22) The pharmaceutical composition as described above under the item (21), which is a prophylactic and/or therapeutic agent for the diseases mediated by the muscarinic receptor;
(23) The pharmaceutical composition as described above under the item (22), wherein the disease mediated by the muscarinic receptor is chronic occlusive pulmonary disease and/or asthma;
(24) A pharmaceutical composition which comprises the compound represented by the general formula (I) as described above under the item (1), its salt or their solvation product, or a prodrug thereof in combination with not less than at least one kind selected from cysLT1-receptor antagonist drugs, cysLT2-receptor antagonist drugs, antihistamine drugs, antiallergy drugs, steroidal drugs, bronchodilators, vaccination therapy agents, gold compound preparations, Chinese herbal medicines, basic non-steroidal anti-inflammatory drugs, 5-lipoxygenase inhibitor drugs, 5-lipoxygenase activating protein antagonist drugs, leucotriene synthesis inhibitor drugs, prostaglandins, cannabinoid-2-receptor stimulant drugs, phosphodiesterase inhibitor drugs, antitusssive drugs, expectorant drugs and extraction liquids from skin inflammations of a household rabbit inoculated with vaccinia virus;
(25) A method for preventing and/or treating a disease mediated by the muscarinic receptor, characterized in that said process comprises administering to a mammal an effective amount of the compound represented by the general formula (I) as described above under the item (1), its salt or their solvation product, or a prodrug thereof;
(26) A use of the compound represented by the general formula (I) as describe above under the item (1), its salt or their solvation product, a prodrug thereof in the manufacture of a prophylactic and/or therapeutic agent for a disease mediated by the muscarinic receptor;
(27) An airway-constriction suppressing agent, characterized in that an effective dose for the airway-constriction suppression is less than the no-effective dose for the bladder constriction;
(28) The agent as described above under the item (27), characterized in that said agent fails to exhibit suppressory activity against the bladder constriction;
(29) The agent as described above under the item (27), wherein the agent comprises an effective amount of the compound as described above under the item (27);
(30) The agent as described above under the item (27), wherein the compound is a compound showing not less than 7 in a pK_{B} value against the tracheal-muscle constriction reaction;
(31) The agent as described above under the item (27), wherein the agent is a prophylactic and/or therapeutic agent for a disease mediated by the muscarinic receptor;
(32) The agent as described above under the item (31), wherein the disease mediated by the muscarinic receptor is chronic occlusive pulmonary disease and/or asthma;
(33) The agent as described above under the item (27), wherein the agent is in the form of an inhalant;
(34) The agent as described above under the item (27), characterized in that said agent is administered once a day;
(35) The agent as described above under the item (27), wherein the compound is a compound represented by the general formula (I): 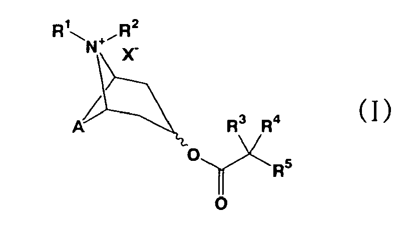 (wherein all the symbols are as defined above under the item (1)), its salt or their solvation product, or a prodrug thereof;
(36) A method for suppressing the airway-constriction, characterized in that said method comprises administering to a mammal an effective dose of a compound, which shows an effective amount for the airway constriction suppression being less than the no-effective dose for the bladder constriction; and
(37) A use of the compound which exhibits an effective amount for the airway-constriction suppression being less than the no-effective dose for the bladder constriction in the manufacture of the airway-constriction suppressant.

### [Effect of the Invention]

The compounds of the present invention, which can produce the desired effect at the targeted site and thereafter undergo quick inactivation, are useful as a prophylactic and/or therapeutic agent with reduced side effects or adverse reactions (for example, urinary obstruction, thirst, tachycardia, gastrointestinal disorders, glaucoma, etc.) for the diseases mediated by the muscarinic receptors.

Furthermore, the compounds of the present invention, which antagonize the muscarinic receptor specifically but exert weak antagonistic activity against other receptors, show improved selectivity and exhibit sustained airway-constricting action.

The compounds of the present invention also show improved solubility and absorbability. In addition, the compounds of the present invention elicit extremely lowered inhibitory activities against drug-metabolizing enzymes. Such characteristics are the most required physical, chemical and pharmacological properties in the development of pharmaceutical drugs, and consequently, the compounds of the present invention are considered to possess the conditions of providing outstandingly excellent pharmaceutical preparations [The Merck Manual of Diagnosis and Therapy (17^{th} Ed.), published by Merck & Co.].

### [Brief Description of the Drawings]

Fig. 1 is graphs showing the suppressory effect against the airway constriction brought about in guinea pigs by the compound as described in Example 14 (4), the compound of the present invention;
Fig. 2 is graphs showing the suppressory effect against the airway constriction manifested in guinea pigs by tiotropium;
Fig. 3 is graphs showing the suppressory effect against the bladder constriction produced in guinea pigs by the compound as described in Example 14 (4), the compound of the present invention; and
Fig. 4 is graphs showing the suppressory effect against the bladder constriction manifested in guinea pigs by tiotropium.

### [The Best Mode for Carrying Out the Invention]

As used herein, the substituent represented by R¹ includes, for example, (1) optionally substituted alkyl groups, (2) optionally substituted alkenyl groups, (3) optionally substituted alkynyl groups, (4) optionally substituted carbon cyclic groups, (5) optionally substituted heterocyclic groups, (6) optionally protected hydroxy groups, (7) optionally protected mercapto groups, (8) optionally protected amino groups, (9) a nitro group, (10) a cyano group, (11) an amidiono group or (12) groups represented by the formula: 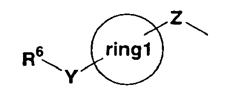 (wherein all the symbols are as defined above).

The alkyl group in the "optionally substituted alkyl groups" as a substituent represented by R¹ may be exemplified by straight-chain or branched C1~20 alkyl groups, such as a methyl, ethyl, n-propyl, isopropyl, n-butyl, isobutyl, sec-butyl, tert-butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, icosyl group, etc., and the like. On the above-described occasions, the substituent on the alkyl groups includes, for example, a hydroxy group , an amino group, a carboxyl group, a nitro group, an azido group, mono- or di-C1~6 alkylamino groups (e.g., methylamino, ethylamino, propylamino, dimethylamino, diethylamino, etc.), N-aromatic-ring amino groups (e.g., N-phenylamino, etc.), N-aromatic-ring-N-alkylamino groups (e.g., N-phenyl-N-methylamino, N-phenyl-N-ethylamino, N-phenyl-N-propylamino, N-phenyl-N-butylamino, N-phenyl-N-pentylamino, N-phenyl-N-hexylamino, etc.), acylamino groups, N-acyl-N-alkylamino groups, C1~6 alkoxy groups (e.g. , methoxy, ethoxy, propoxy, isopropoxy, n-butoxy, sec-butoxy, tert-butoxy, pentyloxy, hexyloxy, etc.), C3~7-cycloalkyl-C1~6-alkoxy groups (e.g., cyclohexylmethyloxy, cyclopentylethyloxy, etc.), C3-7-cycloalkyloxy groups (e.g., cyclohexyloxy, etc.), C7-15-aralkyloxy groups (e.g., benzyloxy, phenethyloxy, phenylpropyloxy, naphthylmethyloxy, naphthylethyloxy, etc.), a phenoxy group, C1~5-alkoxycarbonyl groups (e.g., methoxycarbonyl, ethoxycarbonyl, tert-butoxycarbonyl, etc.), C1~6-alkylcarbonyloxy groups (e.g., acetoxy, ethylcarbonyloxy, etc.), C1~4 alkylthio groups (e.g., methylthio, ethylthio, propylthio, butylthio, etc.), halogen atoms (e.g., fluorine, chlorine, bromine, iodine), alkylsulfonyl groups (e.g. , C1~4 alkylsulfonyl groups, such as methylsulfonyl, ethylsulfonyl, etc., and the like), aromatic-ring sulfonyl groups (e.g., C6~10 aromatic-ring sulfonyl groups, such as phenylsulfonyl, etc., and the like), acyl groups (e.g., C1~6 alkanoyl groups, such as formyl, acetyl, propanoyl, pivaloyl, etc., C6~10 aromatic-ring carbonyl groups, such as benzoyl, etc., and the like), an oxo group, an imino group (HN=), optionally substituted carbon-ring groups and optionally substituted heterocyclic groups, and the like, and these arbitrary substituents may be replaced in number of 1 to 4 at any substitutable positions.

On the above-described occasions, the acyl group in the acylamino and N-acyl-N-alkylamino groups as a substituent for the alkyl groups have the same meaning as defined for the acyl groups as a protective group in the "optionally protected hydroxyl groups", "optionally protected mercapto groups" and "optionally protected amino groups" as a substituent represented by R¹ to be described below. The "alkyl group" in the N-acyl-N-alkylamino groups may be exemplified by straight-chain or branched C1~20 alkyl groups, such as methyl, ethyl, n-propyl, isopropyl, n-butyl, isobutyl, sec-butyl, tert-butyl, pentyl,hexyl, heptyl, octyl, nonyl, decyl, undecyl, dodecyl, tridecyl, tetradecyl, pentadecyl, hexadecyl, heptadecyl, octadecyl, nonadecyl and icosyl groups, etc., and the like.

The carbon-ring group as a substituent for the alkyl group includes, for example, optionally, partially or fully saturated C3~15 monocyclic, bicyclic or tricyclic unsaturated carbon-rings, and the like. The optionally, partially or fully saturated C3~15 monocyclic, bicyclic or tricyclic unsaturated carbon rings may be exemplified by cyclopropane, cyclobutane, cyclopentane, cyclohexane, cycloheptane, cyclooctane, cyclononane, cyclodecane, cycloundecane, cyclododecane, cyclotridecane, cyclotetradecane, cyclopentadecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indane, naphthalene, dihydronaphthalene, tetrahydronaphthalene, perhydronaphthalene, heptalene, perhydroheptalene, biphenylene, as-indacene, s-indacene, acenaphthylene, acenaphthene, fluorene, phenalene, phenanthrene and anthracene rings, etc. In addition, the optionally, partially or fully saturated C3~15 monocyclic, bicyclic or tricyclic unsaturated carbon rings include spiro-linked bicyclic carbon rings and bridged bicyclic carbon rings, as well, and may be exemplified by spiro[4.4]nonane, spiro[4.5]decane, spiro[5.5]undecane, bicyclo[2.2.1]heptane, bicyclo[2.2.1]hept-2-en, bicyclo[3.1.1]heptane, bicyclo[3.1.1]hept-2-en, bicyclo[2.2.2]octane, bicyclo[2.2.2]oct-2-en, adamantane, noradamantane rings etc.

On the above-described occasions, the substituent for the carbon ring as a substituent for the alkyl group may be exemplified by C1~8 alkyl groups (e.g., methyl, ethyl, n-propyl, isopropyl, n-butyl, isobutyl, sec-butyl, tert-butyl, pentyl, hexyl, heptyl, octyl, etc.), a hydroxy group, an amino group, a carboxyl group, a nitro group, mono- or di-C1~6-alkylamino groups (e.g., methylamino, ethylamino, propylamino, dimethylamino, diethylamino, etc.), C1~6-alkoxy groups (e.g., methoxy, ethoxy, propoxy, isopropoxy, n-butoxy, sec-butoxy, tert-butoxy, pentyloxy, hexyloxy, etc.), C1~6 alkoxycarbonyl groups (e.g., methoxycarbonyl, ethoxycarbonyl, tert-butoxycarbonyl, etc.), C1~6-alkylcarbonyloxy groups (e.g., acetoxy, ethylcarbonyloxy, etc.), Cl-4-alkylthio groups (e.g., methylthio, ethylthio, propylthio, butylthio, etc.), halogen atoms (fluorine, chlorine, bromine, iodine), trihalomethyl groups (e.g., trifluoromethyl, etc.), and the like, and these arbitrary substituents may be replaced in number of 1 to 4 at any substitutable positions.

The heterocycle as a substituent for the alkyl group includes, for example, optionally, partially or fully saturated 3-15-membered monocyclic, bicyclic or tricyclic unsaturated heterocycles containing 1 to 5 hetero-atoms selected from oxygen, nitrogen and sulfur atoms, and the like. Among the optionally, partially or fully saturated 3-15-membered monocyclic, bicyclic or tricyclic unsaturated heterocycles containing 1 to 5 hetero-atoms selected from oxygen, nitrogen and sulfur atoms, the 3 to 15-membered monocyclic, bicyclic or tricyclic unsaturated heterocycles containing 1 to 5 hetero-atoms selected from oxygen, nitrogen and sulfur atoms may be exemplified by pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolidine, purine, phthaladine, pteridine, naphthylidine, quinoxaline, quinazoline, cinnoline, benzoxazole, benzothiazole, benzoimidazole, chromene, benzoxepine, benzoxazepine, benzoxadiazepine, benzothiepine, benzothiazepine, benzothiadiazepine, benzoazepine, benzodiazepine, benzofurazan, benzothiadiazole, benzotriazole, carbazole, β-carboline, acridine, phenazine, dibenzofuran, xanthene, dibenzothiophene, phenothiazine, phenoxazine, phenoxathiine, thianthrene, phenanthlidine, phenathroline, perimidine rings, etc.

Among the optionally, partially or fully saturated 3-15-membered monocyclic, bicyclic or tricyclic unsaturated heterocycles containing 1 to 5 hetero-atoms selected from oxygen, nitrogen and sulfur atoms, the partially or fully saturated 3-15-membered monocyclic, bicyclic or tricyclic unsaturated heterocycles containing 1 to 5 hetero-atoms selected from oxygen, nitrogen and sulfur atoms may be exemplified by aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxyrane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiopyran, tetrahydrothiopyran, dihydrothiepine, tetrahydrothiepine, perhydrothiepine, dihydroxazole, tetrahydroxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindolin, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthylidine, tetrahydronaphthylidine, perhydronaphthylidine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine. dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzoazepine, tetrahydrobenzoazepine, dihdyrobenzodiazepine, tetrahydrobenzodiazepine, benzoxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, dioxaindane, benzodioxane, chroman, benzodithiolane, benzodithiane rings, etc.

On the above-described occasions, the substituent for the heterocycles as a substituent for the alkyl group may be exemplified by C1~8 alkyl groups (e.g., methyl, ethyl, n-propyl, isopropyl, n-butyl, isobutyl, sec-butyl, tert-butyl, pentyl, hexyl, heptyl, octyl, etc.), a hydroxyl group, an amino group, a carboxyl group, a nitro group, mono- or di-C1~6 alkylamino groups (e.g., methylamino, ethylamino, propylamino, dimethylamino, diethylamino, etc.), C1~6 alkoxy groups (e.g., methoxy, ethoxy, propoxy, isopropoxy, n-butoxy, sec-butoxy, tert-butoxy, pentyloxy, hexyloxy, etc.), C1~6 alkoxycarbonyl groups (e.g., methoxycarbonyl, ethoxycarbonyl, tert-butoxycarbonyl, etc.), C1~6 alkylcarbonyloxy groups (e.g., acetoxy, ethylcarbonyloxy, etc.), C1~4 alkylthio groups (e.g., methylthio, ethylthio, propylthio, butylthio, etc.), halogen atoms (fluorine, chlorine, bromine, iodine), and the like, and these arbitrary substituents may be replaced in number of 1 to 4 at any substitutable positions.

The alkenyl group in the "optionally substituted alkenyl groups" as a substituent represented by R¹ may be exemplified by straight-chain or branched C2~20 alkenyl groups, such as ethenyl, propenyl, butenyl, pentenyl, hexenyl, etc., and the like, whereby the substituent for the alkenyl group has the same meaning as defined above for the substituent in the "optionally substituted alkyl groups".

The alkynyl group in the "optionally substituted alkynyl group" as a substituent represented by R¹ may be exemplified by straight-chain or branched C2~20 alkynyl groups, such as ethynyl, propynyl, butynyl, pentynyl, hexynyl, etc., and the like, whereby the substituent for the alkynyl groups has the same meaning as defined above for the "optionally substituted alkyl group".

The carbon ring in the "optionally substituted carbon ring groups" as a substituent represented by R¹ includes, for example, optionally, partially or fully saturated C3~15 monocyclic, bicyclic or tricyclic unsaturated carbon rings, etc. The optionally, partially or fully saturated C3~15 monocyclic, bicyclic or tricyclic unsaturated carbon ring may be exemplified by cyclopropane, cyclobutane, cyclopentane, cyclohexane, cycloheptane, cyclooctane, cyclononane, cyclodecane, cycloundecane, cyclododecane, cyclotridecane, cyclotetradecane, cyclopentadecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indane, naphthalene, dihydronaphthalene, tetrahydronaphthalene, perhydronaphthalene, heptalene, perhydroheptalene, biphenylene, as-indacene, s-indacene, acenaphthylene, acenaphthene, fluorene, phenalene, phenanthrene, anthracene rings and the like.

Also, the optionally partially or fully saturated C3~15 monocyclic, bicyclic or tricyclic unsaturated carbon rings comprehend spiro-linked bicyclic carbon rings and bridged bicyclic carbon rings, which may be exemplified by spiro[4.4]nonane, spiro[4.5]decane, spiro[5.5]undecane, bicyclo[2.2.1]heptane, bicyclo[2.2.1]hept-2-en, bicyclo[3.1.1]heptane, bicyclo[3.1.1]hept-2-en, bicyclo[2.2.2]octane, bicyclo[2.2.2]oct-2-en, adamantane, noradamantane rings, and the like.

On the above-described occasions, the substituent for the carbon ring include, for example, C1~4-alkyl groups (e.g., methyl, ethyl, propyl, butyl, etc.), C2-4-alkenyl groups (e.g., ethenyl, propenyl, butenyl, etc.), C2-4-alkynyl groups (e.g., ethynyl, propynyl, butynyl, etc.), a hydroxyl group, C1~4 alkoxy groups (e.g., methoxy, ethoxy, propoxy, butoxy, etc.), C1~6-alkoxycarbonyl groups (e.g., methoxycarbonyl, ethoxycarbonyl, tert-butoxycarbonyl, etc.), a mercapto group, C1~4-alkylthio groups (e.g., methylthio, ethylthio, propylthio, butylthio, etc.), an amino group, mono- or di-C1~4-alkylamino groups (e.g., methylamino, ethylamino, propylamino, dimethylamino, diethylamino, etc.), amino-C1~4 alkyl groups (e.g., aminomethyl, etc.), mono- or di-C1~4-alkylamino-C1~4-alkyl groups (e.g., dimethylaminomethyl, methylaminomethyl, etc.), an imino group, alkyl groups substituted with an imino group(s) (e.g., ethaneimidoyl, etc.), an oxo group, halogen atoms (fluorine, chlorine, bromine, iodine), trihalomethyl groups (e.g., trifluoromethyl, etc.), trihalomethoxy groups (e.g., trifluoromethoxy, etc.), trihalomethylthio groups (e.g., trifluoromethylthio, etc.), dihalomethylthio groups (e.g., difluoromethylthio, etc.), optionally substituted cyclic groups, a cyano group, C1~4-alkylsulfonyl groups (e.g., methylsulfonyl, ethylsulfonyl, etc.), a nitro group, mono- or di-C1~4-alkyldioxolanes (e.g., 2,2-dimethyldioxolane, etc.) and the like, and these arbitrary substituents may be replaced in number of 1 to 4 at any substitutable positions.

On the above-described occasions, the "optionally substituted cyclic group" as a substituent for the carbon rings in the "optionally substituted carbon ring groups" as a substituent has the same meaning as defined above for the "carbon ring" in the optionally substituted carbon rings which are denoted as a substituent as the substituent represented by R¹ or for the "heterocycle" in the optionally substituted heterocyclic groups.

The substituent in the "optionally substituted cyclic groups" as a substituent for the carbon rings in the "optionally substituted carbon ring groups" as a substituent represented by R¹ is understood to have the same meaning as defined above for the substituent for the carbon rings as a substituent for the "optionally substituted alkyl groups" as a substituent, and these arbitrary substituents may be replaced in number of 1 to 4 at any substitutable positions.

The heterocycle in the "optionally substituted heterocyclic groups" as a substituent represented by R¹ includes, for example, optionally, partially or fully saturated 3-15-membered monocyclic, bicyclic or tricyclic unsaturated heterocycles containing 1 to 5 hetero-atoms selected from oxygen, nitrogen and sulfur atoms, and the like. Among the optionally, partially or fully saturated 3-15-membered monocyclic, bicyclic or tricyclic unsaturated heterocycles containing 1 to 5 hetero-atoms selected from oxygen, nitrogen and sulfur atoms, the 3-15-membered monocyclic, bicyclic or tricyclic unsaturated heterocycles containing 1 to 5 hetero-atoms selected from oxygen, nitrogen and sulfur atoms are exemplified by pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolidine, purine, phthalazine, pteridine, naphthylidine, quinoxaline, quinazoline, cinnoline, benzoxazole, benzothiazole, benzoimidazole, chromene, benzoxepine, benzoxazepine, benzoxadiazepine, benzothiepine, benzothiazepine, benzothiadiazepine, benzoazepine, benzodiazepine, benzofurazan, benzothiadiazole, benzotriazole, carbazole, β-carboline, acridine, phenazine, dibenzofuran, xanthene, dibenzothiophene, phenothiazine, phenoxazine, phenoxathiin, thianthrene, phenanthridine, phenanthroline, perimidine, oxazolopyridine, thiazolopyrimidine, imidazopyridine rings and the like.

Among the optionally partially or fully saturated 3-15-membered monocyclic, bicyclic or tricyclic unsaturated heterocycles containing 1 to 5 hetero-atoms selected from oxygen, nitrogen and sulfur atoms, the partially or fully saturated 3-15-membered monocyclic, bicyclic or tricyclic unsaturated heterocycles containing 1 to 5 hetero-atoms selected from oxygen, nitrogen and sulfur atoms are exemplified by aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazol, tetrahydroisoxazol (isoxazolidine), dihydrothiazole, tetrahydrothiazole (thiazolidine), dihydroisothiazole, tetrahydroisothiazole (isothiazolidine), dihdyrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthylidine, tetrahydronaphthylidine, perhydronaphthylidine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, dioxaindane, benzodioxane, chromane, benzodithiolane, benzodithiane, tetrahydrooxazolopyridine, tetrahydrothiazolopyrimidine, oxazinane, oxazepane rings, and the like.

On the above-described occasions, it is noted that the substituent for the heterocycles has the same meanings as defined above for the substituents in the "optionally substituted carbon ring groups", and these arbitrary substituents may be replaced in number of 1 to 4 at any substitutable positions.

The protective group in the "optionally protected hydroxy groups", "optionally protected mercapto groups" and "optionally protected amino groups" as a substituent represented by R¹ includes, for example, optionally substituted alkyl groups (as defined above for the "optionally substituted alkyl groups"), optionally substituted carbon ring groups (as defined above for the "optionally substituted carbon ring groups" as a substituent represented by R¹), optionally substituted heterocyclic groups (as defined above for the "optionally substituted heterocyclic groups" as a substituent represented by R¹), alkylsulfonyl groups (e.g., C1~4 alkylsulfonyl groups, such as methylsulfonyl, ethylsulfonyl, etc., and the like), aromatic-ring -sulfonyl groups (e.g., C6~10-aromatic-ring sulfonyl groups, such as phenylsulfonyl group, etc., and the like), acyl groups, and the like, whereby the acyl group may be exemplified by (1) optionally substituted alkylcarbonyl groups, (2) optionally substituted alkenylcarbonyl groups, (3) optionally substituted alkynylcarbonyl groups, (4) optionally substituted carbon-ring carbonyl groups, and (5) optionally substituted heterocyclic carbonyl groups, and these arbitrary substitutes may be replaced in number of 1 to 4 at any substitutable positions. The optionally substituted alkyl in the "optionally substituted alkylcarbonyl groups" has the same meaning as defined above for the "optionally substituted alkyl groups". The optionally substituted alkenyl in the "optionally substituted alkenylcarbonyl groups" has the same meaning as defined above for the "optionally substituted alkenyl groups". The optionally substituted alkynyl in the "optionally substituted alkynylcarbonyl groups" has the same meaning as defined above for the "optionally substituted alkynyl groups". The optionally substituted carbon-ring group in the "optionally substituted carbon-ring carbonyl groups" has the same meaning as defined above for the "optionally substituted carbon-ring groups" as a substitute for the "optionally substituted alkyl groups" represented by R¹. The optionally substituted heterocyclic group in the "optionally substituted heterocyclic carbonyl groups" has the same meaning as defined above for the "optionally substituted heterocyclic groups" as a substituent for the "optionally substituted heterocyclic carbonyl groups" represented by R¹.

As used herein, the ester-linkage containing group represents groups containing within the group:

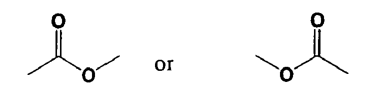

As used herein, the ester-linkage containing group as represented by R⁶ may be exemplified by C1~15 alkoxycarbonyl groups, alkyl groups substituted with C1~15 alkoxycarbonyl groups, alkenyl groups substituted with C1~15 alkoxycarbonyl groups, alkynyl groups substituted with C1~15 alkoxycarbonyl groups, carbon-ring groups substituted with C1~15 alkoxycarbonyl groups, heterocyclic groups substituted with C1~15 alkoxycarbonyl groups, C1~15 acyloxy groups, alkyl groups substituted with C1~15 acyloxy groups, alkenyl groups substituted with C1~15 acyloxy groups, alkynyl groups substituted with C1~15 acyloxy groups, carbon-ring groups substituted with C1~15 acyloxy groups, heterocyclic groups substituted with C1~15 acyloxy groups, and the like. The C1~15 alkoxycarbonyl group includes, for example, methoxycarbonyl, ethoxycarbonyl, n-propoxycarbonyl and isopropoxycarbonyl groups, etc., with a methoxycarbonyl or ethoxycarbonyl group being preferred. The alkyl group in the alkyl groups substituted with C1-15 alkoxycarbonyl groups may be substituted, and has the same meaning as defined above for the "optionally substituted alkyl groups" represented by R¹. The alkenyl group in the alkenyl groups substituted with C1-15 alkoxycarbonyl groups may be substituted, and has the same meaning as defined above for the "optionally substituted alkenyl groups" represented by R¹. The alkynyl group in the alkynyl groups substituted with C1-15 alkoxycarbonyl groups may be substituted, and has the same meaning as defined above for the "optionally substituted alkynyl groups" represented by R¹.

The carbon-ring group in the carbon-ring groups substituted with C1~15 alkoxycarbonyl groups may be substituted, and has the same meaning as defined above for the "optionally substituted carbon-ring groups" represented by R¹. The heterocyclic group in the heterocyclic groups substituted with C1~15 alkoxycarbonyl groups may be substituted, and has the same meaning as defined above for the "optionally substituted heterocyclic groups" represented by R¹. The C1~15 acyloxy group may be exemplified by formyl, acetyl and propionyl groups, etc. , with the acetyl group being preferable. The alkyl group in the alkyl groups substituted with C1~15 acyloxy groups may be substituted, and has the same meaning as defined above for the "optionally substituted alkyl groups" represented by R¹. The alkenyl group in the alkenyl groups substituted with C1~15 acyloxy groups may be substituted, and has the same meaning as defined above for the "optionally substituted alkenyl groups" represented by R¹. The alkynyl group in the alkynyl groups substituted with C1~15 acyloxy groups may be substituted, and has the same meaning as defined above for the "optionally substituted alkynyl groups" represented by R¹. The carbon-ring group in the carbon-ring groups substituted with C1~15 acyloxy groups may be substituted, and has the same meaning as defined above for the "optionally substituted carbon-ring groups" represented by R¹. The heterocyclic group in the heterocyclic groups substituted with C1~15 acyloxy groups may be substituted, and has the same meaning as defined above for the "optionally substituted heterocyclic groups" represented by R¹.

As used herein, the "spacer of 1 to 5 in number of atoms for the main chain" in the optionally substituted spacer of 1 to 5 in number of atoms for the main chain as represented by Y is understood to denote a spacer having 1 to 5 atoms linked together in the main chain, wherein the "number of atoms for the main chain" is understood to be determined through counting in such a manner as may minimize the number of atoms for the main chain. The "spacer of 1 to 5 in number of atoms for the main chain" includes, for example, divalent groups selected from methylene groups (-CH₂-) which may have 1 or 2 substituents , and optionally substituted nitrogen atoms (-NH-), -CO-, -O-, -S-, -SO- and -SO₂-, and the like. In such definitions, the substituents for the methylene group and nitrogen atom denote C1~8 alkyl groups (e.g., methyl, ethyl, n-propyl, isopropyl, n-butyl, isobutyl, sec-butyl, tert-butyl, pentyl, hexyl, heptyl, octyl, etc.), a hydroxy group, an amino group, a carboxyl group, a nitro group, mono- or di-C1~6-alkylamino groups (e.g., methylamino, ethylamino, propylamino, dimethylamino, diethylamino, etc.), C1~6 alkoxy groups (e.g., methoxy, ethoxy, propoxy, isopropoxy, n-butoxy, sec-butoxy, tert-butoxy, pentyloxy, hexyloxy, etc.), C1~6 alkoxycarbonyl groups (e.g., methoxycarbonyl, ethoxycarbonyl, tert-butoxycarbonyl, etc.), C1~6 alkylcarbonyloxy groups (e.g. , acetoxy, ethylcarbonyloxy, etc.), C1~4 alkylthio groups (e.g., methylthio, ethylthio, propylthio, butylthio, etc.), halogen atoms (fluorine, chlorine, bromine, iodine), trihalomethyl groups (e.g., trifluoromethyl, etc.), or two substituents for the methylene group are taken together to represent (i) C2~5 alkylene groups (which alkylene groups may substituted with substituents) which may be replaced with one oxygen, nitrogen or sulfur atom or (ii) optionally substituted C1~6 alkylidene groups.

Specific examples of the "spacer of 1 to 5 in number of atoms for the main chain" include -CR¹⁰¹R¹⁰²-, -NR¹⁰³-, -CO-, -O-, -S-, -SO₂-, -OCR¹⁰¹R¹⁰²-, -NR¹⁰³CO-, -CONR¹⁰³-, -NR¹⁰³COCR¹⁰¹R¹⁰²-, -CONR¹⁰³CR¹⁰¹R¹⁰²-, -C(R¹⁰¹)=C(R¹⁰²)-, -C=C-, -CR¹⁰¹R¹⁰²CO-, -COCR¹⁰¹R¹⁰²-, -CR¹⁰¹R¹⁰²CR¹⁰⁴R¹⁰⁵-, -C(R¹⁰¹)=C(R¹⁰²)SO₂-, -CO-OCR¹⁰¹R¹⁰²-CONR¹⁰³- (wherein R¹⁰¹ to R¹⁰³ each independently denote a hydrogen atom, C1-8 alkyl groups (e.g., methyl, ethyl, n-propyl, isopropyl, n-butyl, isobutyl, sec-butyl, tert-butyl, pentyl, hexyl heptyl, octyl, etc.), a hydroxy group, an amino group, a carboxyl group, a nitro group, mono- or di-C1~6-alkylamino groups (e.g., methylamino, ethylamino, propylamino, dimethylamino, diethylamino, etc.), C1~6-alkoxy groups (e.g., methoxy, ethoxy, propoxy, isopropoxy, n-butoxy, sec-butoxy, tert-butoxy, pentyloxy, hexyloxy, etc.), C1~6-alkoxycarbonyl groups (e.g., methoxycarbonyl, ethoxycarbonyl, tert-butoxycarbonyl, etc.), C1~6-alkylcarbonyloxy groups (e.g., acetoxy, ethylcarbonyloxy, etc.), alkyl groups substituted with C1~15- alkoxycarbonyl groups, alkenyl groups substituted with C1~15- alkoxycarbonyl groups, alkynyl groups substituted with C1~15- alkoxycarbonyl groups, C1~4-alkylthio groups (e.g., methylthio, ethylthio, propylthio, butylthio, etc.), halogen atoms (fluorine, chlorine, bromine, iodine) or trihalomethyl groups (e.g., trifluoromethyl, etc.), or R¹⁰¹ and R¹⁰² or R¹⁰³ and R¹⁰⁴ are taken together to represent (i) C2-5-alkyl groups (which alkyl groups may be substituted with substituents) which may be replaced with one oxygen, nitrogen or sulfur atom or (ii) optionally substituted C1~6-alkylidene groups).

The C2-5-alkylene groups which may be replaced with one oxygen, nitrogen or sulfur atom may be exemplified by C2~5 straight-chain or branched alkylene groups, such as ethylene, propylene, isopropylene, butylene, isobutylene and pentylene groups, etc. , C2-5-alkylene groups, such as ethylene, propylene, isopropylene, butylene, isobutylene and pentylene groups, which have one of their carbon atoms replaced with an oxygen, nitrogen or sulfur atom, and the like, whereby the remaining linkage of the nitrogen atom bonds to a hydrogen atom, C1~6-alkyl group, C2~6 acyl groups or C1~6 alkoxycarbonyl groups (e.g., methoxycarbonyl, ethoxycarbonyl, tert-butoxycarbonyl, etc.). The C2-5-alkylene group may be substituted with a substituent(s). On the above-described occasions, the substituent may be exemplified by C1~8-alkyl groups (e.g., methyl, ethyl, n-propyl, isopropyl, n-butyl, isobutyl, sec-butyl, tert-butyl, pentyl, hexyl, heptyl, octyl, etc.), a hydroxy group, an amino group, a carboxyl group, a nitro group, mono- or di-C1~6-alkylamino groups (e.g., methylamino, ethylamino, propylamino, dimethylamino, diethylamino, etc.), C1~6-alkoxy groups (e.g., methoxy, ethoxy, propoxy, isopropoxy, n-butoxy, sec-butoxy, tert-butoxy, pentyloxy, hexyloxy, etc.), C1~6-alkopxycarbonyl groups (e.g., methoxycarbonyl, ethoxycarbonyl, tert-butoxycarbonyl, etc.), C1~6-alkylcarbonylxoy groups (e.g., acetoxy, ethylcarbonyloxy, etc.), C1~4-alkylthio groups (e.g., methylthio, ethylthio, propylthio, butylthio, etc.), halogen atoms (fluorine, chlorine, bromine, iodine), and the like, and such arbitrary substituents may be replaced in number of 1 to 4 at any substitutable positions. The specific examples of the said C2~5-alkylene group include -(CH₂)₂-, -(CH₂)₃-, -(CH₂)₄-, -(CH₂)₅-, -O-CH₂-, -O-(CH₂)₂-, -O-(CH₂)₃-, -O-(CH₂)₄-, -CH₂-O-CH₂-, -CH₂-O-(CH₂)₂-, -CH₂-O-(CH₂)₃-, -(CH₂)₂-O-(CH₂)₂-, -NH-CH₂-, -NH-(CH₂)₂- , -NH-(CH₂)₃-, -NH-(CH₂)₄-, -CH₂-NH-CH₂-, -CH₂-NH-(CH₂)₂-, -CH₂-NH-(CH₂)₃-, -(CH₂)₂-NH-(CH₂)₂-, -N(CH₃)-CH₂-, -N(CH₃)-(CH₂)₂-, -N(CH₃)-(CH₂)₃-, -N(CH₃)-(CH₂)₄-, -CH₂-N(CH₃)-CH₂-, -CH₂-N(CH₃)-(CH₂)₂-, -CH₂-N(CH₃)-(CH₂)₃-, -(CH₂)₂-N(CH₃)-(CH₂)₂-, -S-CH₂-, -S-(CH₂)₂-, -S-(CH₂)₃-, -S-(CH₂)₄-, -CH₂-S-CH₂-, -CH₂-S-(CH₂)₂-, -CH₂-S-(CH₂)₃-, -(CH₂)₂-S-(CH₂)₂- groups, and the like.

The C1~6-alkylidene group in the "optionally substituted alkylidene groups" may be exemplified by methylidene, ethylidene, propylidene, butylidene, pentylidene, hexylidene, etc., whereby the substituent for the "optionally substituted alkylidene groups" include, for example, a hydroxy group, an amino group, a carboxyl group, a nitro group, an azido group, mono- or di-C1~6-alkylamino groups (e.g., methylamino, ethylamino, propylamino, dimethylamino, diethylamino, etc.), N-aromatic-ring amino groups (e.g., N-phenylamino, etc.), N-aromatic-ring-N-alkylamino groups (e.g., N-phenyl-N-methylamino, N-phenyl-N-ethylamino, N-phenyl-N-propylamino, N-phenyl-N-butylamino, N-phenyl-N-pentylamino, N-phenyl-N-hexylamino, etc.), C1~6-alkoxy groups (e.g., methoxy, ethoxy, propoxy, isopropoxy, n-butoxy, sec-butoxy, tert-butoxy, pentyloxy, hexyloxy, etc.), C3~7-cycloalkyl-C1~6-alkoxy groups (e.g., cyclohexylmethyloxy, cyclopentylmethyloxy, etc.), C3-7-cycloalkyloxy groups (e.g., cyclohexyloxy, etc.), C7~15-aralkyloxy groups (e.g., benzyloxy, phenethyloxy, phenylpropyloxy, naphthylmethyloxy, naphthylethyloxy, etc.), a phenoxy group, C1~6-alkoxycarbonyl groups (e.g., methoxycarbonyl, ethoxycarbonyl, tert-butoxycarbonyl, etc.), C1~6-alkylcarbonyloxy groups (e.g., acetoxy, ethylcarbonyloxy, etc.), C1~4-alkylthio groups (e.g., methylthio, ethylthio, propylthio, butylthio, etc.), halogen atoms (fluorine, chlorine, bromine, iodine), alkylsulfonyl groups (e.g., C1~4-alkylsulfonyl, such as methylsulfonyl, ethylsulfonyl, etc. and the like), aromatic sulfonyl groups (e.g., C6~10-aroamtic sulfonyl groups, such as phenylsulfonyl, etc. and the like), acyl groups (e.g., C1~6-alkanoyl groups, such as formyl, acetyl, propanoyl, pivaloyl, etc., C6~10-aromatic-ring carbonyl groups, such as benzoyl, etc., and the like), and the like, and these arbitrary substituents may be replaced in number of 1 to 4 at any substitutable positions.

As used herein, the "spacer of 1 to 5 in number of atoms for the main chain" in the optionally substituted spacer of 1 to 5 in number of atoms for the main chain as represented by Z has the same meaning as defined above for the "spacer of 1 to 5 in number of atoms for the main chain" represented by Y.

As used herein, the cyclic group in the "cyclic group which may be substituted with a substitutent(s)" as represented by ring 1 includes, for example, optionally, partially or fully saturated C3~15 monocyclic, bicyclic or tricyclic unsaturated carbon-rings, or optionally partially or fully saturated 3-15-membered monocyclic, bicyclic or tricyclic unsaturated heterocycles containing 1 to 5 hetero-atoms selected from oxygen, nitrogen and sulfur atoms, and the like.

The optionally partially or fully saturated C3~15 monocyclic, bicyclic or tricyclic unsaturated carbon-ring represented by ring 1 may be exemplified by cyclopropane, cyclobutane, cyclopentane, cyclohexane, cycloheptane, cyclooctane, cyclononane, cyclodecane, cycloundecane, cyclododecane, cyclotridecane, cyclotetradecane, cyclopentadecane, cyclopentene, cyclohexene, cycloheptene, cyclooctene, cyclopentadiene, cyclohexadiene, cycloheptadiene, cyclooctadiene, benzene, pentalane, perhydropentalane, azulene, perhydroazulene, indene, perhydroindene, indane, naphthalene, dihydronaphthalene, tetrahydronaphthalene, perhydronaphthalene, heptalene, perhydroheptalene, biphenylene, as-indacene, s-indacene, acenaphthylene, acenaphthene, fluorene, phenalene, phenanthrene and anthracene rings, and the like.

The optionally partially or fully saturated C3~15 monocyclic, bicyclic or tricyclic unsaturated carbon-ring also comprehends spiro-linked bicyclic carbon rings and bridged bicyclic carbon rings, which may be exemplified by spiro[4.4]nonane, spiro[4.5]decane, spiro[5.5]undecane, bicyclo[2.2.1]heptane, bicyclo[2.2.1]hept-2-en, bicyclo[3.3.1]heptane, bicyclo[3.1.1]hept-3-en, bicyclo[2.2.2]octane, bicyclo[2.2.2]oct-2-en, adamantane and noradamantane rings, etc.

On the above-mentioned occasions, the substituent for the carbon ring may be exemplified by C1~4-alkyl groups (e.g., methyl, ethyl, propyl, butyl, etc.), C2~4-alkenyl groups (e.g., ethenyl, propenyl, butenyl, etc.), C2-4-alkynyl groups (e.g., ethynyl, propynyl, butynyl, etc.), a hydroxy group, C1~4-alkoxy groups (e.g., methoxy, ethoxy, propoxy, butoxy, etc.), C1~6-alkoxycarbnonyl groups (e.g., methoxycarbonyl, ethoxycarbonyl, tert-butoxycarbonyl, etc.), a mercapto group, C1~4-alkylthio groups (e.g., methylthio, ethylthio, propylthio, butylthio, etc.), an amino group, mono- or di-C1~4-alkylamino groups (e.g., methylamino, ethylamino, propylamino, dimethylamino, diethylamino, etc.), halogen atoms (fluorine, chlorine, bromine, iodine), trihalomethyl groups (e.g., trifluoromethyl, etc.), trihalomethoxy groups (e.g., trifluoromethoxy, etc.), trihalomethylthio groups (e.g., trifluoromethylthio, etc.), dihalomethylthio groups (e.g., difluoromethylthio, etc.), a cyano group, a nitro group, and the like, and these arbitrary substituents may be replaced in number of 1 to 4 at any substitutable positions.

Among the optionally, partially or fully saturated 3- to 15-membered monocyclic, bicyclic or tricyclic unsaturated heterocycles containing 1 to 5 hetero-atoms selected from oxygen, nitrogen and sulfur atoms as represented by ring 1, the 3- to 15-membered monocyclic, bicyclic or tricyclic unsaturated heterocycle containing 1 to 5 hetero-atoms selected from oxygen, nitrogen and sulfur atoms may be exemplified by pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthylidine, quinoxaline, quinazoline, cinnoline, benzoxazole, benzothiazole, benzimidazole, chromen, benzoxepine, benzoxazepine, benzoxadiazepine, benzothiepine, benzothiazepine, benzothiadiazepine, benzoazepine, benzodiazepine, benzofurazan, benzothiadiazole, benzotriazole, carbazole, β-carboline, acridine, phenazine, dibenzofuran, xanthene, dibenzothiophene, phenothiazine, phenoxazine, phenoxathiin, thianthrene, phenanthridine, phenanthroline, perimidine rings, etc.

Among the optionally, partially or fully saturated 3 -15-membered monocyclic, bicyclic or tricyclic unsaturated heterocycles containing 1 to 5 hetero-atoms selected from oxygen, nitrogen and sulfur atoms as represented by ring 1, the partially or fully saturated 3~15-membered monocyclic, bicyclic or tricyclic unsaturated heterocycle containing 1 to 5 hetero-atoms selected from oxygen, nitrogen and sulfur atoms may be exemplified by aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihdyrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihdyrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthylidine, tetrahydronaphthylidine, perhydronaphthylidine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzoazepine, tetrahydrobenzoazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, dioxaindane, benzodioxane, chroman, benzodithiolane, benzodithiane rings, etc.

On the above-mentioned occasions, the substituent for the heterocycles has the same meaning as defined above for the "optionally partially or fully saturated C3~15 monocyclic, bicyclic or tricyclic unsaturated carbon-rings" represented by ring 1, and these arbitrary substituents may be replaced in number of 1 to 4 at any substitutable positions.

As used herein, the substituent represented by R² includes, for example, (1) optionally substituted alkyl groups, (2) optionally substituted alkenyl groups, (3) optionally substituted alkynyl groups, (4) optionally substituted carbon-ring groups, (5) optionally substituted heterocyclic groups, (6) optionally protected hydroxy groups, (7) optionally protected mercapto groups, (8) optionally protected amino groups, (9) a nitro group, (10) a cyano group or (11) an amidino group, etc.

As used herein, (1) optionally substituted alkyl groups, (2) optionally substituted alkenyl groups, (3) optionally substituted alkynyl groups, (4) optionally substituted carbon-ring groups, (5) optionally substituted heterocyclic groups, (6) optionally protected hydroxyl groups, (7) optionally protected mercapto groups and (8) optionally protected amino groups, as represented by R², have the same meanings as defined above for those represented by R¹.

As used herein, the substituent represented by R³ includes, for example, (1) optionally substituted alkyl groups, (2) optionally substituted alkenyl groups, (3) optionally substituted alkynyl groups, (4) optionally substituted carbon-ring groups, (5) optionally substituted heterocyclic groups, (6) optionally protected hydroxy groups, (7) optionally protected mercapto groups, (8) optionally protected amino groups, (9) optionally substituted carbamoyl groups, (10) optionally substituted sulfamoyl groups, (11) a carboxyl group, (12) alkoxycarbonyl groups (e.g., C1~6-alkoxycarbonyl groups, such as methoxycarbonyl, ethoxycarbonyl, tert-butoxycarbonyl, etc., and the like), (13) a sulfo group (-SO₃H), (14) a sulfino group, (15) a phosphono group, (16) a nitro group, (17) a cyano group, (18) an amidino group, (19) (C1~6-alkoxyimino)methyl groups (e.g., a (methoxyimino)methyl group, etc.), 20 a -B(OH)₂ group, (21) halogen atoms (e.g., fluorine, chlorine, bromine, iodine, etc.), (22) alkylsulfinyl groups (e.g., C1~4- alkylsulfinyl groups, such as methylsulfinyl, ethylsulfinyl, etc. and the like), (23) aromatic-ring sulfinyl groups (e.g., C6~10 aromatic-ring sulfinyl groups, such as phenylsulfinyl, etc. and the like), (24) alkylsulfonyl groups (e.g., C1~4 alkylsulfonyl groups, such as methylsulfonyl, ethylsulfonyl, etc. and the like), (25) aromatic-ring sulfonyl groups (e.g., C6~10 aromatic-ring sulfonyl groups, such as phenylsulfonyl, etc. and the like), (26) acyl groups (e.g., C1~6 alkanoyl groups, such as formyl, acetyl, propanoyl, pivaloyl, etc., C6~10 aromatic-ring carbonyl groups, such as benzoyl, etc., and the like), (27) groups represented by the formula: 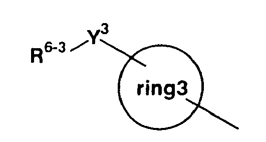 (wherein all the symbols are as defined above), and the like.

As used herein, (1) optionally substituted alkyl groups, (2) optionally substituted alkenyl groups, (3) optionally substituted alkynyl groups, (4) optionally substituted carbon-ring groups, (5) optionally substituted heterocyclic groups, (6) optionally protected hydroxyl groups, (7) optionally protected mercapto groups and (8) optionally protected amino groups, as represented by R³, have the same meanings as defined above for those represented by R¹.

The "optionally substituted carbamoyl group" as a substituent represented by R³ includes, for example, an unsubstituted carbamoyl group, N-mono-C1~4-alkylcarbamoyl (e.g., N-methylcarbamoyl, N-ethylcarbamoyl, N-propylcarbamoyl, N-isopropylcarbamoyl, N-butylcarbamoyl, etc.), N,N-di-C1~4-alkylcarbamoyl (e.g., N,N-dimethylcarbamoyl, N,N-diethylcarbamoyl, N,N-dipropylcarbamoyl, N,N-dibutylcarbamoyl, etc.), 1-piperidylcarbonyl, and the like.

The "optionally substituted sulfamoyl group" as a substituent represented by R³ includes, for example, an unsubstituted sulfamoyl group, N-mono-C1~4-alkylsulfamoyl (e.g., N-methylsulfamoyl, N-ethylsulfamoyl, N-propylsulfamoyl, N-isopropylsulfamoyl, N-butylsulfamoyl, etc.), N,N-di-C1~4-alkylsulfamoyl (e.g., N,N-dimethylsulfamoyl, N,N-diethylsulfamoyl, N,N-dipropylsulfamoyl, N,N-dibutylsulfamoyl, etc.), and the like.

As used herein, the ester-linkage containing group represented by R⁶⁻³ has the same meaning as defined above for the ester-linkage containing groups represented by R⁶.

As used herein, the spacer of 1 to 5 in number of atoms for the main chain which may be substituted with substituents, as represented by Y³, has the same meaning as defined above for the spacer of 1 to 5 in number of atoms for the main chain which may be substituted with substituents, as represented by Y.

As used herein, the "cyclic group which may be substituted with substituents", as represented by ring3, is understood to have the same meaning as defined above for the cyclic groups which may be substituted with substituents, as represented by ring1.

As used herein, the substituent represented by R⁴ includes, for example, (1) optionally substituted alkyl groups, (2) optionally substituted alkenyl groups, (3) optionally substituted alkynyl groups, (4) optionally substituted carbon-ring groups, (5) optionally substituted heterocyclic groups, (6) optionally protected hydroxy groups, (7) optionally protected mercapto groups, (8) optionally protected amino groups, (9) optionally substituted carbamoyl groups, (10) optionally substituted sulfamoyl groups, (11) a carboxyl group, (12) alkoxycarbonyl groups (e.g., C1~6-alkoxycarbonyl groups, such as methoxycarbonyl, ethoxycarbonyl, tert-butoxycarbonyl, etc., and the like), (13) a sulfo group (-SO₃H), (14) a sulfino group, (15) a phosphono group, (16) a nitro group, (17) a cyano group, (18) an amidino group, (19) (C1~6 alkoxyimino)methyl groups (e.g., methoxyimino)methyl group, etc.), (20) a -B(OH)₂ group, (21) halogen atoms (e.g., fluorine, chlorine, bromine, iodine, etc.), (22) alkylsulfinyl groups (e.g., C1~4 alkylsulfinyl groups, such as methylsulfinyl, ethylsulfinyl, etc. and the like), (23) aromatic-ring sulfinyl groups (e.g., C6~10 aromatic-ring sulfinyl groups, such as phenylsulfinyl, etc. and the like), (24) alkylsulfonyl groups (e.g., C1~4 alkylsulfonyl groups, such as methylsulfonyl, ethylsulfonyl, etc. and the like), (25) aromatic-ring sulfonyl groups (e.g., C6~10 aromatic-ring sulfonyl groups, such as phenylsulfonyl, etc. and the like), (26) acyl groups (e.g. , C1~6 alkanoyl groups, such as formyl, acetyl, propanoyl, pivaloyl, etc., C6~10 aromatic-ring carbonyl groups, such as benzoyl, etc., and the like), (27) groups represented by the formula: 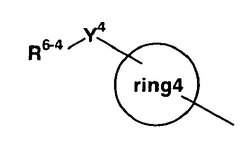 (wherein all the symbols are as defined above), and the like.

As used herein, (1) optionally substituted alkyl groups, (2) optionally substituted alkenyl groups, (3) optionally substituted alkynyl groups, (4) optionally substituted carbon-ring groups, (5) optionally substituted heterocyclic groups, (6) optionally protected hydroxyl groups, (7) optionally protected mercapto groups and (8) optionally protected amino groups, (9) optionally substituted carbamoyl groups, and (10) optionally substituted sulfamoyl groups, as represented by R⁴, have the same meanings as defined above for those represented by R³.

As used herein, the ester-linkage containing group represented by R⁶⁻⁴ has the same meaning as defined above for the ester-linkage containing groups represented by R⁶.

As used herein, the spacer of 1 to 5 in number of atoms for the main chain which may be substituted with substituents, as represented by Y⁴, has the same meaning as defined above for the spacer of 1 to 5 in number of atoms for the main chain which may be substituted with substituents, as represented by Y.

As used herein, the "cyclic group which may be substituted with substituents", as represented by ring4, is understood to have the same meaning as defined above for the cyclic groups which may be substituted with substituents, as represented by ring1.

As used herein, R³ may be: 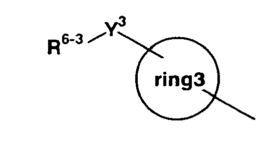 (wherein all the symbols are as defined above), and R⁴ may be: 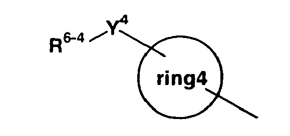 (wherein all the symbols are as defined above).

As used herein, the substituent represented by R⁵ includes, for example, (1) optionally substituted alkyl groups, (2) optionally substituted alkenyl groups, (3) optionally substituted alkynyl groups, (4) optionally substituted carbon-ring groups, (5) optionally substituted heterocyclic groups, (6) optionally protected hydroxy groups, (7) optionally protected mercapto groups, (8) optionally protected amino groups, (9) optionally substituted carbamoyl groups, (10) optionally substituted sulfamoyl groups, (11) a carboxyl group, (12) alkoxycarbonyl groups (e.g., C1~6-alkoxycarbonyl groups, such as methoxycarbonyl, ethoxycarbonyl, tert-butoxycarbonyl, etc., and the like), (13) a sulfo group (-SO₃H), (14) a sulfino group, (15) a phosphono group, (16) a nitro group, (17) a cyano group, (18) an amidino group, (19) (C1~6-alkoxyimino)methyl groups (e.g., a (methoxyimino)methyl group, etc.), (20) a -B(OH)₂ group, (21) halogen atoms (e.g., fluorine, chlorine, bromine, iodine, etc.), (22) alkylsulfinyl groups (e.g., C1~4-alkylsulfinyl groups, such as methylsulfinyl, ethylsulfinyl, etc. and the like), (23) aromatic-ring sulfinyl groups (e.g., C6~10 aromatic-ring sulfinyl groups, such as phenylsulfinyl, etc. and the like), (24) alkylsulfonyl groups (e.g., C1~4 alkylsulfonyl groups, such as methylsulfonyl, ethylsulfonyl, etc. and the like), (25) aromatic-ring sulfonyl groups (e.g., C6~10 aromatic-ring sulfonyl groups, such as phenylsulfonyl, etc. and the like), (26) acyl groups (e.g., C1~6 alkanoyl groups, such as formyl, acetyl, propanoyl, pivaloyl, etc., C6~10 aromatic-ring carbonyl groups, such as benzoyl, etc., and the like), (27) groups represented by the formula: 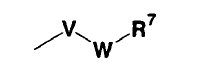 (wherein all the symbols are as defined above), and the like.

As used herein, (1) optionally substituted alkyl groups, (2) optionally substituted alkenyl groups, (3) optionally substituted alkynyl groups, (4) optionally substituted carbon-ring groups, (5) optionally substituted heterocyclic groups, (6) optionally protected hydroxyl groups, (7) optionally protected mercapto groups and (8) optionally protected amino groups, (9) optionally substituted carbamoyl groups, and (10) optionally substituted sulfamoyl groups, as represented by R⁵, have the same meanings as defined above for those represented by R³.

As used herein, the substituent on the carbon atom which may be substituted with a substituent(s), as represented by V, includes, for example, C1~8 alkyl groups (e.g., methyl, ethyl, n-propyl, isopropyl, n-butyl, isobutyl, sec-butyl, tert-butyl, pentyl, hexyl;, heptyl, octyl, etc.), a hydroxy group, an amino group, a carboxyl group, a nitro group, mono-or di-C1~6 alkylamino groups (e.g., methylamino, ethylamino, propylamino, dimethylamino, diethylamino, etc.), C1~6-alkoxy groups (e.g., methoxy, ethoxy, propoxy, isopropoxy, n-butoxy, sec-butoxy, tert-butoxy, pentyloxy, hexyloxy, etc.), C1~6-alkoxycarbonyl group (e.g., methoxycarbonyl, ethoxycarbonyl, tert-butoxycarbonyl, etc.), C1~6-alkylcarbonyloxy groups (e.g., acetoxy, ethylcarbonyloxy, etc.), C1~4-alkylthio groups (e.g., methylthio, ethylthio, propylthio, butylthio, etc.), halogen atoms (fluorine, chlorine, bromine, iodine), trihalomethyl groups (e.g., trifluoromethyl, etc.), and the like, and these arbitrary substituents may be replaced in number of 1 to 2.

As used herein, the substituent on the nitrogen atom which may be substituted with a substituent(s) as represented by V includes, for example, C1~8 alkyl groups (e. g. , methyl, ethyl, n-propyl, isopropyl, n-butyl, isobutyl, sec-butyl, tert-butyl, pentyl, hexyl;, heptyl, octyl, etc.), a hydroxy group, an amino group, a carboxyl group, a nitro group, mono-or di-C1~6 alkylamino groups (e.g., methylamino, ethylamino, propylamino, dimethylamino, diethylamino, etc.), C1~6-alkoxy groups (e.g., methoxy, ethoxy, propoxy, isopropoxy, n-butoxy, sec-butoxy, tert-butoxy, pentyloxy, hexyloxy, etc.), C1~6-alkoxycarbonyl group (e.g., methoxycarbonyl, ethoxycarbonyl, tert-butoxycarbonyl, etc.), C1~6-alkylcarbonyloxy groups (e.g., acetoxy, ethylcarbonyloxy, etc.), C1~4-alkylthio groups (e.g., methylthio, ethylthio, propylthio, butylthio, etc.), halogen atoms (fluorine, chlorine, bromine, iodine), trihalomethyl groups (e.g., trifluoromethyl, etc.), and the like.

As used herein, the optionally oxidized sulfur atom represented by V includes, for example, -S-, -SO- or -SO₂-.

As used herein, the "spacer of 1 to 5 in number of atoms for the main chain" in the spacers of 1 to 5 in number of atoms for the main chain which may be substituted with a substituent(s), as represented by W, has the same meaning as defined above for the "spacer of 1 to 5 in number of atoms for the main chain" represented by Y.

As used herein, the ester-linkage containing group represented by R⁷ is understood to have the same meaning as defined above for the one represented by R⁶.

As used herein, the anion represented by X⁻ means any negatively charged atoms or molecules that can be paired for the quaternary ammonium moiety in the general formula (I). One molecule of the quaternary ammonium moiety in the general formula (I) forms a salt with a 1/n molecule of an n-valent anion. The negatively charged atom may be exemplified by chlorine ion, bromine ion, iodine ion, etc., while the negatively charged molecule includes, for example, inorganic acid ions, such as sulfate ion, phosphate ion, nitrate ion, etc. , and organic acid ions, such as acetate ion, lactate ion, tartarate ion, benzoate ion, citrate ion, methanesulfonate ion, ethanesulfonate ion, methylsulfate ion, benzenesulfonate ion, p-toluenesulfonate ion, isethionate ion, glucuronate ion, gluconate ion, etc.

As used herein, the prodrug of a compound represented by the general formula (I) refers to any compounds that, through the reactions with the enzymes or gastric acid in the living body, can be converted to the compounds represented by the general formula (I). The prodrug of a compound represented by the general formula (I) may be exemplified by the compounds of the general formula (I) where an amino group is contained, in which the said amino group has been allowed to undergo acylation, alkylation or phosphorylation (e.g., the compounds of the general formula (I) in which the said amino group has been allowed to undergo eicosanoylation, alanylation, pentylaminocarbonylation, (5-methyl-2-oxo-1,3-dioxolen-4-yl)-methoxycarbonylation, tetrahydrofuranylation, pyrrolizylmethylation, pivaloyloxymethylation, acetoxymethylation or tert-butylation, and the like); the compounds of the general formula (I) where a hydroxy group is contained, in which the said hydroxy group has been allowed to undergo acylation, alkylation, phosphorylation or boration (e.g., the compounds of the general formula (I) in which the said hydroxy group has been allowed to undergo acetylation, palmitoylation, propanoylation, pivaloylation, succinylation, fumarylation, alanylation or dimethylaminomethylcarbonylation, and the like); the compounds of the general formula (I) where a carboxyl group is contained, in which the said carboxyl group has been allowed to undergo esterification or amidation (e.g., the compounds of the general formula (I) in which the said carboxyl group has been allowed to undergo ethyl-esterification, isopropyl-esterification, phenyl-esterification, carboxymethyl-esterification, dimethylamino-methylesterification, pivaloyloxymethyl-esterification, ethoxycarbonyloxyethyl-esterification, phthalidyl-esterification, (5-methyl-2-oxo-1,3-dioxolene-4-yl)methyl-esterification, cyclohexyloxycarbonylethyl-esterification or methyl-amidation, and the like); the compounds of the general formula (I) where a carboxyl group is contained, in which the said carboxyl group has been allowed to undergo replacement with a hydroxymethyl group, and the like. These compounds can be produced in accordance with the per se known processes. The prodrug of the compound represented by the general formula (I) may be in the form of either hydrated or non-hydrated products.

In the present invention, A is preferably the chemical structures: 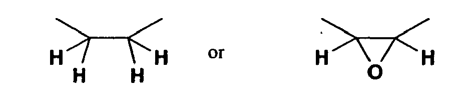 more preferably the chemical structure:

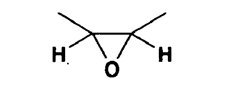

In the present invention, R¹ preferably is optionally substituted alkyl groups, optionally substituted alkenyl groups, optionally substituted alkynyl groups, optionally substituted carbon-ring groups, optionally substituted heterocyclic groups or groups represented by the formula: 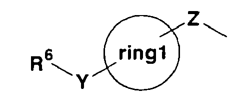 (wherein all the symbols are as defined above), more preferably optionally substituted alkyl groups, optionally substituted alkenyl groups, optionally substituted alkynyl groups, or groups represented by the formula: 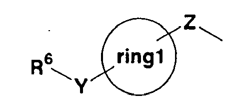 (wherein all the symbols are as defined above), further preferably groups represented by the formula: 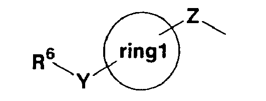 (wherein all the symbols are as defined above).

In the present invention, R² preferably is optionally substituted alkyl groups, optionally substituted alkenyl groups, optionally substituted alkynyl groups, optionally substituted carbon-ring groups or optionally substituted heterocyclic groups, more preferably optionally substituted alkyl groups, optionally substituted alkenyl groups or optionally substituted alkynyl groups.

In the present invention, R³ preferably is optionally substituted alkyl groups, optionally substituted alkenyl groups, optionally substituted alkynyl groups, optionally substituted carbon-ring groups, optionally substituted heterocyclic groups or groups represented by the formula: 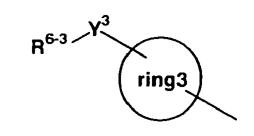 (wherein all the symbols are as defined above), more preferably optionally substituted carbon-ring groups, optionally substituted heterocyclic groups, or groups represented by the formula: 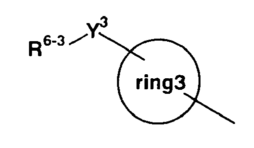 (wherein all the symbols are as defined above). The optionally substituted carbon-ring group or optionally substituted heterocyclic group as represented by R³ preferably is optionally substituted benzene, optionally substituted thiophene, optionally substituted pyridine, optionally substituted cyclopentane, optionally substituted cyclohexane, optionally substituted thiazoles or optionally substituted furan.

In the present invention, the ring3 in the group represented by the formula: 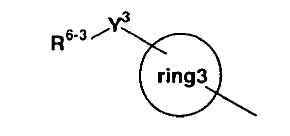 (wherein all the symbols are as defined above) preferably is optionally substituted benzene, optionally substituted thiophene, optionally substituted pyridine, optionally substituted cyclopentane, optionally substituted cyclohexane, optionally substituted thiazole, or optionally substituted furan, more preferably benzene, thiophene or furan, while -Y³-R⁶⁻³ (where all the symbols are as defined above) preferably is -CH=CH-CO-(C1~6-alkoxy), -O-(C1~6-alkyl)-CO-(C1~6-alkoxy), -NH-(C1~6-alkyl)-CO-(C1~6-alkoxy), -N[-(C1~6-alkyl)-CO-(C1~6-alkoxy)]₂ or -COO-(C1~6-alkyl)-CO-(C1~6-alkoxy), more preferably -O-CH₂-COOCH₃ or -COO-CH₂-COOCH₃.

In the present invention, R⁴ preferably is optionally substituted alkyl groups, optionally substituted alkenyl groups, optionally substituted alkynyl groups, optionally substituted carbon-ring groups, optionally substituted heterocyclic groups, or groups represented by the formula: 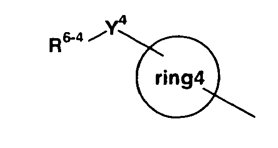 (wherein all the symbols are as defined above), more preferably optionally substituted carbon-ring groups, optionally substituted heterocyclic groups or groups represented by the formula: 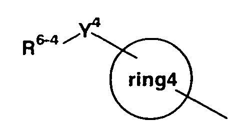 (wherein all the symbols are as defined above). The optionally substituted carbon-ring group and optionally substituted heterocyclic group as represented by R⁴ preferably are optionally substituted benzene, optionally substituted thiophene, optionally substituted pyridine, optionally substituted cyclopentane, optionally substituted cyclohexane, optionally substituted thiazole or optionally substituted furan.

In the present invention, the ring4 in the group represented by the formula: 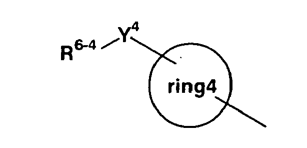 (wherein all the symbols are as defined above) preferably is optionally substituted benzene, optionally substituted thiophene, optionally substituted pyridine, optionally substituted cyclopentane, optionally substituted cyclohexane, optionally substituted thiazole or optionally substituted furan, more preferably benzene, thiophene and furan, while -Y⁴-R⁶⁻⁴ (wherein all the symbols are as defined above) preferably is -CH=CH-CO-(C1~6-alkoxy), -O-(C1~6-alkyl)-CO-(C1~6-alkoxy), -NH-(C1~6-alkyl)-CO-(C1~6-alkoxy), -N[-(C1~6-alkyl)-CO-(C1~6-alkoxy)]₂, or -COO-(C1~6-alkyl)-CO- (C1~6-alkoxy), more preferably -O-CH₂-COOCH₃, or -COO-CH₂-COOCH₃.

In the present invention, the compound of the general formula (I) where R³ contains an ester-linkage containing group as represented by R⁶⁻³ and/or R⁴ contains an ester-linkage containing group as represented by R⁶⁻⁴ preferably has a methyl group as R¹ and R².

In the present invention, R⁵ preferably is optionally substituted alkyl groups, optionally substituted alkenyl groups, optionally substituted alkynyl groups, optionally substituted carbon-ring groups, optionally substituted heterocyclic groups, optionally protected hydroxy groups, optionally protected mercapto groups, optionally protected amino groups, optionally protected carbamoyl groups, an alkoxycarbonyl group, a cyano group, (C1-6-alkoxyimino)methyl groups, halogen atoms, acyl groups or groups represented by the formula: 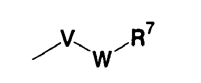 (wherein all the symbols are as defined above), more preferably optionally protected hydroxy groups, optionally protected mercapto groups, optionally protected amino groups, optionally protected carbamoyl groups, alkoxycarbonyl groups, a cyano group, halogen atoms, acyl groups or groups represented by the formula: 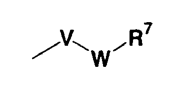 (wherein all the symbols are as defined above), further preferably optionally protected hydroxy groups, halogen atoms, or groups represented by the formula: 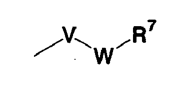 (wherein all the symbols are as defined above).

In the present invention, X⁻ includes preferably chlorine ion, iodine ion, nitrate ion, methylsulfate ion, methanesulfonate ion, benzenesulfonate ion or p-toluenesulfonate ion, more preferably chlorine ion, bromine ion or iodine ion.

In the present invention, the symbol: 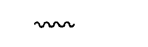 preferably is an endo isomer.

In the present invention, the ester-linkage containing group represented by R¹ preferably is: 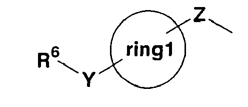 (wherein all the symbols are as defined above), more preferably: 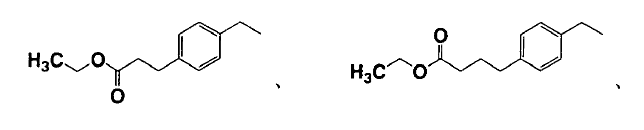 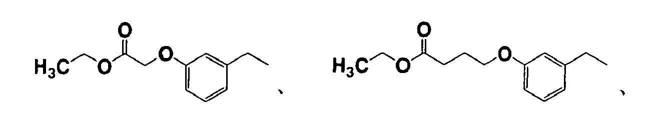 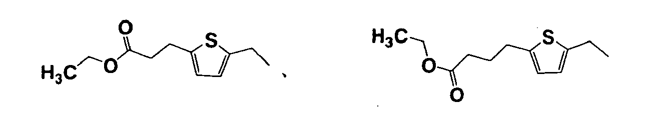 , or

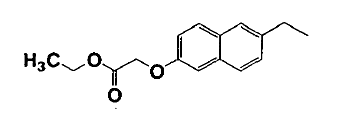

In the present invention, the ester-linkage containing group represented by R⁶ includes preferably C1~15 alkoxycarbonyl groups, alkyl groups substituted with C1~15 alkoxycarbonyl groups, alkenyl groups substituted with C1~15 alkoxycarbonyl groups, alkynyl groups substituted with C1~15 alkoxycarbonyl groups, C1~15 acyloxy groups, alkyl groups substituted with C1~15 acyloxy groups, alkenyl groups substituted with C1~15 acyloxy groups, alkynyl groups substituted with C1~15 acyloxy groups, more preferably C1~15 alkoxycarbonyl groups, alkyl groups substituted with C1~15 alkoxycarbonyl groups, alkenyl groups substituted with C1~15 alkoxycarbonyl groups, alkynyl groups substituted with C1~15 alkoxycarbonyl groups, further preferably C1~15 alkoxycarbonyl groups, in particular preferably methoxycarbonyl, ethoxycarbonyl, n-propoxycarbonyl and isopropoxycarbonyl groups.

In the present invention, the formula: 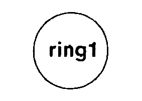 is a cyclic group which may be substituted with a substituent(s), and the said cyclic group preferably is optionally, partially or fully saturated C3~15 monocyclic unsaturated carbon-rings or optionally partially or fully saturated C4~15 bicyclic unsaturated carbon-rings, or optionally, partially or fully saturated C3~15 monocyclic unsaturated heterocycles containing 1 to 5 heteroatoms selected from oxygen, nitrogen and sulfur atoms or optionally, partially or fully saturated C4~15 bicyclic unsaturated heterocycles containing 1 to 5 heteroatoms selected from oxygen, nitrogen and sulfur atoms, more preferably optionally, partially or fully saturated C5~6 monocyclic unsaturated carbon-rings or optionally, partially or fully saturated C9~10 bicyclic unsaturated carbon-rings, or optionally, partially or fully saturated C5~6 monocyclic unsaturated heterocycles containing 1 to 5 heteroatoms selected from oxygen, nitrogen and sulfur atoms or optionally, partially or fully saturated C9~10 bicyclic unsaturated heterocycles containing 1 to 5 heteroatoms selected from oxygen, nitrogen and sulfur atoms, further preferably benzene, thiophene, furan, naphthalene, quinoline, indole, benzothiophene, benzofuran or pyridine, most preferably benzene, thiophene or naphthalene.

In the present invention, Y preferably is methylene groups which may have 1 to 2 substituents, optionally substituted nitrogen, bivalent groups consisting 1 to 5 linkages selected from -CO-, -O- and -S-, more preferably methylene groups which may have 1 to 2 substituents and bivalent groups consisting 1 to 5 linkages selected from -O- and -S-, further preferably -CH₂-, -(CH₂)₂-, -(CH₂)₃-, -(CH₂)₄-, -O-CH₂-, -O-(CH₂)₂-, -O-(CH₂)₃-, -S-CH₂-, -S-(CH₂)₂-, -S-(CH₂)₃-, -CH₂-O-, -(CH₂)₂O-, -(CH₂)₃O-, -CH₂-S-, -(CH₂)₂-S-, and -(CH₂)₃-S-.

In the present invention, Z includes, preferably, -CH₂-, -(CH₂)₂-, -(CH₂)₃-, -(CH₂)₄-, -(CH₂)₅-, -CH₂-O-CH₂-, -CH₂-O-(CH₂)₂-, -CH₂-O-(CH₂)₃-, -(CH₂)₂-O-(CH₂)₂-, -CH₂-S-CH₂-, -CH₂-S-(CH₂)₂-, -CH₂-S-(CH₂)₃-, -(CH₂)₂-S-(CH₂)₂-, -(CH₂)₂-O-CH₂-, -(CH₂)₃-O-CH₂-, -(CH₂)₂-S-CH₂- or -(CH₂)₃-S-CH₂-, more preferably -CH₂-, -(CH₂)₂-, -(CH₂)₃-, -(CH₂)₄- and -(CH₂)₅-, most preferably -CH₂-.

In the present invention, the ester-linkage containing group as presented by R⁵ preferably is groups represented by the formula: 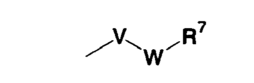 (wherein all the symbols are as defined above), more preferably -O-CH₂-CO₂CH₃, -O-CH₂-CO₂C₂H₅ or -S-CH₂-CO₂CH₃.

In the present invention, the ester-linkage containing group represented by R⁷ preferably is C1~15-alkoxycarbonyl groups, alkyl groups substituted with C1~15-alkoxycarbonyl groups, alkenyl groups substituted with C1~15-alkoxycarbonyl groups, alkynyl groups substituted with C1~15-alkoxycarbonyl groups, more preferably C1~15-alkoxycarbonyl groups or alkynyl groups substituted with C1~15-alkoxycarbonyl groups, furthermore preferably a methoxycarbonyl, ethoxycarbonyl, n-propoxycarbonyl or isopropoxycarbonyl group, with methoxycarbonyl and ethoxycarbonyl groups being particularly preferred.

In the present invention, V preferably is nitrogen atom which may be substituted with a substituent(s), optionally oxidized sulfur or oxygen atom, more preferably -O- or -S-.

In the present invention, W preferably is -CH₂-.

In the present invention, the general formula (I) includes, preferably, the general formula (I-1) or general formula (I-2).

In the present invention, the preferable compounds include, for example, the compounds to be described below in Examples, i.e., (1R,3r,5S,8s)-8-[3-(2-ethoxy-2-oxoethoxy)-bezyl]-3-({hydroxy[di(2-thienyl)]acetyl}oxy)-8-methyl-8-azoniabicyclo[3.2.1]octane bromide, (1R,3r,5S,8s)-8-{[5-(4-ethoxy-4-oxobutyl)-2-thienyl]methyl}-3-({hydroxy-[di(2-thienyl)]acetyl}oxy)-8-methyl-8-azoniabicyclo[3.2.1] octane chloride, (1R,2R,4S,5S,7s,9r)-9-[4-(3-ethoxy-3-oxpropyl)benzyl]-7-({hydroxy[di(2-thienyl)]acetyl}oxy)-9-methyl-3-oxa-9-azoniatricyclo[3.3.1.0^{2,4}]nonane bromide,

(1R,2R,4S,5S,7s,9r)-9-[4-(3-ethoxy-3-oxopropyl)benzyl]-7 -({hdroxy[di(3-thienyl)]acetyl}oxy)-9-methyl-3-oxa-9-azonia - tricyclo[3.3.1.0^{2,4}]nonane bromide,(1R,2R,4S,5S,7s,9r)-9-[3-(2-ethoxy-2-oxoethoxy)benzyl]-7-({hydroxy-[di(2-thienyl)]acetyl}oxy)-9-methyl-3-oxa-9-azoniatricyclo-[3.3.1.0^{2,4}]nonane bromide,(1R,2R,4S,5S,7s,9r)-9-[3-(2-ethoxy-2-oxoethoxy)benzyl]-7-({hydroxy[di(3-thienyl)]-acetyl}oxy)-9-methyl-3-oxa-9-azoniatricyclo[3.3.1.0^{2,4}]-nonane bromide, (1R,2R,4S,5S,7s,9r)-9-[3-(2-ethoxy-2- oxo-oxo-thoxy)benzyl]-7-({hydroxy[diphenyl)acetyl]oxy}-9-methyl-3-o xa-9-azoniatricyclo[3.3.1.0^{2,4}]nonane bromide, (1R,3r,5S,8s)-8-{[5-(4-ethoxy-4-oxobutyl)-2-thienyl]methyl} -3-({hydroxy[di(3-thienyl)]acetyl}oxy)-8-methyl-8-azoniabic yclo[3.2.1]octane chloride, (1R,3r,5S,8s)-3-{[di(2-furyl)(hydroxy)acetyl]oxy}-8-[4-(3-ethoxy-3-oxopropyl)benzyl]-8-methyl-8-azoniabicyclo[3.2.1]octane bromide,(1R,3r,5S,8s)-3-{[di(2-furyl)(hydroxy)acetyl]oxy}-8-[3-(2-ethoxy-2-oxoethoxy)benzyl]-8-methyl-8-azoniabicyclo[3.2.1]octane bromide, (1R,3r,5S,8s)-3-{[di(2-furyl)(hydroxy)acetyl]oxy}-8-{[5-(4-ethoxy-4-oxobutyl)-2-thienyl]methyl}-8-methyl-8-azoniabicyclo[3.2.1]octane chloride, (1R,3r,5S,8s)-8-[3-(3-ethoxy-3- oxopropoxy)benzyl] -3-{[hydroxy(diphenyl)acetyl]oxy}-8-methyl-8-azoniabicyclo-[3.2.1]octane bromide, (1R,3r,5S,8s)-8-[3-(4-ethoxy-4-oxobutoxy)benzyl]-3-({hydroxy(di(2-thienyl)]acetyl}oxy)-8-methyl-8-azoniabicyclo[3.2.1]octane bromide, (1R,3r,5S,8s)-8-[3-(4-ethoxy-4-oxobutoxy)benzyl]-3-({hydroxy(di(3-thienyl)]acetyl}oxy)-8-methyl-8-azoniabicyclo[3.2.1]octane bromide, (1R,3r,5S,8s)-8-[3-(3-ethoxy-3-oxopropoxy)benzyl]-3-({hydroxy[di(2-thienyl)]-acetyl}oxy)-8-methyl-8-azoniabicyclo[3.2.1]octane bromide, (1R,3r,5S,8s)-8-[3-(3-ethoxy-3-oxopropoxy)benzyl]-3-({hydroxy[di(3-thienyl)]acetyl}oxy)-8-methyl-8-azoniabicyclo[3.2.1]octane bromide, (1R,2R,4S,5S,7s,9r)-9-{[5-(4-ethoxy-4-oxobutyl)-2-thienyl]methyl}-7-([hydroxy(diphenyl)-acetyl]oxy)-9-methyl-3-oxa-9-azoniatricyclo[3.3.1.0^{2,4}]-nonane chloride, (1R,2R,4S,5S,7s,9r)-9-{[5-(4-ethoxy-4-oxobutyl)-2-thienyl]methyl}-7-({hydroxy[di(2-thienyl)]-acetyl}oxy)-9-methyl-3-oxa-9-azoniatricyclo[3.3.1.0^{2,4}]-nonane chloride, (1R,2R,4S,5S,7s,9r)-9-{[5-(4-ethoxy-4-oxobutyl)-2-thienyl]methyl}-7-({hydroxy[di(3-thienyl)]-acetyl}oxy)-9-methyl-3-oxa-9-azoniatricyclo[3.3.1.0^{2,4}]-nonane chloride,(1R,2R,4S,5S,7s,9r)-7-{[di(2-furyl)-(hydroxy)acetyl]oxy}-9-{[5-(4-ethoxy-4-oxobutyl)-2-thienyl]methyl}-9-methyl-3-oxa-9-azoniatricyclo[3.3.1.0^{2,4}]-nonane chloride, (1R,3r,5S,8s)-3-{[di(2-furyl)(hydroxy)-acetyl]oxy}-8-[3-(4-ethoxy-4-oxobutoxy)benzyl]-8-methy-8-azoniabicyclo[3.2.1]octane bromide, (1R,3r,5S,8s)-8-{[6-(2-ethoxy-2-oxoethoxy)-2-naphthyl]methyl}-3-({hydroxy[di(2-thienyl)]acetyl}oxy)-8-methyl-8-azoniabicyclo[3.2.1]octane bromide, (1R,3r,5S,8s)-8-{[6-(2-ethoxy-2-oxoethoxy)-2-naphthyl]methyl}-3-({hydroxy(diphenyl)acetyl]oxy}-8-methyl-8-azoniabicyclo[3.2.1]octane bromide, (1R,3s,5S,8r)-8-{[5-(4-ethoxy-4-oxobutyl)-2-thienyl]methyl}-3-{[hydroxy-(diphenyl)acetyl]oxy}-8-methyl-8-azoniabicyclo[3.2.1]oct-6-en chloride, (1R,3r,5S,8r)-8-[4-(3-ethoxy-3-oxopropyl)-bezyl]-3-({hydroxy[di(2-thienyl)acetyl}oxy)-8-methyl-8-azoniabicyclo[3.2.1]oct-6-en bromide, (1R,2R,4S,5S,7s,9r)-9-{[6-(2-ethoxy-2-oxoethoxy)-2-naphthyl]methyl}-7-({hdroxy[di(2-thienyl)]acetyl}oxy}-9-methyl-3-oxa-9-azoniatricyclo[3.3.1.0^{2,4}]nonane bromide, (1R,3s,5S,8r)-8-{[6-(2-ethoxy-2-oxoethoxy)-2-naphthyl]methyl}-3-({hydroxy-[di(3-thienyl)acetyl}oxy)-8-methyl-8-azoniabicyclo[3.2.1]-oct-6-en bromide, (1R,3s,5S,8r)-3-{[cyclopentyl(hydroxy)-phenylacetyl]oxy}-8-[3-(2-ethoxy-2-oxoethoxy)benzyl]-8-methyl-8-azoniabicyclo[3.2.1]oct-6-en bromide, (1R,2R,4S,5S,7s,9r)- 7-{[cyclopentyl(hydroxy)phenylacetyl]-oxy}-9-{[6-(2-ethoxy-2-oxoethoxy)-2-naphthyl]methyl}-9-methyl-3-oxa-9-azoniatricyclo[3.3.1.0^{2,4}]nonane bromide, (1R,3r,5S,8s)-3-{[cyclopentyl(hydroxy)-2-thienylacetyl]oxy }-8-{[6-(2-ethoxy-2-oxoethoxy)-2-naphthyl]methyl}-8-methy-8 -azoniabicyclo[3.2.1]octane bromide, (1R,3s,5S,8r)-3-{[cyclopentyl(hydroxy)-2-thienylacetyl]oxy}-8-[3-(2-ethoxy-2-oxoethoxy)benzyl]-8-methy-8-azoniabicyclo[3.2.1]oct-6-en bromide,(1R,2R,4S,5S,7s)-7-({hydroxy[4-(2-methoxy-2-oxoethoxy)phenyl]phenylacetyl}oxy)-9,9-dimethyl-3-oxa-9-azoniatricyclo[3.3.1.0^{2,4}]nonane bromide, (1R,2R,4S,5S,7s)-7-{[[4-(ethoxycarbonyl)phenyl](hydroxy)phenylacetyl]oxy}-9,9-dimethyl-3-oxa-9-azoniatricyclo[3.3.1.0^{2,4}]nonane bromide, (1R,2R,4S,5S,7s)-7-{[[5-(ethoxycarbonyl)-2-thienyl]-(hydroxy)phenylacetyl]oxy}-9,9-dimethyl-3-oxa-9-azoniatricyclo[3.3.1.0^{2,4}]nonane bromide, (1R,2R,4S,5S,7s)-7-[(hydroxy{5-[(2-methoxy-2-oxoethoxy)carbonyl]-2-thienyl}-2-thie nylacetyl)oxy]-9,9-dimethyl-3-oxa-9-azoniatricyclo-[3.3.1.0^{2,4}]nonane bromide, (1R,2R,4S,5S,7s)-7-({hydroxy[5-(methoxycarbonyl)-2-thienyl]-2-thienylacetyl}oxy]-9,9-dimethyl-3-oxa-9-azoniatricyclo[3.3.1.0^{2,4}]nonane bromide, (1R,2R,4S,5S,7s)-7-({hydroxy[3-(2-methoxy-2-oxoethoxy)-phenyl]phenylaceyl}oxy)-9,9-dimethyl-3-oxa-9-azoniatricyclo[3.3.1.0^{2,4}]nonane bromide, (1R,2R,4S,5S,7s)-7-({hydroxy[3-(2-methoxy-2-oxoethoxy)phenyl]-2-thienylacetyl}-oxy)-9,9-dimethyl-3-oxa-9-azoniatricyclo[3.3.1.0^{2,4}] nonane bromide, (1R,2R,4S,5S,7s)-7-({hydroxy[3-(2-methoxy-2-oxoethoxy)phenyl]-1,3-thiazol-2-ylacetyl}oxy]-9,9-dimethyl-3-oxa-9-azoniatricyclo[3.3.1.0^{2,4}]nonane bromide, (1R,2R,4S,5S,7s)-7-({hydroxy[4-(2-methoxy-2-oxoethoxy)-phenyl]-2-thienylacetyl}- oxy)-9,9-dimethyl-3-oxa-9-azoniatricyclo[3.3.1.0^{2,4}]nonane bromide, (1R,2R,4S,5S,7s)-7-({hydroxy[5-(methoxycarbonyl)-2-furyl]2-thienylacetyl}-oxy)-9,9-dimethyl-3-oxa-9-azoniatricyclo[3.3.1.0^{2,4}]nonane bromide, (1R,2R,4S,5S,7s,9r)-9-[4-(3-ethoxy-3-oxopropyl)-benzyl]-7-{[hydroxy(di-3-thienyl)acetyl]oxy}-9-methyl-3-oxa-9-azoniatricyclo[3.3.1.0^{2,4}]-nonane bromide and (1R,2R,4S,5S,7s,9r)-9-(4-{3-[2-(dimethyl-amino)-2-oxoethyl]-3-oxopropyl}benzyl)-7-{[hydroxy(di-2-thienyl)-acetyl]oxy}-9-methyl-3-oxa-9-azoniatricyclo-[3.3.1.0^{2,4}]-nonane bromide, their salts, their solvation products and prodrugs thereof, and there can be mentioned, more preferably, (1R,3r,5S,8s)-8-{[6-(2-ethoxy-2-oxoethoxy)-2-naphthyl]-methyl}-3-{[hydroxy(diphenyl)acetyl]oxy}-8-methyl-8-azoniabicyclo[3.2.1]octane bromide, (1R,3r,5S,8r)-8-[4-(3-ethoxy-3-oxopropyl)benzyl]-3-({hydroxy[di(2-thienyl)]-acetyl}oxy)-8-methyl-8-azoniabicyclo[3.2.1]oct-6-en bromide, (1R,3s,5S,8r)-8-{[5-(4-ethoxy-4-oxobutyl)-2-thienyl]methyl}-3-{[hydroxy(diphenyl)acetyl]oxy}-8-methyl-8-azoniabicyclo[3.2.1]oct-6-en chloride, (1R,2R,4S,5S,7s,9r)-9-[4-(3-ethoxy-3-oxopropyl)benzyl]-7-{[hydroxy(di-3-thienyl)]acetyl}oxy}-9-methyl-3-oxa-9-azoniatricyclo[3.3.1.0^{2,4}]nonane bromide, (1R,2R,4S,5S,7s,9r)-9-(4-{3-[2-(dimethylamino)-2-oxoethyl] -3-oxopropyl}benzyl)-7-{[hydroxy(di-2-thienyl)acetyl]oxy}-9-methyl-3-oxa-9-azoniatricyclo[3.3.1.0^{2,4}]nonane bromide, (1R,2R,4S,5S,7s)-7-({hydroxy[5-(methoxycarbonyl)-2-thienyl]-2-thienylacetyl}oxy)-9,9-dimethyl-3-oxa-9-azoniatricyclo[3.3.1.0^{2,4}]nonane bromide,(1R,2R,4S,5S,7s)-7-({hydroxy[5-(methoxycarbonyl)-2-furyl]-2-thienylacetyl}-oxy)-9,9-dimethyl-3-oxa-9-azoniatricyclo[3.3.1.0^{2,4}]nonane bromide and (1R,2R,4S,5S,7s)-7-{[[5-(ethoxycarbonyl)-2-thienyl](hydroxy)phenylacetyl]oxy}-9,9-dimethyl-3-oxa-9-azoniatricyclo[3.3.1.0^{2,4}]nonane bromide, their salts, their solvation products and prodrugs thereof.

In the present invention, all the isomers are understood to be included in the compounds of the present invention, unless otherwise indicated particularly. For example, the alkyl, alkenyl, alkynyl, alkoxy, alkylthio, alkylene, alkenylene and alkynylene groups are understood to comprehend their straight-chain and branched ones. Furthermore, isomers (E-and Z-isomers, cis- and trans-isomers) in the double bond, rings or condensed rings, isomers (R- and S-isomers, a- and β-configurations, enatiomers, diastereomers) resulting from the existence of asymmetric carbon, and the like, optically active compounds (D- and L-isomers, d- and 1-isomers), tautomers, polar compounds (high-polar and low-polar compounds) produced by chromatographic separation, equilibrium compounds and rotational isomers, as well as mixtures thereof at any mixing ratios and racemic mixtures are all understood to be included in the present invention.

### [Salts]

The compounds represented by the general formula (I) are converted to salts in accordance with the conventionally known methods, whereby such salts include preferably pharmaceutically acceptable salts.

The salts may be exemplified by alkali metal salts, alkaline-earth metal salts, ammonium salts, organic amine salts, acid-addition salts, etc.

The salts include preferably water-soluble salts. As the suitable salt, for example, there may be mentioned salts with alkali metals (e.g., potassium, sodium, etc.), salts with alkaline earth metals (e.g., calcium, magnesium, etc.), ammonium salts, and salts with pharmaceutically acceptable organic amines (tetramethylammonium, triethylamine, methylamine, dimethylamine, cyclopentylamine, benzylamine, phenethylamine, piperidine, monoethanolamine, diethanolamine, tris(hydroxymethyl)aminomethane, lysine, arginine, N-methyl-D-glucamine, etc.).

It is preferable that the acid-addition salts are water-soluble. Appropriate acid-addition salts include, for example, inorganic acid salts, such as hydrochlorides, hydrobromides, hydroiodides, sulfates, phosphates and nitrates, and organic acid salts, such as acetates, lactates, tartarates, benzoates, citrates, methanesulfonates, benzenesulfonates, toluenesulfonates, isethionates, glucuronates and gluconates.

The compounds represented by the general formula (I) and their salts can also be converted to solvation products.

It is preferred that the solvation products are free from toxicity and water-soluble. As a suitable solvation product, for example, there may be mentioned the solvation products formed with such solvents as water and alcohol solvents (e.g., ethanol, etc.).

### [Process for Producing the Compounds of the Present Invention]

The compounds represented by the general formula (I) can be produced in accordance with the below-described processes, any processes similar thereto or the processes to be described in Examples. In the below-described processes, the starting compounds may be used in the form of salts, whereby the salts may be exemplified by pharmaceutically acceptable salts of the compounds represented by the general formula (I), and the like.

The compounds represented by the general formula (I) can be produced by allowing a compound represented by the general formula (II): 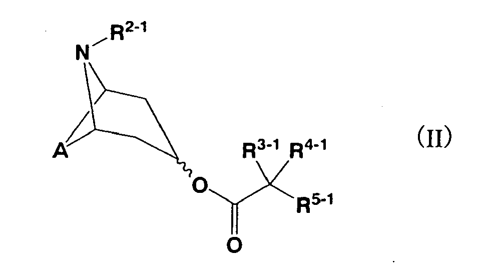 (wherein R²⁻¹, R³⁻¹, R⁴⁻¹ and R⁵⁻¹ are as defined for R², R³, R⁴ and R⁵., whereby a carboxyl, hydroxyl, amino or mercapto group which are included in the groups represented by R²⁻¹, R³⁻¹, R⁴⁻¹ and R⁵⁻¹ is understood to be protected, whenever required to be so) to react with a compound of the general formula (III):

R¹⁻¹-X (III)

(wherein R¹⁻¹ is as defined for R¹, whereby a carboxyl, hydroxyl, amino or mercapto group which are included in the groups represented by R¹⁻¹ is understood to be protected, whenever required to be so; miscellaneous symbols are as defined above), followed by deprotecting reaction for the protective groups, if necessary.

The reaction of the compound represented by the general formula (II) with the compound represented by the general formula (III) constitutes a substitute-introducing reaction.

In the case of the compound represented by the general formula (III) where R¹⁻¹ is an optionally substituted alkyl group or a group represented by the formula: 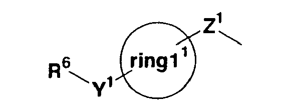 (wherein Z¹ denotes a methylene group which may be substituted with 1 to 2 substituents (a carboxyl, hydroxy, amino or mercapto group as included in the said substituents is understood to be protected, wherever required to be so); ring1¹ and Y¹ are as defined for ring1 and Y, whereby a carboxyl, hydroxy, amino or mercapto group which are included in the groups represented by ring1¹ and Y¹ is understood to be protected, whenever required to be so; and miscellaneous symbols are as defined above), such compound can be produced by the below-described method.

Namely, the method can be conducted into practice, for example, by allowing a compound represented by the general formula (II) and a compound represented by the general formula (III) to undergo reaction in an organic solvent (e.g., use is made of aromatic hydrocarbons, such as benzene, toluene, xylene, etc., halogenated hydrocarbons, such as dichloromethane, chloroform, etc., saturated hydrocarbons, such as hexane, heptane, cyclohexane, etc., ethers, such as diethyl ether, tetrahydrofuran, dioxane, etc., ketones, such as acetone, methylethyl ketone, etc., nitriles, such as acetonitrile, etc., sulf oxides, such as dimethyl sulf oxide, etc. , acid amides, such as N,N-dimethylformamide, dimethylacetamide, 1,3-diemthyl-2-imidazolizinone, etc., esters, such as ethyl acetate, etc. These solvents may be used singly or after numerous kinds thereof, such as kind two kinds or more thereof, being mixed at appropriate ratios, for example, at ratios of 1:1 to 1:10) at about -78 to 100° C. This reaction is preferably carried out under anhydrous conditions in the presence of an inert gas.

The deprotecting reactions for the protective groups for a carboxyl, hydroxyl, amino or thiol group are well known, and may be exemplified by:
(1) Alkali hydrolysis,
(2) Deprotecting reaction under acidic conditions,
(3) Deprotecting reaction through hydrogenolysis,
(4) Deprotecting reaction of silyl group,
(5) Deprotecting reaction with use of a metal,
(6) Deprotecting reaction with use of a metal complex, and the like.

Specific descriptions are to be given below on these procedures, whereby:
(1) The deprotecting reaction through alkali hydrolysis is carried out, for example, in an organic solvent (e.g., methanol, tetrahydrofuran, dioxane, etc.) at a temperature of about 0 to 40°C, while using a hydroxide of an alkali metal (sodium hydroxide, potassium hydroxide, lithium hydroxide, etc.), a hydroxide of an alkaline earth metal (barium hydroxide, calcium hydroxide, etc.) or a carbonate (sodium carbonate, potassium carbonate, etc.), or their aqueous solutions or mixtures thereof;
(2) The deprotecting reaction under acidic conditions is effected, for example in an organic solvent (dichloromethane, chloroform, dioxane, ethyl acetate, anisole, etc.) in the presence of an organic acid (acetic acid, trifluroacetic acid, methanesulfonic acid, p-tosyl acid, etc.) or an inorganic acid (hydrogen chloride, sulfuric acid, etc.) or a mixture thereof (hydrogen bromide/acetic acid, etc.) in the presence of absence of 2,2,2-trifluoroethanol at a temperature of about 0 to 100° C;
(3) The deprotecting reaction through hydrogenolysis is performed, for example, in a solvent [ether-based ones (e.g., tetrahydrofuran, dioxane, dimethoxyethane, diethyl ether, etc.), alcohol-based ones (methanol, ethanol, etc.), benzene-based ones (e.g., benzene, toluene, etc.), ketone-based ones (acetone, methylethyl ketone, etc.), nitriles-based ones (acetonitrile, etc.), amide-based ones (dimethylformamide, etc.), water, ethyl acetate, acetic acid or solvent mixtures of not less than two kinds thereof, etc.], in the presence of a catalyst (palladium-carbon, palladium black, palladium hydroxide-carbon, platinum oxide, Raney-nickel, etc.) under atmospheric or applied pressure or under coexistence of ammonium formate at a temperature about 0 to 200° C;
(4) The deprotecting reaction of a silyl group is carried out, for example, in an organic solvent (tetrahydrofuran, acetonitrile, etc.) being miscible with water at a temperature of about 0 to 40°C with use of tetrabutylammonium fluoride;
(5) The deprotecting reaction with use a metal is effected, for example, in an acidic solvent (acetic acid, a buffer solution with pH of ca. 4.2 to 7.2 or their solution mixtures with organic solvents, such as tetrahydrofuran, etc.) in the presence of powdered zinc at a temperature of about 0 to 40° C, while irradiating with ultrasonic wave, if necessary; and
(6) The deprotecting reaction with use of a metal complex is performed, for example, in an organic solvent (dichloromethane, dimethylformamide, tetrahydrofuran, ethyl acetate, acetonitrile, dioxane, ethanol, etc.), water or a solvent mixture thereof in the presence of a trapping reagent (tributyltin hydride, triethylsilane, dimedone, morpholine, diethylamine, pyrrolidine, etc.), organic acid (acetic acid, formic acid, 2-ethylhexanoic acid, etc.), and/or organic acid salt (sodium 2-ethylhexanaoate, potassium 2-ethylhexanoate, etc.) in the presence or absence of a phohsphine-based reagent (triphenylphosphine, etc.) at a temperature of about 0 to 40° C, while using a metal complex [e.g., tetrakistriphenylphosphine palladium (0), bis(triphenylphosphine)- palladium (II) dichloride, palladium (II) acetate, tris(triphenylphosphine)rhodium (I) chloride, etc.].

In addition to the above-described procedures, the deprotecting reaction can also be carried out, for example, in accordance with the procedures described in T. W. Greene, Protective Groups in Organic Synthesis, Wiley, New York, 1999. The protective group for a carboxyl group may be exemplified by methyl, ethyl, allyl, t-butyl, trichloroethyl, benzyl (Bn), phenacyl, p-methoxybenzyl, trityl and 2-chlorotrityl groups, or solid supports having these structures bonded thereto, and the like.

The protective group for a hydroxyl group includes, for example, methyl, trityl, methoxymethyl (MOM), 1-ethoxyethyl (EE), methoxyethoxymethyl (MEM), 2-tetrahydropyranyl (THP), trimethylsilyl (TMS), triethylsilyl (TES), t-butyldimethylsilyl (TBDMS), t-butyldiphenylsilyl (TBDPS), acetyl (Ac), pivaloyl, benzoyl, benzyl (Bn), p-methoxybenzyl, allyloxycarbonyl (Alloc) and 2,2,2-trichloroethoxycarbonyl (Troc) groups, and the like.

The protective group for an amino group may be exemplified by benzyloxycarbonyl, t-butoxycarbonyl, allyloxycarbonyl (Alloc), 1-methyl-1-(4-biphenyl)ethoxycarbonyl (Bpoc), trifluroacetyl, 9-fluorenylmethoxycarbonyl, benzyl (Bz), p-methoxybenzyl, benzyloxymethyl (BOM) and 2-(trimethylsilyl)ethoxymethyl (SEM) groups, etc.

The protective group for a thiol group includes, for example, benzyl, methoxybenzyl, methoxymethyl (MOM), 2-tetrahydropyranyl (THP), diphenylmethyl and acetyl (Ac) groups, and the like.

The protective groups for a carboxyl, hydroxy, amino or thiol group other than the above-mentioned ones are not particularly limited, only if they can be easily and selectively eliminated. For example, the protective groups described in T. W. Greene, Protective Groups in Organic Synthesis, Wiley, New York, 1999. can be used.

As is easily understandable to a person of ordinary skill in the art, properly selected use of these deprotecting reactions can facilitate the objective compound of the present invention to be produced.

The compounds represented by the general formula (II) can be produced in accordance with the method as illustrated in the reaction scheme I, in which E denotes a eliminating or leaving group (e.g., halogen atoms, methyloxy and tosyloxy groups, etc.) and R¹⁰⁰ are a hydrogen atom or a C1-6 alkyl group, with the remaining symbols being as defined above.

The protecting reaction, which is to be carried out as the case may be, can be conducted in accordance with the procedures as described in T. W. Greene, Protective Groups in Organic Synthesis, Wiley, New York, 1999.

The deprotecting reaction, which is to be carried out as the case may be, can be performed in accordance with the same procedures as described above in the deprotecting reactions for the protective groups.

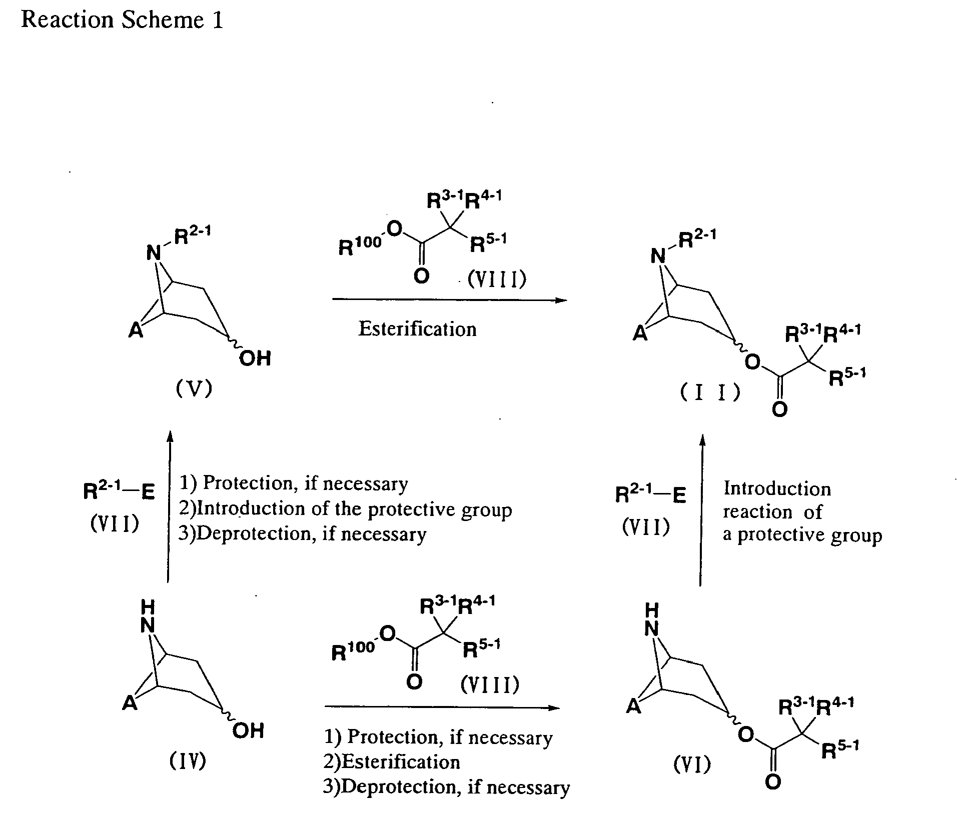

The reaction from a compound represented by the general formula (IV) to a compound represented by the general formula (V) and the reaction from a compound represented by the general formula (VI) to a compound represented by the general formula (II) each constitute a substituent introducing reaction.

For example, the compound represented by the general formula (II) where R²⁻¹ is an optionally substituted alkyl group for example, can be produced in accordance with the below-described method.

Namely, the method is conducted into practice, for example, by allowing a compound represented by the general formula (IV) and a compound represented by the general formula (VII), or a compound represented by the general formula (VI) and a compound represented by the general formula (VII) to undergo reaction in an organic solvent (for example, use is made of aromatic hydrocarbons, such as benzene, toluene, xylene, etc., halogenated hydrocarbons, such as dichloromethane, chloroform, etc., saturated hydrocarbons, such as hexane, heptane, cyclohexane, etc., ethers, such as diethyl ether, tetrahydrofuran, dioxane, etc., ketones, such as acetone, methylethyl ketone, etc., nitriles, such as acetonitrile, etc., sulfoxides, such as dimethyl sulfoxide, acid amides, such as N,N-dimethylformamide, dimethylacetamide, 1,3-dimethyl-2-imidazolidinone, etc., esters, such as ethyl acetate, etc.; these solvents may be used singly or after mixing numerous kinds, or two or more kinds, of these at an appropriate ratio, for example, about 1:1 to 1:10) in the presence or absence of a base [e.g., hydrides of alkali or alkaline earth metals, such as sodium hydride, potassium hydride, etc., alkyllithiums, such as butyllithium, sec-butyllithium, t-butyllithium, etc., alkoxides of alkali metals, such as sodium methoxide, sodium ethoxide, etc., inorganic bases, inclusive of alkali metals, such as metallic sodium, metallic potassium, etc., and the like, carbonates, such as cesium carbonate, sodium carbonate, potassium carbonate, etc., alkylamines, such as triethylamine, tributylamine, N,N-diisopropylethylamine, N-methylmorpholine, etc., aromatic amines, such as N,N-dimethylaniline, pyridine, lutidine, collidine, 4-(dimethylamino)pyridine, etc., organic bases, such as DBU (1,8-diazabicyclo[5,4,0]undecene-7), etc., and miscellaneous ones, such as lithium diisopropylamide, lithium hexamethyldisilazide, potassium hexamethyldisilazide, sodium hexamethyldisilazide, etc. at a temperature of about -78 to 100°C.

The reaction is preferably carried out in the presence of an ienrt gas under the anhydrous conditions.

The reaction from a compound represented by the general formula (IV) to a compound represented by the general formula (VI) and the reaction from a compound represented by the general formula (V) to a compound represented by the general formula (II) each constitute a esterification reaction.

The esterification reaction may be exemplified by:
(1) The procedure of esterification with use of an acid halide,
(2) The proceudre of esterification with use of a mixed acid anhydride,
(3) The procedure of esterification with use of a condensing agent, or
(4) The procedure of transesterification.

Specific descriptions are to be given below on these procedures:
(1) The procedure of esterification with use of an acid halide is carried out, for example, by reacting a carboxylic acid with an acid halidation agent (oxalyl chloride, thionyl chloride, etc.) in an organic solvent (chloroform, dichloromethane, diethyl ether, tetrahydrofuran, etc.) or without solvent at a temperature of -20°C to a refluxing temperature, followed by reaction of the resultant acid halide with an alcohol in an organic solvent (e.g., chloroform, dichloromethane, diethyl ether, tetrahydrofuran, etc.) in the presence of a base (e.g., pyridine, triethylamine, dimethylaniline, dimethylaminopyridine, diisopropylethylamine, etc.) at a temperature of about 0 to 40° C. Additionally, the procedure can be conducted by reaction with an acid halide in an organic solvent (dioxane, tetrahydrofuran, etc.) while using an aqueous alkali solution (aqueous sodium hydrogencarbonate solution or aqueous sodium hydroxide solution, etc.) at a temperature of about 0 to 40° C;
(2) The procedure of esterification with use of a mixed acid anhydride is effected, for example, by reacting a carboxylic acid with an acid halide (pivaloyl chloride, tosyl chloride, mesyl chloride, etc.) or an acid derivative (ethyl chloroformate, isobutyl chloroformate, etc.) in an organic solvent (chloroform, dichloromethane, diethyl ether, tetrahydrofuran, etc.) or without solvent in the presence of a base (pyridine, triethylamine, dimethylaniline, dimethylaminopyridine, diisopropylethylamine, etc.) at a temperature of about 0 to 40° C, followed by reaction of the resultant mixed acid anhydride with an alcohol in an organic solvent (e.g., chloroform, dichloromethane, diethyl ether, tetrahydrofuran, etc.) at a temperature of about 0 to 40° C.;
(3) The procedure of esterification with use of a condensing agent is carried out, for example, by allowing a carboxylic acid and an alcohol to undergo a reaction in an organic solvent (chloroform, dichloromethane, dimethylformamide, diethyl ether, tetrahydrofuran, etc.) or without solvent in the presence or absence of a base (pyridine, triethylamine, dimethylaniline, dimethylaminopyridine, etc.) at a temperature of about 0 to 40° C, while using a condensing agent [1,3-dicyclohexylcarbodiimide (DCC), 1-ethyl-3-[3-(dimethylamino)propyl]carbodiimide (EDC), 1,1'-carbonyldiimidazole (CDI), 2-chloro-1-methylpyridinium iodide, 1-propylphosphonic acid cyclic anhydride (PPA), etc.], with or without use of 1-hydroxybenzotriazole (HOBt); and
(4) The procedure of transesterification is effected, for example, by allowing an ester and an alcohol to undergo a reaction in an organic solvent [e.g., ether-based ones (e.g., tetrahydrofuran, dioxane, dimethoxyethane, diethyl ether, etc.), benzene-based ones (e.g., benzene, toluene, etc.), ketone-based ones (e.g., acetone, methylethyl ketone, etc.), nitrile-based ones (e.g., acetonitrile, etc.), amide-based ones (e.g., dimethylformamide, etc.), halogen-based ones (e.g., dichloromethane, chloroform, dichloroethane, etc.) or without solvent in the presence or absence of a base [for example, hydrides of alkali metals or alkaline earth metals, such as sodium hydride, potassium hydride, etc., alkyllithiums, such as butyllithium, sec-butyllithium, t-butyllithium, etc., alkoxides of alkali metals, such as sodium methoxide, sodium ethoxide, etc., inorganic bases, inclusive of alkali metals, such as metallic sodium, metallic potassium, etc. , and the like, carbonates, such as cesium carbonate, sodium carbonate, potassium carbonate, etc., alkylamines, such as triethylamine, tributylamine, N,N-diisopropylethylamine, N-methylmorpholine, etc., aromatic amines, such as N,N-dimethylaniline, pyridine, lutidine, collidine, 4-(dimethylamino)pyridine, etc., organic bases, such as DBU (1,8-diazabicyclo[5,4,0]undecene-7), etc., and miscellaneous ones, such as lithium diisopropylamide, lithium hexamethyldisilazide, potassium hexamethyldisilazide, sodium hexamethyldisilazide, etc.) at a temperature of about 0°C to the refluxing point of the solvent used.

These reactions as enumerated above under (1) to (4) all are desirably carried out in an atmosphere of an inert gas (argon, nitrogen, etc.) under the anhydrous conditions. The compounds represented by the general formula (III) are known or can be easily produced by employing the known methods, such as the methods as described in "Comprehensive Organic Transformations: A Guide to Functional Group Preparations, 2nd Edition (Richard C. Larock, John Wiley & Sons Inc., 1999)" or the methods as delineated in Examples.

The compounds represented by the general formulae (IV), (V), (VII) and (VIII), which are used as a starting compound in the reaction scheme 1, are known or can be easily produced by employing the known methods, such as the methods as described in "Comprehensive Organic Transformations: A Guide to Functional Group Preparations, 2nd Edition (Richard C. Larock, John Wiley & Sons Inc., 1999)".

Out of the compounds represented by the general formula (IV), for example, nortropine can be purchased from Boehlinger Ingelheim Inc., and others, while among the compounds represented by the general formula (V), for example, tropine can be purchased from Kantoh Chemical Co., Tokyo Kasei Co. , etc.

Out of the compounds represented by the general formula according to the present invention, any other compounds than the above-described compounds can be produced by employing in combination the methods as described in Examples given herein or the known methods, such as those as described in "Comprehensive Organic Transformations: A Guide to Functional Group Preparations, 2nd Edition (Richard C. Larock, John Wiley & Sons Inc., 1999)".

In each of the reactions mentioned herein, the reactions accompanied by heating can be performed by use of a water bath, oil bath, sand bath or microwave.

In each of the reactions described herein, appropriate use may be made of solid-phase supported reagents consisting of the reagents supported on high molecular polymers (e.g., polystyrene, polyacrylamide, polypropylene, polyethylene glycol, etc.).

In each of the reactions described herein, the reaction product can be purified by ordinary purification means, such as distillation under atmospheric pressure or reduced pressure, high performance liquid chromatography with use of silica gel or magnesium silicate, thin-layer chromatography, ion exchange resins, scavenger resins or column chromatography, or the procedure of washing, recrystallization, etc. Such purification may be done for each reaction step or after completion of several reaction steps.

### [Toxicity]

The compounds represented by the general formula (I), their salts, solvation product thereof, or prodrugs thereof (herein after in some instances referred to briefly as "compounds of the present invention") show an extremely lowered degree of toxicity and are adequately safe to be used as a medicinal drug.

### [Application to Pharmaceutical Preparations]

The compounds of the present invention antagonize the muscarinic receptors. Furthermore they produce the effect on the targeted sites, thereafter undergoing quick inactivation, and are useful as a prophylactic and/or therapeutic agent with reduced adverse reactions (for example, urinary obstruction, thirst, tachycardia, gastrointestinal disorders, glaucoma, etc.) against disease mediated by the muscarinic receptor. The targeted site includes the airways and/or lungs, and is preferably the airways.

The method for measuring antagonistic activity of the compound of the present invention is described, for example, in European Journal of Pharmacology, vol. 250, No. 2, pp. 267-279, 1993, and others.

In the present invention, the mode of inactivation of a compound may be exemplified by metabolism, excretion, absorption, distribution, etc., with the metabolism being preferred.

In the present invention, the site of inactivation of the compound includes, for example, blood, or the lung, liver, or gastrointestinal tract and the like, preferably blood, or the lung, and more preferably blood.

In the present invention, the sample of inactivation of the compound includes, for example, the whole blood, blood plasma, blood serum or their diluted solutions, as well as the lungs, lung homogenates, diluted solutions of the lung homogenates, and the like.

In the present invention, the length of time required for the inactivation of the compound of the present invention is preferably shortened periods of time, more preferably within about 15 min., further preferably within 1 min., further more preferably within 30 sec. , and particularly preferably within 15 sec. The method for measuring the length of time required for inactivation is described, for example, in Kiso to Rinshoh (Fundamentals & Clinic), vol. 31, No. 9, pp. 2913-2923, 1997, and the like.

As used herein, the disease mediated by the muscarinic receptor includes, for example, respiratory diseases (e.g., chronic occlusive pulmonary diseases (COPD), asthma, etc.), and preferably is chronic occlusive pulmonary disease or asthma.

In the present invention, the effective dose to suppress the airway constriction refers to the minimum dose at which a compound can act to suppress not less than ca. 80 % of the airway constriction induced in a mammal by a cholinergic substance ([e.g., metacholine, acetylcholine, etc. (at a dose of 1 to 100 pg/kg) at a point of time falling within the length of time of about 60 minutes after administration of the compound, provided, however, that such effective dose shall be in such a range as may allow the compound, when applied at a dose equivalent to one third of the effective dose, to exhibit less than ca. 80 % of the airway constriction suppression ratio at any points of time falling within the length of time of about 60 minutes after administration of the compound. The mammal includes, for example, humans, dogs, monkeys, mice, guinea-pigs and rats, etc.

In the present invention, the no-effective dose against the bladder constriction refers to the maximum dose at which a compound can act to suppress less than ca. 20 % of the bladder constriction as induced in a mammal by a cholinergic substance [e.g., metacholine, acetyl choline, etc. (1 to 100 µg/kg)] at a point of time within about 60 minutes after administration of the compound, provided, however, that such maximum dose shall be in such a range as may allow the compound, when applied at a dose equivalent to threefold of the no-effective dose, to exhibit not less than ca. 20 % of the airway-constriction suppression ratio at all the points of time within about 60 minutes after administration of the compound. The mammal includes, for example, humans, dogs, monkeys, mice, guinea-pigs and rats, etc.

In the present invention, the expression "failure to exhibit suppressory activity against the bladder constriction" means that the compound exhibits less than ca. 20 % of the suppression ratio against the bladder constriction as induced in a mammal by a cholinergic substance [e.g., metacholine, acetyl choline, etc. (1 to 100 pg/kg)] at any points of time within about 60 minutes after administration of the compound.

In the present invention, any testing systems for evaluating the effective dose to suppress the airway constriction and the no-effective dose against the bladder constriction is not particularly limited, only if they can establish each of the above-mentioned minimum doses, and preferred are the testing systems to be described in the below-given examples of pharmacologic experiment.

In the present invention, the animals to be used in evaluating the effective dose of suppression against the airway constriction and the no-effective dose against the bladder constriction may be of the same species or of different species, with the animals of the same species being preferable.

In the present invention, the effective dose to suppress the airway constriction is preferably less than ca. one tenth of the no-effective dose against the bladder constriction, preferably less than ca. one thirtieth, more preferably less than ca. one hundredth, further preferably less than ca. one thousandth.

In the present invention, the pK_{B} value against the constriction reaction with the tracheal muscle denotes the pK_{B} value as found in the constriction reaction with the tracheal muscle removed from a mammal. The calculation method for the pKa is described, for example, in Nippon Rinshoh (Clinics in Japan), vol. 47, Special Issue, pp. 19 to 30, 1989, and the like. Such mammal may be exemplified by humans, dogs, monkeys, guinea-pigs and rats, etc. The pK_{B} value for the constriction reaction of the tracheal muscle is not less than 7, preferably not less than ca. 8, more preferably not less than ca. 9, furthermore preferably not less than ca. 10.

The compounds of the present invention may also be administered to patients as a concomitant drug in combination with other medicinal drugs for the purposes of (1) supplementation and/or enhancement of the prophylactic and/or therapeutic effects produced by the therapeutic agent of the present invention, (2) amelioration or improvement of the pharmacodynamics/absorption and reduction of the dose of the therapeutic agent of the present invention, and/or (3) alleviation of adverse reactions or side effects of the therapeutic agent of the present invention.

The concomitant drug preparation of the therapeutic agent of the present invention with other medicinal drug may be administered to a patient in the dosage form of a combination drug consisting of two ingredients formulated in one pharmaceutical preparation, or may assume the dosage forms intended for use through administration in separate pharmaceutical preparations. Adminsitration in the form of such different pharmaceutical preparations includes simultaneous and intermittent, time-lag administration. In the intermittent, time-lag administration, the therapeutic agent of the present invention may firstly be administered to a patient, followed by application of other medicinal drugs, or *vice versa*, and the respective methods of administration may be the same or different.

The above-mentioned other medicinal drug may be either lower-molecular compounds or high-molecular proteins, polypeptides, polynucleotides (DNA, RNA, genes), antisenses, decoys, antibodies, or vaccines, etc. The dose of other medicinal drug can be appropriately chosen, while taking the clinically used dose as a standard. The formulation ratio between the therapeutic agent of the present invention and other medicinal drug can be suitably chosen, depending upon the age and body weight of a subject to be treated through administration, the method and time of administration, the disease to be targeted, its symptom or conditions, the combination, etc. For example, about 0.01 to 100 parts by mass of other medicinal drug may be used against 1 part by mass of the therapeutic agent of the present invention. Other medicinal drug may be combined for administration in not less than two of any arbitrary ones. The other medicinal drug which acts to supplement and/or enhance the prophylactic and/or therapeutic effects of the therapeutic agent of the present invention is understood to comprehend not only the ones which have already been found out, but also the ones which will be discovered, on the basis of the above-mentioned mechanism.

The diseases on which the prophylactic and/or therapeutic effects works with the above described concomitant drugs are not especially limited. The diseases may be those which compensate for and/or enhance the prophylactic and/or therapeutic effect of the compounds in the present invention.

The other medicinal drugs, which act to supplement and/or enhance the prophylactic and/or therapeutic effects for respiratory diseases of the therapeutic agent of the present invention, include, for example, leucotriene receptor antagonists (cysLT1-receptor antagonists, cyLT2-receptor antagonists), antihistaminine drugs, antiallergy drugs (mediator liberation inhibitors, histamine antagonists, thromboxane synthase inhibitor drugs, thromboxane antagonists, Th2 cytokine inhibitor drugs), steroidal drugs, bronchodilators (xanthine derivatives, sympathomimetics), vaccine therapy agents, gold compound preparations, Chinese herbal medicines, non-steroidal anti-inflammatory agents (basic non-steroidal anti-inflammatory drugs, acidic non-steroidal anti-inflammatory drugs), 5-lipoxygenase inhibitor drugs, 5-lipoxygenase activating protein antagonists, leukotriene synthase inhibitors, prostaglandins, cannabinoid-2 receptor stimulants, antitussives, expectrants, phosphodiesterase inhibitors, extraction liquid from skin inflammations of a household rabbit inoculated with vaccinia virus, and the like.

Among the leukotriene receptor antagonists, the cysLT1 receptor antagonists include, for example, pranlukast hydrate, montelukast sodium, zafirlukast, MK-571, LY-203647, WY-46016, WY-48422, WY-49353, WY-49451, RG-12553, MDL-43291, CGP-44044A, RG-14524, LY-287192, LY-290324, L-695499, RPR-105735B, WAY-125007, OT-4003, LM-1376, LY-290154, SR-2566, L-740515, LM-1453, CP-195494, LM-1484, CR-3465, ablukast, pobilukast, sulukast, L-648051, RG-12525, RG-7152, SK&F-106203, SR-2640, WY-50295, iralukast sodium, verlukast, MCC-847, BAY-x-7195, ritolukast, cinalukast, CGP-44826, FK-011, YM-158, MEN-91507, KCA-757,RS-601, RS-635, S-36496, ZD-3523, DS-4574, pirodomast, AS-35, YM-57158, MCI826, NZ-107, 4414-CERM, YM-16638, Wy-48252, Wy-44329, Wy-48090, VUF-4679, tomelukast, SM-11044, SC-39070, OT-3473, N-2401, LY-243364, L-649923, doqualast, DP-1934, YM-17551, Wy-47120, VUF-K-8707, SK&F-88046, SK&F-101132, SK&F-102922, LY-137617, LY-163443, LY-302905, L-647438, L-708738, KY-234, FPL-55712, CP-288886, S-36527, CGP-35949, CS-615, MDL-19301D, SCH-40120, or ZD-3705.

The antihistamine drugs may be exemplified by diphenhydramine, diphenylpyraline hydrochloride, diphenylpyraline teoclate, clemastine fumarate, dimenhydrinate, dl-chlorpheniramine maleate, d-chloropheniramine maleate, triprolidine hydrochloride, promethazine hydrochloride, arimemazine tartarate, isothipendyl hydrochloride, homochlorcyclizine, hydroxyzine, cyproheptadine hydrochloride, levocabastine hydrochloride, astemizole, bepotastine, desloratadine, TAK-427, ZCR-2060, NIP-530, mometasone furoate, mizolastine, BP-294, andorast, auranofin, acrivastine, etc.

Among the antiallergy drugs, the mediator liberation inhibitor includes, for example, sodium cromoglycate, tranilast, amlexanox, repirinast, ibuzilast, potassium pemirolast, dazanolast, nedocromyl, cromoglycate, israpafant, etc.

Among the antiallergy drugs, the histamine antagonist may be exemplified by ketotifen fumarate, azelastine hydrochloride, oxatomide, mequitazine, terfenadine, emedastine fumarate, epinastine hydrochloride, ebastine, cetirizine hydrochloride, olopatadine hydrochloride, loratadine, fexofenadine, etc.

Among the antiallergy drugs, the thromboxane synthase inhibitor drug includes, for example, ozagrel hydrochloride, imitrodast sodium, etc.

Among the antiallergy drugs, the thromboxane antagonist includes, for example, seratrodast, ramatroban, domitroban calcium hydrate, KT-2-962, etc.

Among the antiallergy drugs, the Th2 cytokine inhibitors may be exemplified by spratast tosylate, etc.

Among the steroidal drugs, the steroidal drug for external application to the skin includes, for example, clobetasol propionate, diflorasone acetate, fluocinonide, mometasone furancarboxylate, betamethasone dipropionate, betamethasone butyrate propionate, betamethasone valerate, difluprednate, pdesonide, diflucoltolone valerate, amcinonide, halcinonide, dexamethasone, dexamethasone propionate, dexamethasone valerate, dexamethasone acetate, hydrocortisone acetate, hydrocortisone butyrate, hydrocortisone butyrate propionate, deprodone propionate, prednisolone valerate propionate, fluocinolone acetonide, beclometasone propionate, triamcinolone acetonide, flumethasone pivalate, alclometasone propionate, clobetasone butyrate, prednisolone, beclomethasone propionate, fludroxycortide, etc.

The steroidal drug for internal or injectable application may be exemplified by cortisone acetate, hydrocortisone, hydrocortisone sodium phosphate, hydrocortisone sodium succinate, fludrocortisones acetate, prednisolone, prednisolone acetate, prednisolone sodium succinate, prednisolone butyl acetate,prednisolone sodium phosphate, halopredone acetate, methyl prednisolone, methyl prednisolone acetate, methyl prednisolone sodium succinate, triamcinolone, triamcinolone acetate, triamcinolone acetonide, dexamethasone, dexamethasone acetate, dexamethasone sodium phosphate, dexamethasone palmitate, paramethasone acetate, betamethasone, etc., while the steroidal drug in the form of an inhalant includes, for example, beclomethasone propionate, fluticasone propionate, budesonide, flunisolide, triamcinolone, ST-126P, ciclesonide, dexamethasone palomitionate, mometasone furancarbonate, prasterone sulfonate, deflazacort, methylprednisolone suleptanate, methylprednisolone sodium succinate, etc.

Among the bronchodilators, the xanthine derivative may be exemplified by aminophylline, theophylline, doxophylline, sympamphylline, diprophylline, proxyphylline, cholinetheophylline, etc.

Among the bronchodilators, the sympathomimetics drug includes, for example, epinephrine, ephedrine hydrochloride, dl-methylephedrine hydrochloride, methoxyphenamine hydrochloride, isoproterenol sulfate, isoproterenol hydrochloride, orciprenaline sulfate, chloroprenaline sulfate, trimethoquinol hydrochloride, salbutamol sulfate, terbutalin sulfate, hexoprenalin sulfate, tulobuterol hydrochloride, procaterol hydrochloride, fenoterol hydrobromide, formoterol fumarate, clenbuterol hydrochloride, mabuterol hydrochloride, salmeterol xinafoate, R,R-formoterol, tulobuterol hydrochloride, pilbuterol hydrochloride, ritodrine hydrochloride, banbuterol, dopexamin hydrochloride, meradrin tartarate, AR-C68397, levosarbutamol, KUR-1246, KUL-7211, AR-C89855, S-1319, etc.

The vaccine therapy agent includes, for example, paspat, asthremedine, broncasma berna, CS-560, etc.

The gold compound preparation may be exemplified by sodium aurothiomalate, etc.

The non-steroidal anti-inflammatory drug includes, for example, aspirin, loxonin, diclofenac, celecoxib, tiaprofenic acid, alminoprofen, flurbiprofen axetil, zaltoprofen, suprofen, ketoprofen, pranoprofen, fentiazac, droxicam, ibuprofen, aceclophenac, amfenac sodium, tenoxicam, oxaprodin, piroxicam, emorfazone, tolfenamic acid, indometacin falnecil, proglumetacin maleate, sulindac, mofezolac, etodolac, lonazolac calcium, ampiroxicam, mesaladine, deflazacort, nimetsuride, etoricoxib, ketorolac trometamol, parecoxib, robenzalit disodium, auranofin, loxoprofen sodium, bucillamin, actarit, piroxicam cinnamate, nabumetone, salazosulfapyridine, lornoxicam, meloxicam, diacerein, rofecoxib, valdecoxib, etc.

Among the non-steroidal anti-inflammatory drugs, the basic non-steroidal anti-inflammatory drug includes, for example, tiaramide hydrochloride, tinoridine hydrochloride, epirizole, emorfazone, etc.

The 5-lipoxygenase inhibitor drug includes, for example, zileuton, docebenone, pilipost, SCH-40120, WY-50295, E-6700, ML-3000, TMK-688, ZD-2138, dalbferon mesylate, R-68151, E-6080, DuP-654, SC-45662, CV-6504, NE-11740, CMI-977, NC-2000, E-3040, PD-136095, CMI-392, TZI-41078, Orf-20485, IDB-18024, BF-389, A-78773, TA-270, FLM-5011, CGS-23885, A-79175, ETH-615, etc.

The 5-lipoxygenase activating protein antagonist may be exemplified by MK-591, MK-886, etc.

The leukotriene synthase inhibitor drug includes, for example, auranofin, proglumethacin maleate, L-674636, A-81834, UPA-780, A-93178, MK-886, REV-5901A, SCH-40120, MK-591, Bay-x-1005, Bay-y-1015, DTI-0026, amlexanox, E-6700, etc.

The prostaglandins (hereinafter referred to briefly as "PG") include, for example, PG receptor agonists, PG receptor antagonists, etc.

The PG receptor may be exemplified by PGE recptors (EP1, EP2, EP3, EP4), PGD receptors (DP, CRTH2), PGF recptor (FP), PGI receptor (IP), TX receptor (TP), etc.

The antitussive drug includes, for example, codeine phosphate, dihydrocodeine phosphate, oxymetebanol, dextromethorphan hydrogenbromide, pentoxybelin citrate, dimemorphan phosphate, oxeladin citrate, chloperastine, benproperine phosphate, clofedanol hydrochloride, fominoben hydrochloride, noscapine, tipemidine hibenzoate, eprazinone hydrochloride, plantago extract, etc.

The expectrant drug includes, for example, foeniculated ammonia spirit, sodium hydrogencarbonate, potassium iodide, bromhexine hydrochloride, cherry-tree bark extract, carbocysteine, fudosteine, ambroxol hydrochloride, controlled release preparation of ambroxol hydrochloride, methylcysteine hydrochloride, acetyl cysteine, L-ethylcysteine hydrochloride, tyloxapol, etc.

The phosphodiesterase inhibitor drug may be exemplified by doxofylline, roflumilast, exisulind, cilomilast, arofylline, ONO-6126, pumafentrine, BAY-19-8004, OSI-461, GW-842470, atizoram, cipamfylline, GRC-3886, YM-976, SCH-351591, etc.

The said other drugs are preferably cysLT1 receptor antagonists, steroidal drugs or sympathomimetic drugs.

The dosage form, which is aimed at conducting the present invention into practice, may be in the form of etiher a pharmaceutical preparation comprising the compound of the present invention and other drug for supplementation and/or enhancement of the therapeutic effect of the said compound formulated in one dosage form, or a pharmaceutical preparation comprising each of the ingredients processed individually into separate dosage forms. Such processing into the dosage forms can be carried out in accordance with the known method.

The compound of the present invention can be incorporated with the additives which are allowable as a medicinal drug, as the case may be, and processing into the dosage form can be performed as a single dosage form or as a combination dosage form with use of the extensively employed processing technology.

The concomitant drug consisting of the compound of the present invention with other drug(s), when used for the above-mentioned purposes, is ordinarily administered to a subject locally or parenterally.

The compound of the present invention, whose dose may vary depending upon the age and body weight of a patient, symptom, therapeutic effect, method or route of administration, duration of the treatment, etc., is generally administered to a human adult parentereally in a dose of about 0.1 ng to 100 mg each time, preferably 0.1 µg to 100 mg, further preferably about 10 µg to 10 mg, more preferably about 100 µg to 1 mg, once to several times a day, preferably once to three times a day, further preferably once to twice a day, more preferably once a day.

As has been described in the above, naturally, the dose may fluctuate according to various conditions, and a dose lower than the above-described dose may in some instances be adequate, whereas a dose in excess of the dose range may in some cases be required.

The pharmaceutical preparation for parenteral use includes, for example, an inhalant and the like. Such dosage forms may be in the form of a controlled-release preparation, such as an accelerated-release or sustained-release preparation, etc. These dosage forms can be produced in accordance with the known method, for example, the methods described in The Japanese Pharmacopeia, etc.

The inhalant intended for use through parenteral administration comprehends an aerosol, powder for inhalation, liquid for inhalation (e.g., a solution for inhalation, suspension for inhalation, etc.) or capsule-shaped inhalant, whereby the said liquid for inhalation may assume such a form as may be used after being dissolved or suspended in water or other suitable medium on the occasion of use. These inhalants can be applied by use of an appropriate container and, for example, use can be made of a spraying apparatus or device (e. g. , an atomizer, nebulizer), etc., when a liquid preparation for inhalation is given to a patient, whereas use may be made of an application device through inhalation for a powdered drug, etc. on the occasion of adminsitration of a powder for inhalation.

These inhalants are produced in accordance with the known methods, for example, by rendering the compound of the present invention into a powder or liquid form, and formulating the same in a propellant and/or carrier for inhalation, followed by filling into a suitable container for inhalation. When the compound of the present invention is pulverized, pulverization is effected in accordance with the conventional procedures. For example, the compound of the present invention is finely pulverized together with lactose, starch, magnesium stearate, etc. to produce a uniform mixture or granules, followed by processing into a powdered dosage form. In converting the compound of the present invention into the liquid state, additionally, the said compound may be dissolved, for example, in a liquid carrier, such as water, physiological saline or organic sovlent, etc. As a propellant, there may be utilized the conventionally known propellants, such as alternatives for chlorofluorocarbons, liquefied gas propellants (e.g., fluorinated hydrocarbons, liquefied petroleum gas, diethyl ether, dimethyl ether, etc.), compressed gases (e.g., soluble gases (e.g., carbon dioxide, nitrous oxide, etc.), insoluble gases (e.g., nitrogen gas, etc.), and the like.

The inhalant may further be suitably incorporated with additives, as the case may be. The additive may be any additives, only if they are generally utilized, and for example, there are used solid excipients (e.g., white sugar, lactose, glucose, mannit, sorbit, maltose, cellulose, etc.), liquid excipients (e.g., propylene glycol, etc.), binders (e.g., starch, dextrin, methylcellulose, hydroxypropyl cellulose, polyvinylpyrrolidone, polyethylene glycol, white sugar, etc.), lubircants (e.g., magnesium stearate, light anhdyrous silicic acid, talc, sodium lauryl sulfate, etc.), corrigents (e.g., citric acid, menthol, ammonium glycyrrhizate, glycine, orange powder, etc.), preservatives (e.g., sodium benzoate, sodium hydrogensulfate, methyl paraben, propyl paraben, etc.), stabilizers (e.g., citric acid, sodium citrate, etc.), suspending agents or emulsifeirs (e.g., methylcellulose, polyvinylpyrrolidone, polyvinyl alcohol, lecithin, sorbitan trioleate, etc.), dipersants (e.g., surfactants, etc.), solvents (e.g., water, etc.), isotonic agents (e.g., sodium chloride, concentrated glycerol, etc.), pH regulating agents (e.g., hydrochloric acid, sulfuric acid, etc.), solubilizers (e.g., ethanol, etc.), antiseptics (e.g., Benzalkonium chloride, parabens, etc.), coloring agents, buffering agents (e.g., sodium phosphate, sodium acetate, etc.), thickening agents (e.g., carboxyvinyl polymers, etc.), absorption accelerators, and the like. For example, the liquid preparation for inhalation is produced by suitably selecting a preservative, coloring agent, buffering agent, isotonic agent, thickening agent, absorption accelerator, etc., as the case may be. The powder pharmaceutical preparation for inhalation is produced by appropriately selecting a lubricant, binder, excipient, coloring agent, preservative, absorption accelerator (bile salts, chitosan, etc.) and the like.

Furthermore, the inhalant may be incorporated with biodegradable polymers in order to provide the compound of the present invention with the controlled release property. The biodegradable polymer includes, for example, polymers of fatty acid esters or their copolymers, polyacrylates, polyhydroxybutyrates, polyalkylene oxalates, polyorthoesters, polycarbonates and polyamino-acids, and these can be used solely as one kind thereof or as a mixture of not less than two kinds thereof. Also, phospholipids, such as yolk lecithin, etc. , chitosan and the like may be utilized. The polymers of fatty acid esters and their copolymers may be exemplified by polylactic acid, polyglycolic acid, polycitric acid, polymalic acid and copolymers from lactic acid and glycolic acid, and these can be used solely as one kind thereof or as a mixture of not less than two kinds thereof. Besides, there can be used one kind or mixtures of not less than two kinds out of poly-a-cyanoacrylate, poly-β-hydroxybutyric acid, polymethylene oxalate, polyiorthoester, polyorthocarbonate, polyethylenecarbonate, poly-γ-benzyl-L-glutamate and poly-L-alanine. Preferable are polylactic acid, polyglycolic acid or copolymers from lactic acid and glycolic acid, and more preferable are copolymers from lactic acid and glycolic acid. Moreover, there may be prepared the microspheres or nanospheres which consist of a drug substance encapsulated with a biodegradable piolymer, such as polylactic acid, polyglycolic acid or copolymers from lactic acid and glycolic acid.

### Examples

Examples are to be described below to illustrate the present invention in detail, but the present invention is not understood to be limited to them.

The parenthesized solvents as indicated in the descriptions relating to chromatographic separation and under the heading of TLC denote the elution solvents or development solvents as used, with the ratio being on a volume basis.

The parenthesized sovlents as indicated under the heading of NMR denote the solvent used in the measurement.

### Example 1

### Ethyl (2E)-3-(4-formylphenyl)acrylate:

Added to a N,N-dimethylformamide solution (80 mL) of 4-formylcinnamic acid (2.9 g) were potassium carbonate (2.27 g) and ethyl iodide (1.4 mL), followed by stirring at room temperature for 5 hours. The reaction mixture was charged in water, and extraction was effected with ethyl acetate. The organic layer was washed with water and an aqueous saturated sodium chloride solution, dried over anhydrous sodium sulfate and concentrated. The resultant residue was purified on a silica-gel column chromatography (n-hexane:ethyl acetate = 4:1) to give the subject title compound (1.45 g) showing the following physico-chemical values:
TLC: Rf 0.51 (n-hexane:ethyl acetate = 4:1);
¹H-NMR (CDCl₃): d 1.35, 4.29, 6.55, 7.64-7.76, 7.91, 10.03.

### Example 2

### Ethyl 3-[4-(hydroxymethyl)phenyl]propanoate

10 % Palladium carbon (70 mg) was added to an ethanol solution (15 mL) of the compound (1.44 g), as produced in Example 1, under an argon atmosphere, followed by stirring at room temperature for 4 hours under a hydrogen atmosphere. The reaction mixture was filtered through Cellite (the registered trademark), and the filtrate was concentrated. The resultant residue was purified on a silica-gel column chromatography (n-hexane:ethyl acetate = 4:1) to give the subject title compound (1.35 g) showing the following physico-chemical values:
TLC: Rf 0.12 (n-hexane:ethyl acetate = 4:1);
¹H-NMR (CDCl₃): d1.24, 1.61, 2.61, 2.95, 4.13, 4.66, 7.20, 7.29.

### Example 3

### Ethyl 3-[4-(bromomethyl)phenyl]propanoate

Carbon tetrabromide (1.27 g) and triphenylphosphine (805 mg) were added to a methylene chloride solution (5 mL) of the compound (533 mg) as produced in Example 2, followed by stirring at room temperature for 30 min. An aqueous saturated sodium hydrogencarbonate solution was added to the reaction mixture, and extraction was effected with ethyl acetate. The organic layer was washed with an aqueous saturated sodium chloride solution, dried over anhydrous sodium sulfate and concentrated. The resultant residue was purified on a silica gel column chromatography (n-hexane:ethyl acetate = 9:1) to give the subject title compound (667 mg) showing the following physico-chemical values:
TLC: Rf 0.61 (n-hexane:ethyl acetate = 4:1);
¹H-NMR (CDCl₃): d 1.23, 2.61, 2.94, 4.13, 4.48, 7.18, 7.32.

### Example 4

(1R,3r,5S)-8-Methyl-8-azabicyclo[3.2.1]octa-3-yl hydroxy(diphenyl)acetate

Dissolved in toluene (30 mL) were tropine (3.11 g) and methyl benzylate (6.93 g). Sodium hydride (60 %, in oil, 890 mg) was added to the reaction mixture under ice-cooling, followed by refluxing for 6 hours. The reaction mixture was left cooled and then admixed with water, followed by extraction with ethyl acetate. The organic layer was washed with water and an aqueous saturated sodium chloride solution, successively, dried over anhydrous sodium sulfate and concentrated. The resultant residue was washed with ethyl acetate to give the subject title compound (5.03 g) showing the following physico-chemical values:
TLC: Rf 0.49 (n-butanol:acetic acid:water = 4:2:1);
¹H-NMR (CDCl₃): d 1.16, 1.66, 2.14, 2.96, 4.34, 5.18, 7.37.

### Example 5

(1R,3r,5S,8s)-8-[4-(3-Ethoxy-3-oxopropyl)benzyl]-3-{[hydrox y(diphenyl)acetyl]oxy}-8-methyl-8-azoniabicyclo[3.2.1]-octane bromide

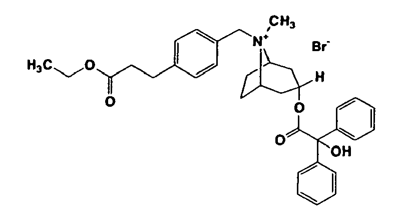

Dissolved in a solvent mixture of methylene chloride (0.5 mL) and acetone (1 mL) was the compound (100 mg) as produced in Example 4. The reaction mixture was admixed with the compound (100 mg) as produced in Example 3, followed by stirring at room temperature for one day. The resultant solid was obtained by filtration and washed with acetone under heating to give the subject title compound (99.6 mg) showing the following physico-chemical values:
TLC: Rf 0.63 (n-butanol:acetic acid:water = 4:2:1);
¹H-NMR (CD₃OD): d 1.20, 1.69-1.80, 1.86-1.98, 2.24-2.37, 2.53-2.71, 2.91-3.01, 3.71-3.84, 4.08, 4.35, 5.24, 7.28-7.49.

### Examples 5 (1) to (6)

The same procedure as described in Example 5 was crried out, while using the corresponding halide in place of the compound as produced in Example 3 to give the compounds of the present invention showing the following physico-chemical values:

### Example 5 (1)

(1R,3r,5S,8s)-3-{[Hydroxy(diphenyl)acetyl]oxy}-8-[4-(3-methoxy-3-oxopropyl)benzyl]-8-methyl-8-azoniabicyclo-[3.2.1]octane bromide
TLC: Rf 0.56 (n-butanol:acetic acid:water = 4:2:1);
¹H-NMR (DMSO-d₆): d 1.59-1.70, 1.71-1.83, 2.19-2.31, 2.49-2.57, 2.65, 2.83-2.92, 3.58, 3.67-3.75, 4.32, 5.12, 6.77, 7.27-7.44.

### Example 5 (2)

(1R,3r,5S,8s)-8-[3-(2-Ethoxy-2-oxoethoxy)benzyl]-3-{[hydroxy(diphenyl)acetyl]oxy}-8-methyl-8-azoniabicyclo-[3.2.1]octane bromide
TLC: Rf 0.59 (n-butanol:acetic acid:water = 4:2:1);
¹H-NMR (DMSO-d₆): d 1.21, 1.57-1.71, 1.71-1.84, 2.17-2.32, 2.49-2.61, 2.89, 3.68-3.78, 4.16, 4.32, 4.82, 5.12, 6.77, 7.00-7.12, 7.24-7.48.

### Example 5 (3)

(1R,3r,5S,8s)-8-[3-(3-Ethoxy-3-oxopropyl)benzyl]-3-{[hydroxy(diphenyl)acetyl]oxy}-8-methyl-8-azoniabicyclo-[3.2.1]octane bromide
TLC: Rf 0.61 (n-butanol:acetic acid:water = 4:2:1);
¹H-NMR (CD₃OD): d 1.19, 1.69-1.81, 1.86-1.99, 2.24-2.40, 2.54-2.70, 2.92-3.01, 3.72-3.85, 4.06, 4.37, 5.24, 7.28-7.47.

### Example 5 (4)

(1R,3r,5S,8s)-8-{[5-(4-Ethoxy-4-oxobutyl)-2-thienyl]-methyl}-3-{[hydroxy(diphenyl)acetyl]oxy}-8-methyl-8-azoniabicyclo[3.2.1]octane chloride (containing about 10% of the 8-position isomer)
TLC: Rf 0.59 (n-butanol:acetic acid:water = 4:2:1)
¹H-NMR (DMSO-d₆): d 1.17, 1.53-1.69, 1.73-1.93, 2.05-2.20, 2.35, 2.49-2.60, 2.82, 3.00, 3.67-3.78, 4.04, 4.54, 5.11, 6.77, 6.90, 7.20, 7.24-7.45.

### Example 5 (5)

(1R,3r,5S,8s)-8-{[5-(3-Ethoxy-3-oxopropyl)-2-thienyl]-methyl}-3-{[hydroxy(diphenyl)acetyl]oxy}-8-methyl-8-azoniabicyclo[3.2.1]octane chloride (containing about 10 % of the 8-position isomer)
TLC: Rf 0.64 (n-butanol:acetic acid:water = 4:2:1);
¹H-NMR (DMSO-d₆): d 1.16, 1.53-1.68, 1.72-1.86, 2.06-2.20, 2.49-2.60, 2.67, 2.99, 3.06, 3.66-3.77, 4.05, 4.54, 5.12, 6.78, 6.93, 7.20, 7.26-7.42.

### Example 5 (6)

(1R,3r,5S,8s)-8-[3-(4-Ethoxy-4-oxobutoxyl)benzyl}-3-{[hydroxy(diphenyl)acetyl]oxy}-8-methyl-8-azoniabicyclo-[3.2.1]octane bromide
TLC: Rf 0.70 (n-butanol:acetic acid:water = 4:2:1);
¹H-NMR (DMSO-d₆): d 1.17, 1.59-1.71, 1.71-1.85, 1.90-2.03, 2.19-2.31, 2.40-2.61, 2.91, 3.70-3.78, 3.96-4.11, 4.31, 5.12, 6.76, 6.97-7.11, 7.26-7.45.

### Example 6

### (1R,3r,5S)-8-Azabicyclo[3.2.1]oct-3-yl hydroxy(diphenyl)-acetate hydrochloride

1-Chloroethyl chloroformate (3.6 mL) was addded to a 1,2-dichloroethane solution (70 mL) of the compound (5.00 g) as produced in Example 4 under ice-cooling, followed by refluxing for 4 hours. The reaction mixture was left cooled and concentrated under reduced pressure. The resultant residue was admixed with ethyl acetate, and the insolubles were separated out through filtration. The filtrate was washed with 1N hydrochloric acid, water and an aqueous saturated sodium chloride solution, successively, dried over anhydrous sodium sulfate and then concentrated. The resultant residue was dissolved in methanol (70 mL), followed by refluxing for 1.5 hours. The reaction mixture was concentrated, and the resultant residue was subjected to azeotropy with toluene to give the subject title compound (5.08 g) showing the following physico-chemical values.
TLC: Rf 0.42 (n-butanol:acetic acid:water = 8:2:1);
¹H-NMR (CD₃OD): d 1.54, 1.74, 1.96, 2.29, 3.87, 5.20, 7.37.

### Example 7

### Ethyl 3-{4-[((1R,3r,5S)-3-{[hydroxy(diphenyl)acetyl]oxy}-8-azabicyclo[3.2.1]oct-8-yl)methyl]phenyl}propanoate

The compound (173 mg) as produced in Example 3 and potassium carbonate (155 mg) were addded to an N,N-dimethylformamide solution (5 mL) of the compound (200 mg) as produced in Example 6 under an argon atmosphere, followed by stirring at room teperature for one day. The reaction mixture was charged in water and extraction was effected with ethyl acetate. The organic layer was washed with water and an aqueous saturated sodium chloride solution, dried over anhydrous sodium sulfate and concentrated. The resultant residue was purified on a silica-gel column chromatography (n-hexane:ethyl acetate = 1:1 - ethyl acetate:methanol = 9:1) to give the subject title compound (237 mg) showing the following physico-chemical values:
TLC: Rf 0.53 (methylene chloride:methanol = 9:1);
¹H-NMR (CDCl₃): d 1.14-1.29, 1.60-1.74, 2.05-2.19, 2.60, 2.92, 2.96-3.04, 3.37, 4.12, 4.30, 5.22, 7.12, 7.22, 7.29-7.47.

### Example 8

### (1R,3r,5S,8r)-8-[4-(3-Ethoxy-3-oxopropyl)benzyl]-3-{[hydroxy(diphenyl)acetyl]oxy}-8-methyl-8-azoniabicyclo-[3.2.1]octane iodide

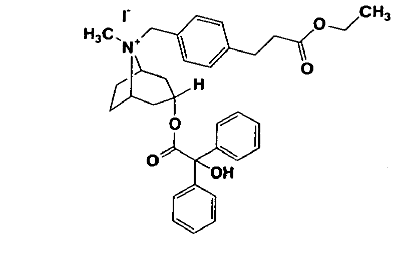

Methyl iodide (1 mL) was addded to an acetone solution (5 mL) of the compound (270 mg) as produced in Example 7, followed by stirring at room teperature for one day. The precipitated solid was recovered by filtration and washed with acetone. The resultant solid was recrystallized from acetonitrile to give the compound (91.5 mg) of the present invention showing the following physico-chemical values:
TLC: Rf 0.57 (n-butanol:acetic acid:water = 4:2:1);
¹H-NMR (CD₃OD): d 1.19, 1.62-1.74, 1.94-2.11, 2.64, 2.69, 2.91-3.09, 3.76-3.84, 4.08, 4.67, 5.38, 7.29-7.47.

### Example 9

### (1R,3r,5S)-8-Methyl-8-azabicyclo[3.2.1]oct-6-en-3-yl hydroxy(diphenyl)acetate

Tropenol (which can be produced by the procedure as described in Journal of the American Chemical Society, vol. 100(6), pp. 1786 (1978) and Tetrahedron, vol. 41 (24), pp. 5879 (1985) or Chemical Abstracts, vol. 40, pp. 6488 (1946))(1.12 g) and methyl benzylate (2.32 g) were dissolved in toluene (15 mL) under an argon atmosphere. The reaction mixture was admixed with sodiumhydride (60 %, in oil, 330 mg), followed by refluxing for 5 hours. The reaction mixture was left cooled and admixed with ice-water, and extraction was effected with methylene chloride. The organic layer was dried over anhydrous sodium sulfate and concentrated, and the resultant residue was washed with ethyl acetate to give the subject title compound (1.99 g) showing the following physico-chemical values:
TLC: Rf 0.39 (n-butanol:acetic acid:water = 4:2:1);
¹H-NMR (CDCl₃): d 1.55-1.68, 2.16-2.28, 3.25-3.31, 4.32, 5.13, 5.48, 7.29-7.45.

### Example 10

### (1R,2R,4S,5S,7s)-9-Methyl-3-oxa-9-azatricyclo[3.3.1.0^{2.4}]-non-7-yl hydroxy(diphenyl)acetate

Vanadium pentaoxide (20 mg), water (0.42 mL) and a hydrogen peroxide/urea complex (322 mg) were added successively to an N,N-dimethylformamide solution (8.5 mL) of the compound (500 mg) as produced in Example 9, followed by stirring at 40° C for 6 hours. The reaction mixture was left cooled to room temperature and adjusted to a pH value of ca. 4 with 1N hydrochloric acid. The reaction mixture was admixed with an aqueous solution (0.8 mL) of sodium hydrogensulfite (80 mg) to decompose excessive hydrogen peroxide. The reaction mixture was diluted with 2N hydrochloric acid and washed with methylene chloride. The aqueous layer was adjusted to a pH value of ca. 8 with 5N aqueous sodium hydroxide solution, and extraction was effected with methylene chloride. The organic layer was dried over anhydrous sodium sulfate and concentrated. The resultant residue was purified on a silica-gel column chromatography (methylene chloride:methanol = 20:1) to give the subject title compound (157 mg) showing the following physico-chemical values:
TLC: Rf 0.08 (n-butanol:acetic acid:water = 4:2:1);
¹H-NMR (CDCl₃): d 1.47-1.56, 2.07-2.19, 2.45, 2.78, 2.99-3.04, 4.23, 5.18, 7.31-7.42.

### Example 11

### (1R,2R,4S,5S,7s,9r)-9-[4-(3-Ethoxy-3-oxopropyl)benzyl]-7-{[hydroxy(diphenyl)acetyl]oxy}-9-methyl-3-oxa-9-azoniatricyclo[3.3.1.0^{2.4}]nonane bromide

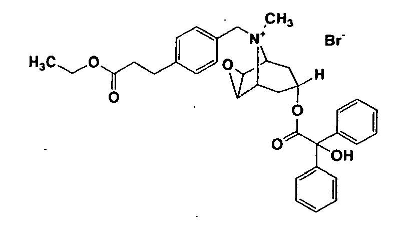

The compound (87 mg) as produced in Example 10 was dissolved in a solvent mixture of methylene chloride (2 mL) and acetone (1 mL), and the reaction mixture was admixed with the compound (83 mg) as produced in Example 3, followed by stirring at room temperature for 4 days. The reaction mixture was concentrated, and the resultant residue was washed with tert-butylmethylether to give the subject title compound (132 mg) showing the following physico-chemical values:
TLC: Rf 0.55 (n-butanol:acetic acid:water = 4:2:1);
¹H-NMR (CD₃OD): d 1.21, 1.93, 2.57-2.74, 2.96, 3.05, 3.27, 3.93, 4.09, 4.81, 5.23, 7.26-7.46.

### Example 11 (1)

The same procedure as described in Example 9 was conducted into practice, while using in place of methyl benzylate the compound obtained by the same procedure as described in Example 12 with use of the corresponding Grignard reagent in place of 2-thienylmagnesium bromide, and the same procedures as described in Example 10 to Example 11 were carried out to give the compounds of the present invention showing the following physico-chemical values:

### Example 11 (1)

### (1R,2R,4S,5S,7s,9r)-9-[4-(3-Ethoxy-3-oxopropyl)benzyl]-7-{[hydroxy(di-3-thienyl)acetyl]oxy}-9-methyl-3-oxa-9-azoniatricyclo[3.3.1.0^{2.4}]nonane bromide

The same procedure as described in Example 9 was conducted into practice, while using in place of methyl benzylate the compound obtained by the same procedure as described in Example 12 with use of 3-thienylmagnesium iodide in place of 2-thienylmagnesium bromide, and furthermore, the same procedures as described in Example 10 to Example 11 were carried out to give the compound of the present invention showing the following physico-chemical values:
TLC: Rf 0.37 (n-butanol:acetic acid:water = 8:4:1);
¹H-NMR (DMSO-D₆): d 1.15, 1.79-1.92, 2.53-2.69, 2.88, 2.99, 3.57, 3.92-3.99, 4.04, 4.75, 5.07, 6.68, 7.10, 7.31-7.43, 7.50.

### Example 12

### Methyl hydroxy[di(2-thienyl)]acetate

2-Thienylmagnesium bromide (ca. 0.3 M hexane-THF mixture solution, 380 mL) was added to an anhydrous THF solution (100 mL) of dimethyl oxalate (7.0 g) under an argon atmosphere and under ice-cooling, followed by stirring for 2 hours under ice-cooling. The reaction mixture was admixed with an aqueous saturated ammonium sulfate solution, followed by separation. The aqueous layer was extracted with ethyl acetate, and the extract was combined with the previously obtained organic layer, washed with an aqueous saturated sodium chloride solution, dried over anhydrous sodium sulfate and concentrated. The resultant residue was purified on a silica-gel column chromatography (ethyl acetate) to give the subejct title compound (8.07 g) showing the following physico-chemical values:
TLC: Rf 0.49 (n-hexane:ethyl acetate = 4:1);
¹H-NMR (CDCl₃): d 3.90, 4.68, 6.98, 7.16, 7.29.

### Example 13

### (1R,3r,5S)-8-Methyl-8-azabicyclo[3.2.1]oct-3-yl hydroxy[di(2-thienyl)]acetate

Tropine (500 mg) and the compound (1.17 g) as produced in Example 12 were dissolved in toluene (25 mL) under an argon atmosphere. The reaction mixture was admixed with sodium hydride (60%, in oil, 144 mg), followed by refluxing for 5 hours. The reaction mixture was left cooled and admixed with ice-water, and extraction was effected with methylene chloride. The organic layer was washed with water and an aqueous saturated sodium chloride solution, dried over anhydrous sodium sulfate and concentrated. The resultant residue was purified on a silica-gel column chromatography (methylene chloride:methanol = 9:1 ~ methylene chloride : methanol : water = 90:10:1 ~ methylene chloride:methanol:water = 50:10:1) to give the subject title compound (706 mg) showing the following physico-chemical values:
TLC: Rf 0.39 (n-butanol:acetic acid:water = 4:2:1);
¹H-NMR (CDCl₃): d 1.28-1.38, 1.66-1.84, 2.10-2.20, 2.21, 2.97-3.05, 4.83-4.94, 5.14, 7.00, 7.18, 7.31.

### Example 14

### (1R,3r,5S,8s)-8-[4-(3-Ethoxy-3-oxopropyl)benzyl]-3-({hydroxy[di(2-thienyl)]acetyl}oxy)-8-methyl-8-azoniabicyclo[3.2.1]octane bromide

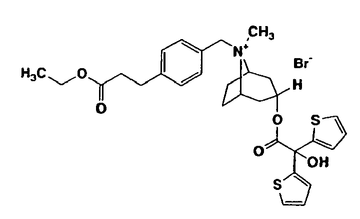

The compound (50 mg) as produced in Example 13 was dissolved in a solvent mixture of methylene chloride (0.5 mL) and acetone (0.5 mL). The reaction mixutre ws admixed with the compound (50 mg) as produced in Example 3, followed by stirring at room temperature for one day. The precipitated solid was recovered by filtration and washed with tert-butylmethylether. The resultant solid was washed with acetonitrile under heating to give the compound (60 mg) of the present inention showing the following physico-chemical values:
TLC: Rf 0.53 (n-butanol:acetic acid:water = 4:2:1);
¹H-NMR (CD₃OD): d 1.20, 1.87-2.07, 2.36-2.50, 2.56-2.69, 2.92-3.02, 3.77-3.89, 4.09, 4.40, 5.21, 7.03, 7.18, 7.37, 7.40-7.47.

### Examples 14 (1) to (4)

The same procedures as described in Example 12, Example 13, and Example 14 were carried out, while using the corresponding Grignard reagent in place of 2-thienymagnesium bromide and the corresponding halide in place of the compound as produced in Example 3 , to give the compounds of the present invention showing the following physico-chemical values:

### Example 14 (1)

### (1R,3r,5S,8s)-3-({Hydroxy[di(2-thienyl)]acetyl}oxy)-8-[4-(4-methoxy-4-oxobutyl)benzyl]-8-methyl-8-azoniabicyclo-[3.2.1]octane bromide

TLC: Rf 0.30 (n-butanol:acetic acid:water = 8:2:1)
¹H-NMR (CD₃OD): d 1.80-2.10, 2.35, 2.39-2.52, 2.55-2.79, 2.98, 3.64, 3.74-3.93, 4.40, 5.21, 7.03, 7.17, 7.29-7.53.

### Example 14 (2)

### (1R,3r,5S,8s)-8-[4-(3-Ethoxy-3-oxopropyl)benzyl]-3-({hydroxy[di(3-thienyl)acetyl}oxy)-8-methyl-8-azoniabicyclo[3.2.1]octane bromide

TLC: Rf 0.58 (n-butanol:acetic acid:water = 4:2:1);
¹H-NMR (DMSO-d₆): d 1.14, 1.68-1.88, 2.28-2.39, 2.49-2.58, 2.63, 2.82-2.94, 3.68-3.78, 4.04, 4.34, 5.06, 6.72, 7.10, 7.30-7.43, 7.53.

### Example 14 (3)

### (1R,3r,5S,8s)-8-[3-(2-Ethoxy-2-oxoethoxy)benzyl]-3-({hydroxy[di(3-thienyl)acetyl}oxy)-8-methyl-8-azoniabicyclo[3.2.1]octane bromide

TLC: Rf 0.55 (n-butanol:acetic acid:water = 4:2:1);
¹H-NMR (DMSO-d₆): d 1.21, 1.68-1.87, 2.28-2.38, 2.49-2.60, 2.91, 3.72-3.80, 4.16, 4.35, 4.83, 5.06, 6.73, 7.02-7.13, 7.36-7.44, 7.53.

### Example 14 (4)

### (1R,3r,5S,8s)-8-[3-(2-Ethoxy-2-oxoethoxy)benzyl]-3-({hydroxy[di(2-thienyl)]acetyl}oxy)-8-methyl-8-azoniabicyclo[3.2.1]octane bromide

TLC: Rf 0.68 (n-butanol:acetic acid:water = 4:2:1);
¹H-NMR (DMSO-d₆): d 1.21, 1.72-1.91, 2.30-2.44, 2.51-2.64, 2.91, 3.74-3.82, 4.16, 4.36, 4.83, 5.09, 6.99-7.15, 7.41, 7.45, 7.54.

### Example 14 (5)

### (1R,3r,5S,8s)-8-{[6-(2-Ethoxy-2-oxoethoxy)-2-naphthyl]-methyl}-3-({hydroxy[di(2-thienyl)acetyl}oxy)-8-methyl-8-azoniabicyclo[3.2.1]octane bromide

The same procedure as described in Example 14 was carried out, while using the compound as produced in Example 22 as the corresponding halide, to give the compound (95 mg) of the present invention showing the following physico-chemical values:
TLC: Rf 0.60 (n-butanol:acetic acid:water = 4:2:1);
¹H-NMR (CD₃OD): d 1.29, 1.86-2.12, 2.43-2.73, 3.03, 3.81-3.98, 4.26, 4.57, 4.85, 5.21, 7.05, 7.19, 7.23-7.34, 7.45, 7.52, 7.88, 7.91, 7.99.

### Example 15

### tert-Butyl (1R,3r,5S)-3-{[hydroxy(diphenyl)acetyl]oxy}-8-azabicyclo[3.2.1]octane-8-carboxylate

The compound (1.13 g) as produced in Example 6 was suspended in an aqueous 1N sodium hydroxide solution (3mL), and the reaction mixture was admixed with a dioxane solution (3 mL) of di-tert-butyl dicarbonate (659 mg), followed by stirring at room temperature for 2 hours. The precipitated solid was recovered by filtration and washed with a solvent mixture (1:1) of dioxane and water to give the subject title compound (1.24 g) showing the following physico-chemical values:
TLC: Rf 0.30 (n-hexane:ethyl acetate = 4:1);
¹H-NMR (CDCl₃): d 1.09-1.36, 1.56, 1.63-1.76, 2.03-2.27, 2.96, 4.04, 4.14, 5.13, 7.30-7.39, 7.40-7.51.

### Example 16

### tert-Butyl (1R,3r,5S)-3-{[(2-methoxy-2-oxoethoxy)-(diphenyl)acetyl]oxy}-8-azabicyclo[3.2.1]octane-8-carboxylate

Cesium carbonate (245 mg) and methyl bromoacetate (0.050 mL) were added to an N,N-dimethylformamide solution (1.5 mL) of the compound (220 mg) as produced in Example 15, followed by stirring at room temperature overnight. Cesium carbonate (2.39 g) and methyl bromoacetate (0.75 mL) were added to the reaction mixture, followed by stirring at room temperature for 4 days. The reaction mixture was admixed with 1N aqueous hydrochloric acid solution, and extraction was effected with ethyl acetate. The organic layer was washed with water, an aqueous saturated sodium hydrogencarbonate solution and an aqueous saturated sodium chloride solution, successively, dried over anhydrous sodium sulfate and concentrated. The same type of the reaction was carried out with use of the compound (220 mg) as produced in Example 15. The crude products were combined and purified on a silica-gel column chromatography (n-hexane:ethyl acetate = 4:1) to give the subject title compound (390 mg) showing the following physico-chemical values:
TLC: Rf 0.23 (n-hexane:ethyl acetate = 4:1)
¹H-NMR (CDCl₃): d 1.15-1.32, 1.42, 1.50-1.67, 1.91-2.25, 3.68, 3.90-4.15, 5.21, 7.29-7.49.

### Example 17

### (1R,3r,5S)-8-Azabicyclo[3.2.1]oct-3-yl (2-methoxy-2-oxoethoxy)(diphenyl)acetate hydrochloride

The compound (378 mg) as produced in Example 16 was dissolved in a 4N-hydrochloric acid/dioxane solution (4 mL), followed by stirring at room temperature for 30 min. The reaction mixture was concentrated, and the concentrate was subjected to azeotropic distillation with toluene. The resultant residue was washed with tert-butylmethylether to give the subject title compound (288 mg) showing the following physico-chemical values:
TLC: Rf 0.34 (n-butanol:acetatic acid:water = 8:2:1);
¹H-NMR (CD₃OD): d 1.42-1.57, 1.64-1.79, 1.82-1.97, 2.15-2.33, 3.65, 3.79-3.91, 4.05, 5.19, 7.31-7.52.

### Example 18

### (1R,3r,5S)-8-Methyl-8-azabicyclo[3.2.1]oct-3-yl (2-methoxy-2-oxoethoxy)(diphenyl)acetate

The compound (142 mg) as produced in Example 17 was admixed with an aqueous saturated sodium hydrogencarbonate solution, and extraction was effected with methylene chloride. The organic layer was dried over anhydrous sodium sulfate and concentrated. The resultant residue was dissolved in acetonitrile (3 mL), and the solution was admixed with methyl iodide (1 mL), followed by stirring at room temperature for 2 days. The reaction mixture was concentrated, and the residue was admixed with an aqueous saturated sodium hydrogencarbonate solution, followed by extraction with methylene chloride. The organic layer was dried over anhydrous sodium sulfate and concentrated. The residue was washed with ethyl acetate, and the filtrate was concentrated. The resultant residue was dissolved in tetrahydrofuran (1 mL), and the solution was admixed with di-tert-butyl dicarbonate (53 mg), followed by stirring at room temperature for 2 hours. The reaction mixture was concentrated, and the residue was dissolved in ethyl acetate, followed by reverse extraction with 1N hydrochloric acid. The aqueous layer was neutralized with sodium hydrogencarbonate, and extraction was effected with methylene chloride. The organic layer was dried over anhydrous sodium sulfate and concentrated to give the subject title compound (56mg) showing the following physico-chemical values:
TLC: Rf 0.40 (n-butanol:acetatic acid:water = 4:2:1);
¹H-NMR (CDCl₃): d : 1.09-1.38, 1.55, 1.62-1.80, 2.05-2.27, 2.95, 3.68, 4.06, 5.04-5.21, 7.30-7.54.

### Example 19

### (1R,3r,5S)-3-{[(2-Methoxy-2-oxoethoxy)(diphenyl)acetyl]-oxy}-8,8-dimethyl-8-azoniabicyclo[3.2.1]octane bromide

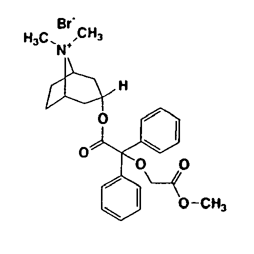

An acetone solution (1 mL) of the compound (56 mg) as produced in Example 18 was admixed with a tert-butyl methyl ether solution (2.0 M, 2 mL) of methyl bromide, followed by stirring at room temperature for 2 days. The precipitated solid was recovered by filtration, and washed with a solvent mixture of tert-butyl methyl ether and acetone to give the subject title compound (34 mg) of the present invention showing the following physico-chemical values:
TLC: Rf 0.19 (n-butanol:acetatic acid:water = 4:2:1);
¹H-NMR (CD₃OD): d 1.45-1.66, 1.84, 1.97-2.12, 2.51-2.80, 3.00, 3.13, 3.58-3.78, 4.06, 5.21, 7.24-7.58.

### Example 19(1)

The same procedures as described in Example 16, Example 17, Example 18, and Example 19 were carried out, while using the corresponding halide in place of methyl bromoacetate, to give the compounds of the present invention showing the following physico-chemical values:

### Example 19 (1)

### (1R,3r,5S)-3-{[(2-Ethoxy-2-oxoethoxy)(diphenyl)acetyl]oxy}-8,8-dimethyl-8-azoniabicyclo[3.2.1]octane bromide

TLC: Rf 0.23 (n-butanol:acetatic acid:water = 4:2:1);
¹H-NMR (CD₃OD): d 1.21, 1.44-1.66, 1.85, 1.96-2.14, 2.52-2.74, 3.00, 3.13, 3.69, 4.04, 4.11, 5.21, 7.24-7.51.

### Example 20

### Ethyl [(6-formyl-2-naphthyl)oxy]acetate

Ethyl iodoacetate (0.75 mL) and potassium carbonate (1.2 g) were added to an N,N-dimethylformamide solution (5 mL) of 6-hydroxy-2-naphthoaldehyde (1.0 g), followed by stirring at room temperature for 2 hours. The reaction mixture was charged into water, and extraction was effected with ethyl acetate. The organic layer was washed with water and an aqueous saturated sodium chloride solution, dried over anhydrous sodium sulfate and concentrated to give the subject title compound (1.52 g) showing the following physico-chemical property:
TLC: Rf 0.54 (n-hexane:ethyl acetate = 1:1);
¹H-NMR (CDCl₃): d 1.32. 4.31, 4.78, 7.12, 7.33, 7.80, 7.90-7.97, 8.27, 10.11.

### Example 21

### Ethyl {[6-(hydroxymethyl)-2-naphthyl]oxy}acetate

Sodium borohydride (110 mg) was added to an ethanol solution (15 mL) of the compound (1.52 g) as produced in Example 20, under ice-cooling, followed by stirring at 0° C for 15 min. The reaction mixture was admixed with an aqueous saturated sodium hydrogencarbonate solution, followed by extraction with ethyl acetate. The organic layer was washed with water and an aqueous saturated sodium chloride solution, dried over anhydrous sodium sulfate and concentrated. The resultant compound was purified on a silica gel column chromatography (n-hexane:ethyl acetate = 2:1) to give the subject title compound (1.34 g) showing the following physico-chemical values:
TLC: Rf 0.39 (n-hexane:ethyl acetate = 1:1);
¹H-NMR (CDCl₃): d 1.31, 4.30, 4.74, 4.83, 7.08, 7.25, 7.46, 7.69-7.79.

### Example 22

### Ethyl {[6-(bromomethyl)-2-naphthyl]oxy}acetate

Carbon tetrabromide (100 mg) and triphenylphosphine (60 mg) were added to a methylene chloride solution (1 mL) of the compound (52 mg) as produced in Example 21, followed by stirring at room temperature for 15 min. The reaction mixture was purified on a silica gel chromatography (n-hexane: ethyl acetate = 2:1) to give the subject title compound (108 mg) showing the following physico-chemical values:
TLC: Rf 0.66 (n-hexane:ethyl acetate = 2:1);
¹H-NMR (CD₃OD): d 1.31, 4.30, 4.66, 4.74, 7.07, 7.25, 7.48, 7.71, 7.75, 7.77.

### Example 23

### (1R,3r,5S,8r)-8-{[6-(2-Ethoxy-2-oxoethoxy)-2-naphthyl]-methyl}-3-{[hydroxy(diphenyl)acetyl]oxy}-8-methyl-8-azoniabicyclo[3.2.1]octane bromide

The compound (65 mg) as produced in Example 22 was added to an acetone (1 mL)-methylene chloride (1 mL) mixed solution of the compound (50 mg) as produced in Example 4 , followed by stirring at room temperature for 2 days. The resultant solid was recovered by filtration and washed with tert-butylmethylether to give the compound (81 mg) of the present invention showing the following physico-chemical values:
TLC: Rf 0.81 (n-butanol:acetic acid:water = 4:2:1);
¹H-NMR (DMSO-d₆): d 1.22, 1.62-1.85, 1.84, 2.28-2.39, 2.49-2.59, 2.94, 3.75-3.84, 4.18, 4.49, 4.92, 5.12, 6.78, 7.25-7.41, 7.51, 7.87, 7.94, 8.00.

### Example 24

### (1R,3r,5S,8r)-8-{[5-(4-Ethoxy-4-oxobutyl)-2-thienyl]-methyl}-3-{[hydroxy(diphenyl)acetyl]oxy}-8-methyl-8-azoniabicyclo[3.2.1]oct-6-en chloride

Ethyl 4-[5-(chloromethyl)-2-thienyl]butanoate (89mg) was added to a methylene chloride (1.5 mL)-acetone (1.5 mL) mixed solution of the compound (70 mg) as produced in Example 9, followed by stirring at 35° C for 2 days. The reaction mixture was concentrated, and the resultant residue was washed with tert-butyl methyl ether and purified on a silica gel column chromatography (methylene chloride:methanol = 10:1 to methylene chloride:methanol:water = 40:10:1) to give the compound (80 mg) of the present invention showing the following physico-chemical values:
TLC: Rf 0.52 (n-butanol:acetic acid:water = 8:2:1);
¹H-NMR (DMSO-d₆): d 1.17, 1.70-1.91, 2.35, 2.49-2.59, 2.81, 3.10, 4.04, 4.11-4.18, 4.70, 5.09, 5.76, 6.56, 6.91, 7.13, 7.27-7.43.

### Example 25

### (1R,3r,5S,8r)-8-[4-(3-Ethoxy-3-oxopropyl)benzyl]-3-({hydroxy[di(2-thienyl)]acetyl}oxy)-8-methyl-8-azoniabicyclo[3.2.1]oct-6-en bromide

The compound (76 mg) as produced in Example 3 was added to methylene chloride (2 mL) and ethyl acetate (2 mL) solutions of the compound (100 mg) which was produced by the same procedure as described in Example 13 from the compound as produced in Example 12 and tropenol (described in Example 9), followed by stirring at room temperature for one day. The precipitated solid was recovered by filtration and washed with ethyl acetate to give the compound (120 mg) of the present invention showing the following physic-chemical property:
TLC: Rf 0.58 (n-butanol:acetic acid:water = 4:2:1);
¹H-NMR (CD₃OD): d 1.20, 1.90-2.05, 2.51-2.71, 2.97, 3.14, 4.09, 4.17-4.23, 4.61, 5.18, 5.99-6.05, 7.05, 7.16, 7.27-7.41, 7.43.

### Example 26

### (1R,2R,4S,5S,7s,9r)-9-{[6-(2-Ethoxy-2-oxoethoxy)-2-naphthyl]methyl}-7-({hydroxy[di(2-thienyl)]acetyl}oxy)-9-methyl-3-oxa-9-azoniatricyclo[3.3.1.0^{2,4}]nonane bromide

An acetonitrile solution (3 mL) of the compound (90 mg), which was produced by carrying out the same procedure as described in Example 10 with the compound as produced by the procedure described in Example 13 using the compound as produced in Example 12 and tropenol, and an acetonitrile solution of the compound (77 mg) as produced in Example 22 were left on standing at room temperature overnight. The reaction mixture was admixed with isopropyl ether, and the precipitated solid was recovered by filtration. The resultant residue was washed with isopropyl ether to give the compound (115 mg) of the present invention showing the following physico-chemical values:
TLC: Rf 0.60 (n-butanol:acetic acid:water = 4:2:1);
¹H-NMR (CD₃OD): d 1.29, 1.92-2.12, 2.61-2.81, 3.15, 3.51-3.58, 3.99-4.10, 4.26, 4.85, 5.03, 5.22, 7.03, 7.17, 7.23-7.34, 7.44, 7.50-7.59, 7.84-7.95, 7.99.

### Example 27

### (1R,3r,5S,8r)-8-{[6-(2-Ethoxy-2-oxoethoxy)-2-naphthyl]-methyl}-3-({hydroxy[di(3-thienyl)]acetyl}oxy)-8-methyl-8-azoniabicyclo[3.2.1]oct-6-en bromide

The compound (33 mg) as produced in Example 22 was added to a methylene chloride (0.5 mL)-acetone (0.5 mL) mixed solution of (1R,3r,5S)-8-methyl-8-azabicyclo[3.2.1]oct-6-en-3-yl hydroxy(di-3-thienyl)acetate (30 mg), which was produced by the same procedure as described in Example 13 while using methyl hydroxy[di(3-thienyl)]acetate as produced by the same procedure as described in Example 12 from dimethyl oxalate and 3-thienylmagnesium bromide and also the compound as produced in Example 9, followed by stirring at room temperature for one day. The reaction solution was admixed with tert-butyl methyl ether, and the precipitated solid was recovered by filtration. The resultant residue was washed with methyl tert-butyl ether to give the compound (54 mg) of the present invention showing the following physico-chemical values:
TLC: Rf 0.44 (n-butanol:acetic acid:water = 8:4:1);
¹H-NMR (DMSO-d₆): d 1.22, 1.70-1.83, 2.46-2.58, 3.12, 4.12-4.24, 4.69, 4.93, 5.06, 6.05, 6.57, 7.08, 7.30, 7.34-7.39, 7.41, 7.52, 7.87, 7.90-7.97.

### Example 28

### (1R,3s,5S)-8-Methyl-8-azabicyclo[3.2.1]oct-6-en-3-yl oxo(phenyl)acetate

A catalytic amount of N,N-dimethylformamide was added to an ethyl acetate solution (30 mL) of oxo (phenyl) acetic acid (4.5 g) and thionyl chloride (3.3 mL), followed by stirring at 95° C for 2 hours. After concentration under reduced pressure, the remaining thionyl chloride and hydrogen chloride were removed through azeotropic distillation with use of ethyl acetate. The methylene chloride solution (10 mL) of resultant residue was added to a solution of tropenol (3.5 g) and triethylamine (4.2 mL) in methylene chloride (140 mL), followed by stirring at room temperature overnight. After concentration under reduced pressure, the resultant residue was admixed with an aqueous saturated sodium hydrogencarbonate solution, and extraction was effected with ethyl acetate, followed by washing with water. The organic layer was dried over anhydrous magnesium sulfate, and concentrated. The resultant residue was purified on a silica gel column chromatography (methylene chloride:methanol = 6:1) to give the subject title compound (2.6 g) showing the following physico-chemical values:
TLC: Rf 0.30 (methylene chloride:methanol:acetic acid = 8:1.5:0.5);
¹H-NMR (CDCl₃): d 1.79-2.01, 2.39, 2.43-2.58, 3.48-3.65, 5.31, 5.95-6.11, 7.46-7.58, 7.62-7.58, 7.62-7.79, 7.89-8.01.

### Example 29

### (1R,3s,5S)-8-Methyl-8-azabicyclo[3.2.1]oct-6-en-3-yl cyclopentyl(hydroxy)phenyl acetate

Cyclopentylmagnesium bromide (1.0M, a tetrahydrofuran solution (2.2. mL)) was added to a tetrahydrofuran solution (5 mL) of the compound (542 mg) as produced in Example 28, followed by stirring at room temperature for 2 hours, and the reaction mixture was admixed with 2N hydrochloric acid to be made acidic. An aqueous saturated sodium hydrogencarbonate solution was added to the same to be made alkaline again, followed by extraction with ethyl acetate, and the organic layer was dried over anhydrous sodium sulfate and concentrated. The resultant residue was purified on a silica gel column chromatography (methylene chloride:methanol = 8:1) to give the subject title compound (220 mg) showing the following physico-chemical values:
TLC: Rf 0.31 (methylene chloride:methanol:acetic acid = 8:1.5:0.5);
¹H-NMR (CDCl₃): d 1.24-1.88, 2.06-2.40, 2.78-2.95, 3.26-3.49, 3.79, 5.00, 5.87, 6.01, 7.13-7.44, 7.54-7.71.

### Example 30

### (1R,3s,5S,8r)-3-{[Cyclopentyl(hydroxy)phenylacetyl]oxy}-8-[3-(2-ethoxy-2-oxoethoxy)benzyl]-8-methyl-8-azoniabicyclo-[3.2.1]oct-6-en bromide

tert-Butylmethylether (2.5 mL) was added to an acetonitrile solution (2.5 mL) of the compound (80 mg) as produced in Example 29 and ethyl {[3-(bromomethyl)phenyl]oxy}acetate (63 mg), and the reaction mixture was left on standing at room temperature overnight. The reaction mixture was concentrated under reduced pressure, and the resultant residue was washed with ethyl acetate to give the compound (54 mg) of the present invention showing the following physico-chemical values:
TLC: Rf 0.55 (n-butanol:acetic acid:water = 4:2:1);
¹H-NMR (CD₃OD): d 1.25-1.32, 1.33-1.82, 1.88-2.11, 2.37-2.79, 2.88-3.10, 3.15, 4.01-4.35, 4.01-4.35, 4.62, 4.78, 5.05, 6.07, 6.34, 6.88-7.16, 7.20-7.50, 7.58-7.68.

### Example 31

### (1R,2R,4S,5S,7s)-9-Methyl-3-oxa-9-azatricyclo[3.3.1.0^{2,4}]-non-7-yl cyclopentyl(hydroxy)phenyl acetate

Water (1 mL) and vanadium pentaoxide (24 mg) were added to an N,N-dimethylformamide solution (20 mL) of the compound (440 mg) as produced in Example 29, and a hydrogen peroxide-urea complex (292 mg) was added to the mixture under stirring, followed by stirring at 40° C for 8 hours. The reaction solution was admixed with an aqueous saturated sodium hydrogencarbonate solution, followed by extraction with methylene chloride and washing with water. The organic layer was dried over anhydrous sodium sulfate and concentrated to give the subject title compound (380 mg) showing the following physico-chemical values:
TLC: Rf 0.67 (methylene chloride:methanol = 4:1);
¹H-NMR (CDCl₃): d 1.26-1.84, 1.95-2.25, 2.49, 2.85-2.98, 3.00-3.07, 3.16, 3.52, 3.83, 4.99, 7.16-7.44, 7.52-7.64.

### Example 32

### (1R,2R,4S,5S,7s,9r)-7-{[Cyclopentyl(hydroxy)phenylacetyl]-oxy}-9-{[6-(2-ethoxy-2-oxoethoxy)-2-naphthyl]methyl}-9-methyl-3-oxa-9-azoniatricyclo[3.2.1.0^{2,4}]nonane bromide

The same procedure as described in Example 30 was conducted into practice, while using the compound (50 mg) as produced in Example 29 and the compound (68 mg) as produced in Example 22, to give the compound (30 mg) of the present invention showing the following physico-chemical values:
TLC: Rf 0.62 (n-butanol:acetic acid:water = 4:2:1);
¹H-NMR (CD₃OD): d 1.29, 1.36-2.04, 2.45-2.76, 3.02-3.13, 3.22-3.27, 3.77-3.81,3.92-4.06, 4.26, 4.84, 5.00, 5.04, 7.23-7.44, 7.51, 7.57-7.64, 7.87-7.90, 7.97.

### Example 33

### (1R,3r,5S,8s)-3-{[Cyclopentyl(hydroxy)-2-thienylacetyl]-oxy}-8-{[6-(2-ethoxy-2-oxoethoxy)-2-naphthyl]methyl}-8-methyl-8-azoniabicyclo[3.2.1]octane bromide

The same procedures as described in Example 28 and Example 29 were conducted into practice, while using oxo(2-thienyl)acetic acid (1.6 g) in place of oxo (phenyl) acetic acid and tropine (1.8g) in place of tropenol, and the procedure as described in Example 32 was furthermore carried out to give the compound (120 mg) of the present invention showing the following physico-chemical values:
TLC: Rf 0.64 (n-butanol:acetic acid:water = 4:2:1);
¹H-NMR (CD₃OD): d 1.29, 1.51-1.76, 1.79-2.10, 2.29-2.84, 2.93-3.07, 3.85-4.03, 4.26, 4.60,4.85, 5.10, 7.03, 7.20, 7.24-7.34, 7.37, 7.53, 7.85-7.94, 8.00.

### Example 34

### (1R,3r,5S,8r)-3-{[Cyclopentyl(hydroxy)-2-thienylacetyl]-oxy}-8-[3-(2-ethoxy-2-oxoethoxy)benzyl]-8-methyl-8-azoniabicyclo[3.2.1]oct-6-en bromide

The same procedures as described in Example 28 and Example 29 were conducted into practice, while using oxo(2-thienyl)acetic acid (1.6 g) in place of oxo(phenyl)acetic acid, and the procedure as described in Example 30 was carried out with the reaction mixture to give the compound (41 mg) of the present invention showing the following physico-chemical values:
TLC: Rf 0.60 (n-butanol:acetic acid:water = 4:2:1);
¹H-NMR (CD₃OD): d 1.28, 1.40-1.77, 1.78-2.14, 2.44-2.76, 2.81-2.99, 3.17, 4.13-4.37, 4.65, 4.78, 5.10, 6.44, 6.85-7.23, 7.28-7.53.

### Example 35

### (1R,2R,4S,5S,7s)-7-({Hydroxy[4-(2-methoxy-2-oxoethoxy)-phenyl]phenylacetyl}oxy)-9,9-dimethyl-3-oxa-9-azoniatricyclo[3.3.1.0^{2,4}]nonane bromide

The same procedures as described in Example 29 was conducted into practice, while using [4-(tetrahydro-2H-pyran-2-yloxy)phenyl]magnesium bromide in place of cyclopentylmagnesium bromide. Furthermore, the resultant compound was subjected to a deprotecting reaction with acetic acid by carrying out the procedure as described in Example 10, and the same procedures as described in Example 16 and Example 19 were conducted into practice to give the compound of the present invention showing the following physico-chemical values:
TLC: Rf 0.18 (n-butanol:acetic acid:water = 4:2:1);
¹H-NMR (CD₃OD): d 1.87-2.01, 2.63-2.86, 3.07, 3.20-3.23, 3.30, 3.77, 3.91-4.04, 4.72, 5.22, 6.92, 7.19-7.49.

### Example 36

### (1R,2R,4S,5S,7s)-7-{[[4-(Ethoxycarbonyl)phenyl](hydroxy)-phenylacetyl]oxy}-9,9-dimethyl-3-oxa-9-azoniatricyclo-[3.2.1.0^{2,4}]nonane bromide

The same procedure as described in Example 29 was conducted into practice, while using the compound as produced in Example 28 and ethyl 4-iodobenzoate and isopropylmagnesium bromide in place of cyclopentylmagnesium bromide. Furthermore, the same procedures as described in Example 10 and Example 19 were carried out with use of the resultant compound to give the compound of the present invention showing the following physico-chemical values:
TLC: Rf 0.25 (n-butanol:acetic acid:water = 4:2:1);
¹H-NMR (CD₃OD): d 1.37, 1.84-2.06, 2.67-2.87, 3.08, 3.22-3.27, 3.28-3.34, 3.90-4.01, 4.36, 5.25, 7.23-7.43, 7.47-7.63, 7.94-8.09.

### Example 37

### (1R,2R,4S,5S,7s)-7-{[[5-(Ethoxycarbonyl)-2-thienyl]-(hydroxy)phenylacetyl]oxy}-9,9-dimethyl-3-oxa-9-azoniatricyclo[3.2.1.0^{2,4}]nonane bromide

The same procedure as described in Example 10 was conducted into practice, using the compound as produced in Example 28, and the resultant compound was subjected to the same procedure as described in Example 29, while using ethyl 5-bromo-2-thiophenecarboxylate and isopropylmagnesium bromide in place of cyclopentylmagnesium bromide. The same procedures as described in Example 19 were carried out with use of the resultant compound to give the compound of the present invention showing the following physico-chemical values:
TLC: Rf 0.30 (n-butanol:acetic acid:water = 4:2:1);
¹H-NMR (CD₃OD): d 1.34, 1.84-2.13, 2.69-2.87, 3.09, 3.21-3.25, 3.32, 3.38-3.43, 3.95-4.06, 4.32, 5.23, 7.17, 7.33-7.52, 7.69.

### Examples 37(1) to (2)

The same procedure as described in Example 28 was conducted into practice, while using oxo(thiophenyl)acetic acid in place of oxo(phenyl)acetic acid. The same procedures as described in Example 10, Example 29 and Example 19 were carried out with the resultant compound to give the below-described compounds.

### Example 37 (1)

### (1R,2R,4S,5S,7s)-7-[(Hydroxy{5-[(2-methoxy-2-oxoethoxy)-carbonyl]-2-thienyl}-2-thienylacetyl)oxy]-9,9-dimethyl-3-oxa-9-azoniatricyclo[3.3.1.0^{2,4}]nonane bromide

In the same procedure as described in Example 29, 2-(methyloxy)-2-oxoethyl 5-bromo-2-thiophenecarboxylate and isopropylmagnesium bromide were used in place of cyclopentylmagnesium bromide to give the compound of the present invention showing the following physico-chemical values:
TLC: Rf 0.16 (n-butanol:acetic acid:water = 4:2:1);
¹H-NMR (CD₃OD): d 1.96-2.16, 2.71-2.88, 3.11, 3.35, 3.46-3.49, 3.52-3.57, 3.77, 4.03-4.08, 4.84, 5.24, 7.03, 7.17, 7.28, 7.46, 7.78.

### Example 37 (2)

### (1R,2R,4S,5S,7s)-7-({Hydroxy[5-(methoxycarbonyl)-2-thienyl] -2-thienylacetyl}oxy)-9,9-dimethyl-3-oxa-9-azoniatricyclo-[3.3.1.0^{2,4}]- nonane bromide

In the same procedure as described in Example 29, methyl 5-bromo-2-thiophenecarboxylate and isopropylmagnesium bromide were used in place of cyclopentylmagnesium bromide to give the compound of the present invention showing the following physico-chemical values:
TLC: Rf 0.22 (n-butanol:acetic acid:water = 4:2:1);
¹H-NMR (CD₃OD): d 1.94-2.11, 2.69-2.89, 3.11, 3.35, 3.42-3.53, 3.86, 4.00-4.12, 5.23, 7.03, 7.16, 7.24, 7.45, 7.70.

### Example 37 (3)

### (1R,2R,4S,5S,7s)-7-({Hydroxy[5-(methoxycarbonyl)-2-furyl]-2-thienylacetyl}oxy)-9,9-dimethyl-3-oxa-9-azoniatricyclo-[3.3.1.0^{2,4}]nonane bromide

In the same procedure as described in Example 29, methyl 5-bromo-2-furancarboxylate and isopropylmagnesium bromide were used in place of cyclopentylmagnesium bromide to give the compound of the present invention showing the following physico-chemical values:
TLC: Rf 0.31 (n-butanol:acetic acid:water = 4:2:1);
¹H-NMR (CD₃OD): d 1.96-2.18, 2.70-2.87, 3.11, 3.36, 3.57-3.60, 3.79-3.82, 3.88, 4.02-4.12, 5.23, 6.59, 7.04, 7.18, 7.24, 7.47.

### Example 38

### Methyl hydroxy(3-{[(methoxy)methyl]oxy}phenyl)phenylacetate

Sodium hydride (440 mg) was added to an N,N-dimethylformamide (20 mL) solution of 3-iodophenol (2.2. g) under ice-cooling, followed by stirring at room temperature for 30 min., and bromomethylmethylether (0.84 mL) was added to the reaction mixture under ice-cooling, followed by stirring at room temperature for an hour. The reaction solution was admixed with water, followed by extraction with ethyl acetate, and the organic layer was admixed with hexane, washed with water, dried over anhydrous sodium sulfate and concentrated to give 1-iodo-3-{[(methoxy)methyl]oxy}benzene (2.7 g).

A tetrahydrofuran solution (50 mL) of 1-iodo-3-{[(methoxy)methyl]oxy}benzene (2.7 g) as obtained, at the internal temperature of -20 to -30°C, was admixed with 15 mL of a tetrahydrofuran solution (0.65 mol/L) of isopropylmagnesium bromide, followed by stirring at the same temperature for 30 min., and then a tetrahydrofuran (10 mL) solution of methyl oxo(phenyl)acetate (1.6 g) was added to the reaction solution, followed by stirring at room temperature overnight. The reaction solution was admixed with an aqueous saturated ammonium chloride solution, followed by extraction with ethyl acetate, and the organic layer was dried over anhydrous magnesium sulfate and concentrated. The resultant residue was purified on a silica gel column chromatography (n-hexane:acetic acid = 4:1) to give the compound (1.4 g) showing the following physico-chemical values:
TLC: Rf 0.30 (n-hexane:acetic acid = 2:1);
¹H-NMR (CD₃OD): d 3.46, 3.86, 4.19, 5.15, 7.11-7.15, 7.30-7.45.

### Example 39

### (1R,2R,4S,5S,7s)-7-({Hydroxy[3-(2-methoxy-2-oxoethoxy)-phenyl]phenylacetyl}oxy)-9,9-dimethyl-3-oxa-9-azoniatricyclo[3.3.1.0^{2,4}]nonane bromide

The same procedure as described in Example 13 was conducted into practice with use of the compound as produced in Example 38 and tropenol, and the same procedures as described in Example 10 → Example 17 → Example 16 → Example 19 were carried out with use of the resultant compound to give the compound showing the below-described physico-chemical values:
TLC: Rf 0.18 (n-butanol:acetic acid:water = 4:2:1);
¹H-NMR (CD₃OD): d 1.88-2.05, 2.62-2.84, 3.08, 3.14-3.17, 3.21-3.24, 3.28-3.32, 3.75, 3.92-4.02, 4.70, 5.24, 6.79-7.07, 7.19-7.48.

### Examples 39 (1) to (3)

The same procedure as described in Example 13 was conducted into practice, while using the corresponding compound in place of methyl oxy(phenyl)acetate, and the same procedures as described in Example 10, Example 17, Example 16 and Example 19 were furthermore carried out with the resultant compound to give the below-described compounds.

### Example 39 (1)

### (1R,2R,4S,5S,7s)-7-({Hydroxy[3-(2-methoxy-2-oxoethoxy)-phenyl]-2-thienylacetyl}oxy)-9,9-dimethyl-3-oxa-9-azoniatricyclo[3.3.1.0^{2,4}]nonane bromide

TLC: Rf 0.22 (n-butanol:acetic acid:water = 4:2:1);
¹H-NMR (CD₃OD): d 1.83-2.12, 2.57-2.87, 3.02-3.06, 3.09, 3.31, 3.39-3.45, 3.76, 3.94-4.05, 4.72, 5.21, 6.88-6.94, 6.99-7.09, 7.13, 7.23-7.33, 7.45.

### Example 39 (2)

### (1R,2R,4S,5S,7s)-7-({Hydroxy[3-(2-methoxy-2-oxoethoxy)-phenyl]-1,3-thiazol-2-ylacetyl}oxy)-9,9-dimethyl-3-oxa-9-azoniatricyclo[3.3.1.0^{2,4}]nonane bromide

TLC: Rf 0.18 (n-butanol:acetic acid:water = 4:2:1);
¹H-NMR (CD₃OD): d 1.83-1.99, 2.08-2.22, 2.65-2.83, 3.08, 3.24-3.28, 3.33, 3.74-3.84, 3.95-4.08, 4.72, 5.20, 6.92, 7.06-7.14, 7.29, 7.66, 7.83.

### Example 39 (3)

### (1R,2R,4S,5S,7s)-7-({Hydroxy[4-(2-methoxy-2-oxoethoxy)-phenyl]-2-thienylacetyl}oxy)-9,9-dimethyl-3-oxa-9-azoniatricyclo[3.3.1.0^{2,4}]nonane bromide

TLC: Rf 0.21 (n-butanol:acetic acid:water = 4:2:1);
¹H-NMR (CD₃OD): d 1.83-2.10, 2.66-2.86, 3.08, 3.14-3.18, 3.32, 3.41-3.46, 3.77, 3.93-4.04, 4.73, 5.21, 6.88-6.97, 7.00-7.11,7.33-7.48.

### Example 40

### Ethyl 3-{4-[(methoxymethoxy)methyl]phenyl}propanoate

A tetrahydrofuran solution (16 mL) of the compound (1.64 g) as produced in Example 2 was admixed with triethylamine (1.6 mL) and chloromethylmethylether (0.71 mL), followed by stirring at room temperature for one day. The reaction mixture was diluted with ethyl acetate, and the organic layer was washed with water and an aqueous saturated sodium chloride solution, dried over anhydrous sodium sulfate and concentrated. The resultant residue was purified on a silica gel column chromatography (n-hexane:ethyl acetate = 4:1) to give the subject title compound (1.47 g) showing the below-described physico-chemical values.
TLC: Rf 0.43 (n-hexane:ethyl acetate = 3:1);
¹H-NMR (CDCl₃): d 1.24, 2.57-2.65, 2.95, 3.41, 4.13, 4.56, 4.70, 7.19, 7.28.

### Example 41

### 3-{4-[(Methoxymethoxy)methyl]phenyl}propanoic acid

An aqueous sodium hydroxide solution (1 mol/L, 6 mL) was added to a 1,2-dimethoxyethane solution (6 mL) of the compound (1.47 g) as produced in Example 40, followed by stirring at room temperature for 7 hours. The reaction mixture was admixed with hydrochloric acid (1 mol/L, 6 mL) to make neutralization, followed by extraction with ethyl acetate, and the organic layer was washed with an aqueous saturated sodium chloride solution, dried over anhydrous sodium sulfate and concentrated to give the subject title compound (1.33 g) showing the below-described physico-chemical values.
TLC: Rf 0.61 (acetic acid:ethyl acetate = 1:100);
¹H-NMR (CDCl₃): d 2.63-2.73, 2.96, 3.41, 4.56, 4.70, 7.20, 7.29.

### Example 42

### 2-(Dimethylamino)-2-oxoethyl 3-{4-[(methoxymethoxy)methyl]-phenyl}propanoate

An N, N-dimethylformamide solution (10 mL) of the compound (250 mg) as produced in Example 41 was admixed with cesium carbonate (544 mg) and 2-chloro N,N-dimethylamide (160 mg), followed by stirring at room temperature for 16 hours. The reaction mixture was admixed with water, followed by extraction with ethyl acetate, and the organic layer was washed with water and an aqueous saturated sodium chloride solution, dried over anhydrous sodium sulfate and concentrated to give the subject title compound (335 mg) showing the below-described physico-chemical values.
TLC: Rf 0.46 (acetic acid:ethyl acetate = 1:100);
¹H-NMR (CDCl₃): d 2.73-2.81, 2.94-3.05, 3.41, 4.56, 4.70, 4.72, 7.21, 7.28.

### Example 43

### 2-(Dimethylamino)-2-oxoethyl 3-[4-(hydroxymethyl)phenyl]-propanoate

A 1,4-dioxane solution (5 mL) of the compound (320 mg) as produced in Example 42 was admixed with a hydrogen chloride/1,4-dioxane solution (4 mol/L, 1 mL), followed by stirring at room temperature for 18 hours. The reaction mixture was concentrated under reduced pressure, and after the residue was diluted with ethyl acetate, the organic layer separated out was washed with water and an aqueous saturated sodium chloride solution, dried over anhydrous sodium sulfate and concentrated. The resultant residue was purified on a silica gel chromatography (ethyl acetate) to give the subject title compound (92 mg) showing the below-described physico-chemical values.
TLC: Rf 0.20 (ethyl acetate);
¹H-NMR (CDCl₃): d 2.72-2.82, 2.96, 2.97, 2.97-3.05, 4.65, 4.71, 7.21, 7.28.

### Example 44

### 2-(Dimethylamino)-2-oxoethyl 3-[4-(bromomethyl)phenyl]-propanoate

A methylene chloride solution (1 mL) of the compound (39 mg) as produced in Example 43 was admixed with carbon tetrabromide (73 mg) and triphenylphopsphine (46 mg), followed by stirring at room temperature for 30 min. The reaction mixture was concentrated under reduced pressure, and the resultant residue was purified on a silica gel chromatography (methylene chloride:ethyl acetate = 9:1) to give the subject title compound (46 mg) showing the below-described physico-chemical values.
TLC: Rf 0.42 (ethyl acetate);
¹H-NMR (CDCl₃): d 2.72-2.81, 2.95, 2.96-3.04, 4.47, 4.71, 7.18, 7.30.

### Example 45

### (1R,2R,4S,5S,7s,9r)-9-(4-{3-[2-(Dimethylamino)-2-oxoethyl]-3-oxopropyl}benzyl)-7-{[hydroxy(di-2-thienyl)acetyl]oxy}-9-methyl-3-oxa-9-azoniatricyclo[3.3.1.0^{2,4}]nonane bromide

The same procedure as described in Example 11 was conducted into practice, while using the compound as produced by the same procedures as described in Example 9 → Example 10 from the compound as produced in Example 12 and tropenol and also the compound as produced in Example 44, to give the compound of the present invention showing the below-described physico-chemical values.
TLC: Rf 0.29 (n-butanol:acetic acid:water = 4:2:1);
¹H-NMR (DMSO-D₆): d 1.87, 2.56-2.76, 2.80, 2.88-2.96, 3.00, 3.58, 3.96-4.02, 4.71-4.80, 5.11, 7.01, 7.12, 7.34-7.45, 7.52.

### Pharmacologic Experiment Examples

The below-described experiments demonstrated that the compounds of the present invention can produce the intended effect at the targeted site and thereafter undergo rapid inactivation.

### Experiment Example 1: Evaluation of the Effect on the Airways Constriction in Guinea Pigs

The present experiment was carried out with Hartley male guinea pigs (supplied by Nippon SLC Co.) as divided into groups each consisting of 3 to 4 animals. The guinea pigs were anesthetized with pentobarbital sodium (60 mg/kg, given intraperitoneally), and were subjected individually to insertion of a cannula into the airways. Each airway cannula was connected with a volume-controlled respirator (Model SN-480-N, manufactured by Shinano Seisakusho Co.) to perform artificial respiration at a ventilation volume of 5 mL and at a ventilation rate of ca. 70/min. For the purpose of application of metacholine, a catheter was inserted into the carotid vein. The test substance was dissolved in an aqueous solution or aqueous solution containing DMSO, and the solution was applied into the trachea with use of an intratracheal application device for a liquid IA-1b (supplied by Pen-Century Co.)(the application amount of the liquid; 0.8 mL/kg). Prior to application of the test substance, and from 5 to 60 min. thereafter, respectively, metacholine was applied intravenously (10 µg/kg) to induce the airway-constriction, and the pressure at which air was passed through the airways, or the air-passage pressure through the airways, was measured with use of the Konzett-Rossler method. The airways-constriction ratio was taken as a proportion, expressed in percentage, of the air-passage pressure through the airways measured after individual application of the test substance to the maximum air-passage pressure through the airways found when the airways were completely clogged and occluded. The constriction-suppression ratio of the test substance was expressed as a proportion of the average or mean airway-constriction ratio found for the groups not treated through application of the test substance to the average or mean airway-constriction ratio found for the groups treated through application of the test substance. It is to be noticed that the minimum dose, at which not less than 80 % of the present reaction is suppressed at a point of time falling within the length of time of 60 min. after application of the test substance, is taken as the effective dose (provided, however, that the airway-constriction suppression ratio, when applied at a dose equivalent to one third of the effective dose, shall be less than 80 % at all the points of time falling within the length of time 60 min. after application of the test substance).

### Experiment Example 2: Evaluation of the Effects on the Bladder Constriction in Guinea Pigs

The present experiment was carried out with Hartley male guinea pigs (supplied by Nippon SLC Co.) as divided into groups each consisting of 3 to 4 animals. The guinea pigs were anesthetized with a urethane (1.8 mg/kg, given subcutaneously), and were subjected individually to insertion of a catheter into the carotid vein in order to apply metacholine. The cervix of the bladder was ligated, whilst the vertex of the bladder had a catheter inserted therein for regulation and measurement of the internal pressure of the bladder. All the ureters were ligated in order to prevent the urine from flowing into the bladder from the kidney. The internal pressure of the bladder was measured by means of a pressure transducer, whereby the bladder was filled with physiological saline to such an extent as might exert lower pressure than the urination threshold pressure against the inner wall. The test substance was dissolved in an aqueous solution containing DMSO, and the solution was applied into the trachea with use of an intratracheal application device for a liquid IA-1b (supplied by Pen-Century Co.)(the application amount of the liquid; 0.8 mL/kg). Prior to application of the test substance, and from 5 to 60 min. thereafter, respectively, metacholine was applied intravenously (10 pg/kg) to induce the bladder constriction, and the internal pressure of the bladder was measured in a time-course manner. The bladder-constriction ratio was taken as the proportion of the internal pressure of the bladder measured after application of the test substance to the internal pressure of the bladder before application of the test substance. It is to be noticed that the maximum dose, at which less than 20 % of the present reaction is suppressed at all points of time falling within the length of time of 60 min. after application of the test substance, is taken as the no-effective dose (provided, however, that the bladder-constriction suppression ratio found at doses three-times greater than the no-effective dose shall be not less than 20 % at a point of time falling within the length of time 60 min. after application of the test substance).

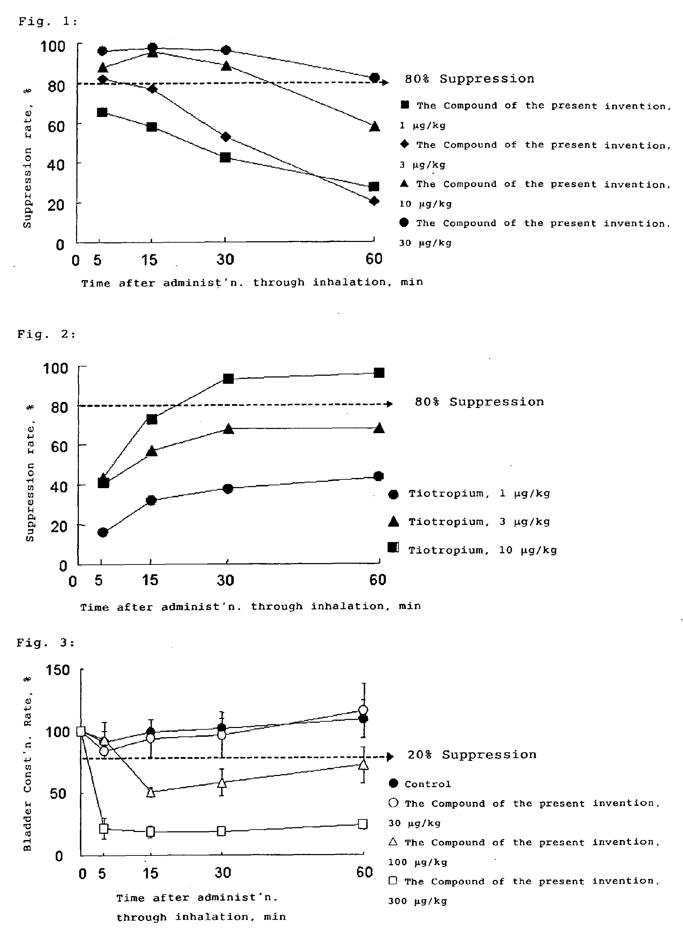

Presented in Figs. 1, 2, 3 and 4 are the test results obtained with the compound as described in Example 14 (4), the compound of the present invention, and the conventional anticholinergic drug, tiotropium.

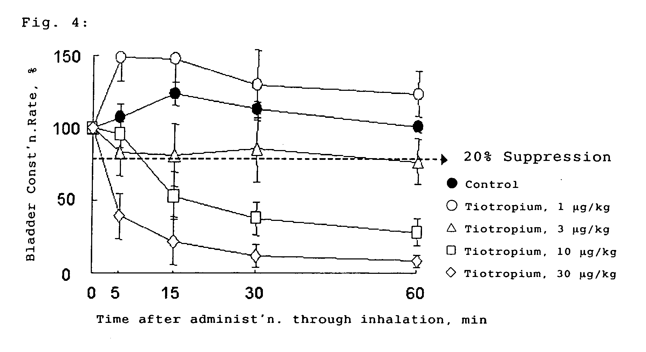

From Figs. 1 and 3, it has been demonstrated that the compound of the present invention individually shows 3 µg/kg of the effective dose for the airway-constriction suppression and 30 pg/kg of the no-effective dose for the bladder constriction. From Figs. 2 and 4, on the other hand, it has been proven that the conventional drugs individually show 10 pg/kg of the effective dose for the airway-constriction suppression and 1 µg/kg of the no-effective dose for the bladder constriction. From the above findings, consequently, it follows that the compound of the present invention exhibits 1/10 of the proportion of the effective dose for the airway-constriction suppression to the no-effective dose for the bladder constriction, whereas the conventional drug shows 10 of the proportion, with the compounds as described in Examples 23, 24 and 25 showing ca. 1/30 of the proportion.

From the above-stated results, it is evident that the compounds of the present invention produce weaker effect against the bladder than the conventional drug. Namely, the compounds of the present invention manifest less side effects or adverse reactions than the conventional drug.

### [Pharmaceutical Preparation Examples]

### Pharmaceutical Preparation Example 1

The below-described ingredients were mixed to give a solution for inhalation.
- (1R,3r,5S,8s)-8-[3-(2-ethoxy-2-oxoethoxy)benzyl]-3-({hydroxy[di(2-thienyl)]acetyl}oxy)-8-methyl-8-azoniabicyclo[3.2.1]octane bromide 40 mg
- Purified water 1000 mL

### Pharmaceutical Preparation Example 2

Dissolved in distilled water were 5.6 g of (1R,3r,5S,8s)-8-[3-(2-ethoxy-2-oxoethoxy)benzyl]-3-({hydroxy[di(2-thienyl)]acetyl}oxy)-8-methyl-8-azoniabicyclo[3.2.1]octane bromide and 400 g of maltose, followed by addition of 20 g of disodium hydrogen-phosphoric acid 12 hydrate, and the solution was made to the total of 4000 mL with distilled water. In accordance with the conventional procedure, lyophilization and pulverization were effected to give a powder preparation for inhalation.

### [Industrial Applicability]

The compounds of the present invention, which can produce the desired effect at the targeted site and thereafter undergo rapid inactivation, are useful as a prophylactic and/or therapeutic agent with reduced development of side effects or adverse reactions (for example, disuresia, thirsty, tachycardia, gastrointestinal disorders, glaucoma, etc.) against muscarinic-receptor mediated diseases.

## Claims

1. A compound represented by the general formula (I): 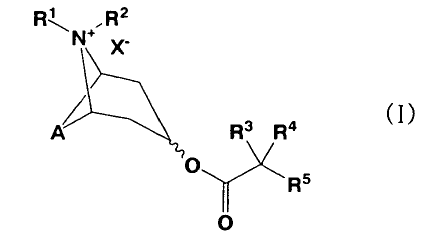 (wherein A represents the following structures: 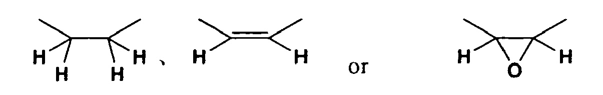 R¹ is a hydrogen atom or a substituent; R² is a hydrogen atom or a substituent; R³ is a hydrogen atom or a substituent; R⁴ is a hydrogen atom or a substituent; R⁵ is a substituent; X⁻ is an anion; the symbol: 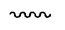 denotes an exo-form or endo-form, or their mixture), its salt or solvation products thereof.

2. The compound according to Claim 1, wherein R¹, R³, R⁴ and/or R⁵ each are(or is) an ester-linkage containing group.

3. The compound according to Claim 1, wherein R¹ is an ester-linkage containing group.

4. The compound according to Claim 3, wherein R¹ constitutes: 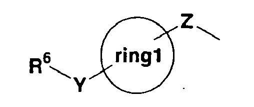 (wherein R⁶ is an ester-linkage containing group; the symbol: 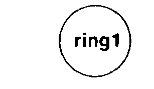 represents a cyclic group which may be substituted with a substituent(s); Y is a linkage(s) or a spacer of 1 to 5 carbon atoms for the main chain which may be substituted with a substituent(s); Z is a linkage(s) or a spacer of 1 to 5 carbon atoms for the main chain which may be substituted with a substituent(s)).

5. The compound according to Claim 4, wherein the cyclic group as defined in Claim 4 is a C3~15 monocyclic unsaturated carbon ring which may be partially or fully saturated, or a C4~15 bicyclic unsaturated carbon ring which ring may be partially or fully saturated, or a C3~15-membered monocyclic unsaturated heterocyclic ring containing 1 to 5 heteroatoms selected from oxygen, nitrogen and sulfur atoms which ring may be partially or fully saturated, or a C4~15 bicyclic unsaturated heterocyclic ring containing 1 to 5 heteroatoms selected from oxygen, nitrogen and sulfur atoms which ring may be partially or fully saturated.

6. The compound according to Claim 4, wherein the cyclic group as defined in Claim 4 is benzene, thiophene or naphthalene.

7. The compound according to Claim 4, which is represented by the general formula (I-1): 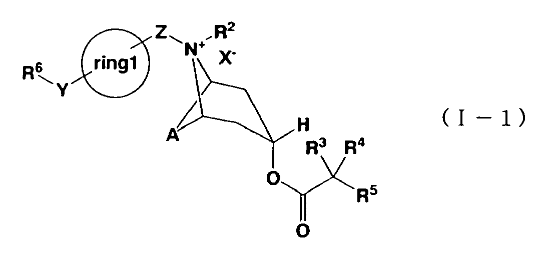 (wherein all the symbols have the same meanings as defined in claim 1 and 4).

8. The compound according to Claim 7, which is selected from (1R,3r,5S,8s)-8-{[6-(2-ethoxy-2-oxoethoxy)-2-naphthyl]-methyl}-3-{[hydroxy(diphenyl)acetyl]oxy}-8-methyl-8-azoniabicyclo[3,2,1]-octane bromide, (1R,3s,5S,8r)-8-{[4-(3-ethoxy-3-oxopropyl)benzyl]-3-({hydroxy[di(2-thieny 1]acetyl}oxy)-8-methyl-8-azoniabicyclo[3,2,1]oct-6-en bromide, (1R,3s,5S,8r)-8-{[5-(4-ethoxy-4-oxobutyl)-2-thienyl]methyl}-3-{[hydroxy(diphenyl)acetyl]oxy}-8-methyl-8-azoniabicyclo[3,2,1]oct-6-en chloride, (1R,2R,4S,5S,7s,9r)-9-[4-(3-ethoxy-3-oxopropyl)benzyl]-7-{[hydroxy(di-3-thienyl)acetyl]oxy}-9-methyl-3-oxa-9-azoniatricyclo[3,3,1,0^{2,4}]nonane bromide and (1R,2R,4S,5S,7s,9r)-9-(4-{3-[2-(dimethylamino)-2-oxoethyl)-3-oxopropyl]benzyl)-7-{[hydroxy(di-2-thienyl)acetyl]oxy}-9-methyl-3-oxa-9-azoniatricyclo[3,3,1,0^{2,4}]nonane bromide;

9. The compound according to Claim 1, wherein R³ and/or R⁴ each are an ester-linkage containing group.

10. The compound according to Claim 9, wherein R³ is represented by the formula: 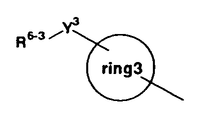 (wherein R⁶⁻³ is an ester-linkage containing group; Y³ is a linkage (s) or a spacer of 1 to 5 atoms for the main chain which may be substituted with a substituent(s); and the symbol: 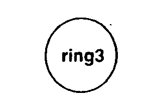 represents a cyclic group which may be substituted with a substituent(s)).

11. The compound according to Claim 9, wherein R³ is represented by the formula: 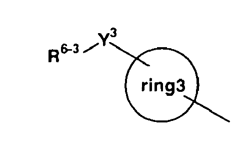 (wherein all the symbols are as defined in Claim 10), and R⁴ is represented by the formula: 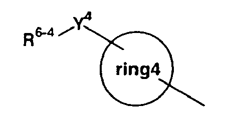 (wherein R⁶⁻⁴ is an ester-linkage containing group; Y⁴ is a linkage (s) or a spacer of 1 to 5 atoms for the main chain which may be substituted with a substituent (s); and the symbol: 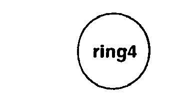 represents a cyclic group which may be substituted with a substituent(s)).

12. The compound according to Claim 10, wherein "ring 3" is benzene, thiophene or furan.

13. The compound according to Claim 10, wherein A is represented by the formula:

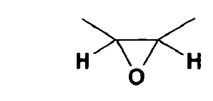

14. The compound according to Claim 10, wherein R¹ and R² each are a methyl group.

15. The compound according to Claim 14, which is selected from (1R,2R,4S,5S,7s)-7-({hydroxy[5-(methoxycarbonyl)-2-thienyl] -2-thienylacetyl}oxy)-9,9-dimethyl-3-oxa-9-azoniatricyclo[3 .3.1.0^{2,4}]nonane bromide, (1R,2R,4S,5S,7s)-7-({hydroxy-[5-(methoxycarbonyl)-2-furyl]-2-thienylacetyl}oxy)-9,9-dimethyl-3-oxa-9-azoniatricyclo[3.3.1.0^{2,4}]nonane bromide and (1R,2R,4S,5S,7s)-7-{[[5-(ethoxycarbonyl)-2-thienyl]-(hydroxy)phenylacetyl]oxy)-9,9-dimethyl-3-oxa-9-azoniatricyclo-[3.3.1.0^{2,4}]nonane bromide.

16. The compound according to Claim 1, wherein R⁵ is an ester-linkage containing group,

17. The compound according to Claim 16, wherein R⁵ is represented by: 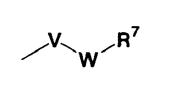 (wherein R⁷ is an ester-linkage containing group; V is a carbon atom which may be substituted with a substituent(s), a nitrogen atom which may be substituted with a substituent(s), a sulfur atom which may be oxidized or an oxygen atom; W is a linkage (s) or a spacer of 1 to 5 atoms for the main chain which may be substituted with a substituent (s).

18. The compound according to Claim 17, which is represented by the general formula (I-2): 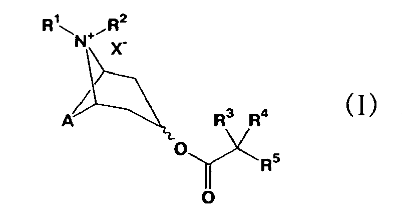 (wherein all the symbols are as defined in Claims 1 and 17).

19. The compound according to Claim 18, wherein R³ and R⁴ each independently are a carbon cyclic group which may be substituted or a heterocyclic group which may be substituted.

20. The compound represented by the general formula (I) according to Claim 1, its salt or a prodrug of a solvation product thereof.

21. A pharmaceutical composition which comprises the compound represented by the general formula (I) according to Claim 1, its salt or their solvation product, or a prodrug thereof as an active ingredient.

22. The pharmaceutical composition according to claim 21, which is a prophylactic and/or therapeutic agent for the diseases mediated by the muscarinic receptor.

23. The pharmaceutical composition according to Claim 22, wherein the disease mediated by the muscarinic receptor is chronic occlusive pulmonary disease and/or asthma.

24. A pharmaceutical composition which comprises the compound represented by the general formula (I) according to Claim 1, its salt or their solvation product, or a prodrug thereof in combination with not less than at least one kind selected from cysLT1-receptor antagonist drugs, cysLT2-receptor antagonist drugs, antihistamine drugs, antiallergy drugs, steroidal drugs, bronchodilators, vaccination therapy agents, gold compound preparations, Chinese herbal medicines, basic non-steroidal anti-inflammatory drugs, 5-lipoxygenase inhibitor drugs, 5-lipoxygenase activating protein antagonist drugs, leucotriene synthesis inhibitor drugs, prostaglandins, cannabinoid-2-receptor stimulant drugs, phosphodiesterase inhibitor drugs, antitusssive drugs, expectrant drugs and extraction liquids from skin inflammations of a household rabbit inoculated with vaccinia virus.

25. A method for preventing and/or treating a disease mediated by the muscarinic receptor, **characterized in that** said process comprises administering to a mammal an effective amount of the compound represented by the general formula (I) according to Claim 1, its salt or their solvation product, or a prodrug thereof.

26. A use of the compound represented by the general formula (I) according to Claim 1, its salt or their solvation product, or a prodrug thereof in the manufacture of a prophylactic and/or therapeutic agent for a disease mediated by the muscarinic receptor.

27. An airway-constriction suppressing agent, **characterized in that** an effective dose for the airway-constriction suppression is less than the no-effective dose for the bladder constriction.

28. The agent according to Claim 27, **characterized in that** said agent fails to exhibit suppressory activity against the bladder constriction.

29. The agent according to Claim 28, wherein the agent comprises an effective amount of the compound according to Claim 27.

30. The agent according to Claim 27, wherein the compound is a compound showing not less than 7 in a pK_{B} value against the tracheal-muscle constriction reaction.

31. The agent according to Claim 27, wherein the agent is a prophylactic and/or therapeutic agent for a disease mediated by the muscarinic receptor.

32. The agent according to Claim 31, wherein the disease mediated by the muscarinic receptor is chronic occlusive pulmonary disease and/or asthma.

33. The agent according to Claim 27, wherein the agent is in the form of an inhalant

34. The agent according to Claim 27, **characterized in that** said agent is administered once a day.

35. The agent according to Claim 27, wherein the compound is a compound represented by the general formula (I): 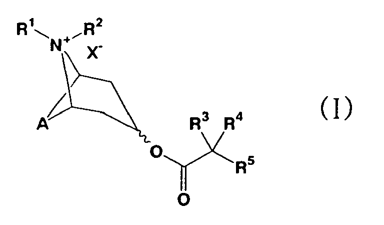 (wherein all the symbols are as defined in Claim 1), its salt or their solvation product, or a prodrug thereof.

36. A method for suppressing the airway constriction, **characterized in that** said method comprises administering to a mammal an effective dose of a compound, which shows an effective amount for the airway- constriction suppression being less than the no-effective dose for the bladder constriction.

37. A use of the compound which exhibits an effective amount for the airway-constriction suppression being less than the no-effective dose for the bladder constriction in the manufacture of the airway-constriction suppresant.
